(19) Europäisches Patentamt
European Patent Office
Office européen des brevets

(11) Veröffentlichungsnummer: **0 019 702**
**B1**

(12) # EUROPÄISCHE PATENTSCHRIFT

(45) Veröffentlichungstag der Patentschrift: **11.12.85**

(21) Anmeldenummer: **80101862.3**

(22) Anmeldetag: **08.04.80**

(51) Int. Cl.[4]: **C 07 C 93/06,** C 07 C 149/24, C 07 C 103/44, C 07 C 103/82, C 07 C 93/187, C 07 C 93/20, C 07 C 101/12, C 07 C 103/54, D 06 L 3/12, C 11 D 3/42 // C07D295/08, C07D295/12, C07F9/40, C11D1/62, D06M13/46

(54) **Distyrylbiphenyle, Verfahren zu deren Herstellung sowie deren Verwendung beim optischen Aufhellen organischer Materialien sowie Waschmittel, Textilbehandlungsmittel und Wäschenachbehandlungsmittel, die sie enthalten.**

(30) Priorität: **11.04.79 CH 3478/79**
**26.06.79 CH 5952/79**

(43) Veröffentlichungstag der Anmeldung:
**10.12.80 Patentblatt 80/25**

(45) Bekanntmachung des Hinweises auf die Patenterteilung:
**11.12.85 Patentblatt 85/50**

(84) Benannte Vertragsstaaten:
**AT BE CH DE FR GB IT LI NL SE**

(56) Entgegenhaltungen:
**CH-A- 554 821**
**DE-A-2 209 223**
**FR-A-2 427 340**
**GB-A-1 247 934**
**US-A-3 927 119**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Postfach**
**CH-4002 Basel (CH)**

(72) Erfinder: **Lüthi, Christian, Dr.**
**Anwilerstrasse 10**
**CH-4059 Basel (CH)**
Erfinder: **Meyer, Hans Rudolf, Dr.**
**Bollwerkstrasse 102**
**CH-4102 Binningen (CH)**
Erfinder: **Weber, Kurt, Dr.**
**Rennweg 98**
**CH-4052 Basel (CH)**

(74) Vertreter: **Zumstein, Fritz sen., Dr. et al**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 702 B1

Courier Press, Leamington Spa, England.

**Beschreibung**

Die vorliegende Erfindung betrifft neue Distyrylbiphenyle, ein Verfahren zu deren Herstellung sowie deren Verwendung zum optischen Aufhellen von organischen Materialien sowie Waschmittel, Textilbehandlungsmittel und Wäschenachbehandlungsmittel, die sie enthalten.

Es sind aus der Literatur z.B. aus der DE—AS 17 93 482, CH—PS 554 821, GB—PS 1 247 934, DE—OS 2 209 223, US—PS 3 927 119 und FR—A 2 427 340 in den Benzolkernen anionisch substituierte wasserlösliche Distyrylbiphenyle bekannt, welche aber mit kationaktiven Textilhilfsmitteln nicht verträglich sind. Aus der CH—PS 554 821 und GB—PS 1 247 934 sind auch gewisse kationisch substituierte Distyrylbiphenyle bekannt, die aber trotz ihres kationischen Charakters zu unbefriedigenden Ergebnissen führen, wenn sie in kationischem Medium zur Anwendung gelagen. Ein in der US—PS 3 927 119 beschriebenes Distyrylbiphenyl enthält in den beiden endständigen Phenylringen in der ortho-Stellung zur Gruppe —CH=CH— jeweils einen Rest $—OCH_2CONH(CH_2)_3—N(CH_3)_2$.

Es wurden nun Distyrylbiphenyle gefunden, welche diese Nachteile nicht aufweisen.

Die Erfindung betrifft demnach neue Distyrylbiphenyle der Formel

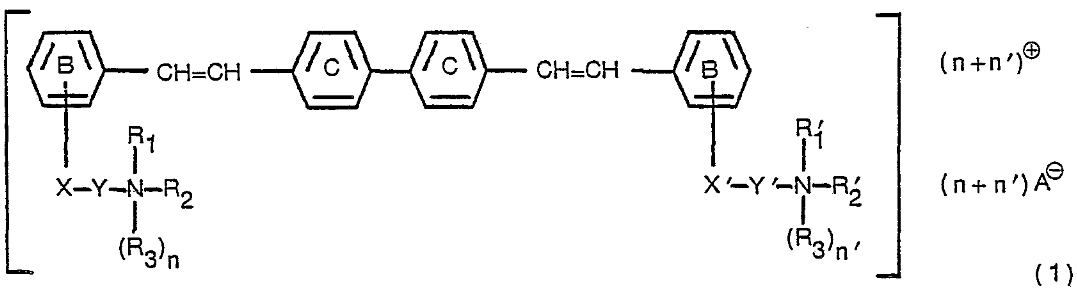

(1)

worin X und X' unabhängig voneinander die direkte Bindung, Sauerstoff, Schwefel, $—O—C_{1-3}$-Alkylen-$CON(R_4)$—, $—CON(R_4)$—, $—O—C_{1-3}$-Alkylen-COO—, —OCO— oder —COO—, wobei, wenn n + n' = 0, X und X' nicht $—CON(R_4)$— oder $—O—C_{1-3}$-Alkylen-$CON(R_4)$— und wenn n + n' = 2 und X und X' $—CON(R_4)$— oder —COO—, $A^{\ominus}$ Nicht Phosphit- oder Phosphonatanion sein dürfen, Y und Y' unabhängig voneinander $C_{1-20}$-Alkylen oder durch eine Hydroxylgruppe substituiertes $C_{1-20}$-Alkylen, $R_1$ und $R_1'$ unabhängig voneinander unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_1$ bzw. $R_1'$ zusammen mit $R_2$ bzw. $R_2'$ und dem N-Atom, an das sie gebunden sind, einen 5- bis 7-gliedrigen heterocyclischen Ring, $R_2$ und $R_2'$ unabhängig voneinander unsubstituiertes oder durch Cyano, Hydroxy Alkoxy, Phenyl, $C_{1-4}$-Alkoxycarbonyl oder Chlor substituiertes $C_{1-8}$-Alkyl, $C_{3-4}$-Alkenyl oder $R_2$ bzw. $R_2'$ zusammen mit $R_1$ bzw. $R_1'$ und dem N-Atom, an das sie gebunden sind, einen 5- bis 7-gliedrigen heterocyclischen Ring, $R_3$ Wasserstoff, unsubstituiertes oder durch Cyano, Hydroxy, Alkoxy, Phenyl oder $C_{1-4}$-Alkoxycarbonyl substituiertes $C_{1-4}$-Alkyl oder $C_{3-4}$-Alkenyl, $R_1$, $R_2$ und $R_3$ zusammen mit dem N-Atom, an das sie gebunden sind, bzw. $R_1'$, $R_2'$ und $R_3$ zusammen mit den N-Atom, an das sie gebunden sind, auch den Pyridin- oder Picolinring, $R_4$ Wasserstoff oder unsubstituiertes oder durch Cyano, Carbamoyl, Carbalkoxy, Hydroxy, Halogen oder $C_{1-4}$-Alkoxy substituiertes $C_{1-6}$-Alkyl, $A^{\ominus}$ ein farbloses Anion und n und n' unabhängig voneinander die Zahl 0 oder 1 bedeuten, wobei die Benzolkerne B und C auch nicht-chromophor substituiert sein können, und worin die Gruppierungen

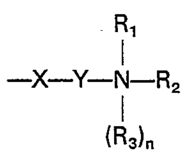 sowie 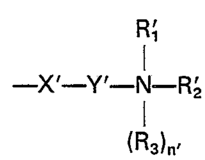

im Falle, dass die Ringe B und C keine nicht-chromophoren Substituenten tragen, auch beide jeweils

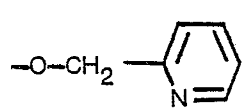

oder beide jeweils

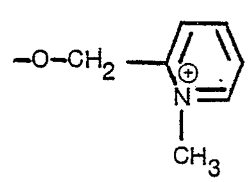

in den ortho-Stellungen zu den beiden Gruppen —CH=CH— sein können.

Die Alkylenreste Y und Y' können verschieden, geradkettig oder verzweigt sein und vorzugsweise 1 bis 12 und besonders 1 bis 6 C-Atome aufweisen.

Geeignete Alkylreste $R_1$, $R_1'$, $R_2$ und $R_2'$ sind solche mit 1 bis 8, vorzugsweise 1 bis 4 C-Atomen. Diese Reste sowie Alkylreste $R_3$ können geradkettig oder verzweigt sein.

Als 5- bis 7-gliedriger heterocyclischer Ring gebildet durch die Reste $R_1$ und $R_2$ sowie $R_1'$ und $R_2'$ zusammen mit dem N-Atom, an das sie gebunden sind, kommt z.B. ein Piperidin-, Pyrrolidin-, Hexamethylenimin, Pyridin-, Triazol-, Imidazol- oder Morpholinring, die durch Alkylgruppen mit 1 bis 4 C-Atomen substituiert sein können, in Betracht.

Ein Alkylrest $R_4$ weist vorzugsweise 1 bis 4 C-Atome auf.

Als nicht-chromophore Substituenten der Benzolringe B und C seien beispielsweise erwähnt: Halogenatome wie Chlor und Brom; $C_{1-4}$-Alkylgruppen, $C_{5-7}$-Cycloalkylgruppen, $C_{3-4}$-Alkenylgruppen; $C_{1-4}$-Alkoxygruppen, $C_{3-4}$-Alkenyloxygruppen; Sulfonylgruppen, z.B. $C_{1-4}$-Alkyl- oder Phenylsulfonylgruppen; $C_{2-6}$-Carbalkoxygruppen; Carbamoylgruppen und Sulfamoylgruppen, sowie die Ergänzung zu einem ankondensierten carboxycyclischen 5- oder 6-gliedrigen Ring.

Im Rahmen der erfindungsgemässen Distyrylbiphenyle verdienen eine besondere Erwähnung solche der Formel

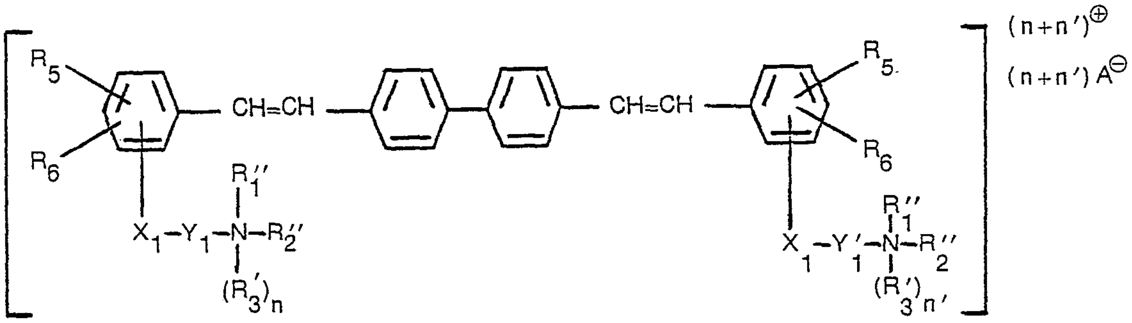

(2)

worin $X_1$ die direkte Bindung, Sauerstoff, Schwefel, —O—$C_{1-3}$-Alkylen-CONH—, —CONH—, —O—$C_{1-3}$-Alkylen-COO—, —OCO— oder —COO—, wobei, wenn n + n' = 0, $X_1$ nicht —CONH— oder —O—$C_{1-3}$-Alkylen-CONH— und wenn n + n' = 2 und $X_1$ —CONH— oder —COO—, $A^{\ominus}$ nicht Phosphit- oder Phosphonatanionen sein dürfen, $Y_1$ und $Y_1'$ unabhängig voneinander $C_{1-4}$-Alkylen oder Hydroxypropylen, $R_1''$ und $R_2''$ unabhängig voneinander $C_{1-4}$-Alkyl oder $R_1''$ und $R_2''$ zusammen mit dem N-Atom, an das sie gebunden sind, einen Pyrrolidin-, Piperidin-, Hexamethylenimin- oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_2$—$C_4$-Hydroxyalkyl oder $C_{2-4}$-Cyanoalkyl, $R_1''$, $R_2''$ und $R_3'$ zusammen mit dem N-Atom, an das sie gebunden sind, auch den Pyridin- oder Picolinring, $R_5$ Wasserstoff, Chlor, $C_{1-4}$-Alkyl, $C_{3-4}$-Alkenyl, $C_{1-3}$-Alkoxy oder $R_5$ zusammen mit $R_6$ eine Trimethylen- oder Tetramethylengruppe, $R_6$ Wasserstoff, Chlor, $C_{1-4}$-Alkyl, $C_{1-3}$-Alkoxy oder $R_6$ zusammen mit $R_5$ eine Trimethylen- oder Tetramethylengruppe, n und n' unabhängig voneinander die Zahl 0 oder 1, und $A^{\ominus}$ ein farbloses Anion bedeuten.

Bevorzugte Distyrylbiphenyle der Formeln (1) und (2) sind solche,

— welche symmetrisch sind, d.h. worin X = X', Y = Y' bzw. $Y_1 = Y_1'$, $R_1 = R_1'$, $R_2 = R_2'$ und n = n';

— worin X und X' nicht —CON($R_4$)—, —O—$C_{1-3}$-Alkylen-CON($R_4$)— oder —COO— und $X_1$ nicht —CONH—, —O—$C_{1-3}$-Alkylen-CONH— oder —COO— bedeuten;

— welche quaterniert sind, d.h. worin n und n' die Zahl 1 bedeuten und

— worin $X_1$ in 2-Stellung und $R_3'$ $C_{1-3}$-Alkyl sind.

In distyrylbiphenylen der Formel (2) bedeuten Alkyl- und Alkoxysubstituenten $R_5$ und $R_6$ vorzugsweise Methyl und Methoxy.

Besonders bevorzugt sind Distyrylbiphenyle der Formel

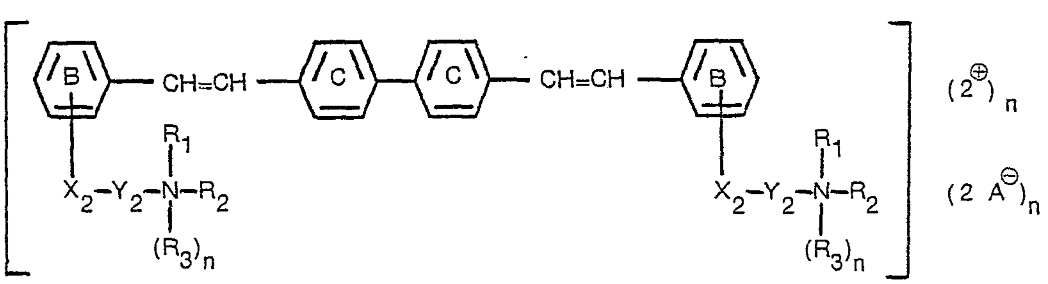

(3)

worin $X_2$ Sauerstoff, Schwefel, —CON($R_4$)—, —OCO— oder —COO— wobei, wenn n = 0, $X_2$ nicht —CONH($R_4$)— und wenn n = 1 und $X_2$ —CONH($R_4$)— oder —COO— ist, $A^\ominus$ nicht Phosphit- oder Phosphonatanionen sein dürfen, $Y_2$ $C_{1-20}$-Alkylen oder durch eine Hydroxylgruppe substituiertes $C_{1-6}$-Alkylen, $R_1$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_1$ zusammen mit $R_2$ und dem N-Atom, an das sie gebunden sind, einen 5- bis 7-gliedrigen heterocyclischen Ring, $R_2$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_2$ zusammen mit $R_1$ und dem N-Atom, an das sie gebunden sind, einen 5- bis 7-gliedrigen heterocyclischen Ring, $R_3$ Wasserstoff, unsubstituiertes oder durch Cyano, Hydroxy, Alkoxy, Phenyl oder $C_{1-4}$-Alkoxycarbonyl substituiertes $C_{1-4}$-Alkyl, $R_1$, $R_2$ und $R_3$ zusammen mit dem N-Atom, an das sie gebunden sind, auch den Pyridin- oder Picolinring, $R_4$ Wasserstoff oder unsubstituiertes oder durch Cyano, Carbamoyl, Carbalkoxy, Hydroxy, Halogen oder $C_{1-4}$-Alkoxy substituiertes $C_{1-6}$-Alkyl, n die Zahl 0 oder 1 und $A^\ominus$ ein farbloses Anion bedeuten, wobei die Benzolkerne B und C auch nicht-chromophor substituiert sein können; solche der Formel

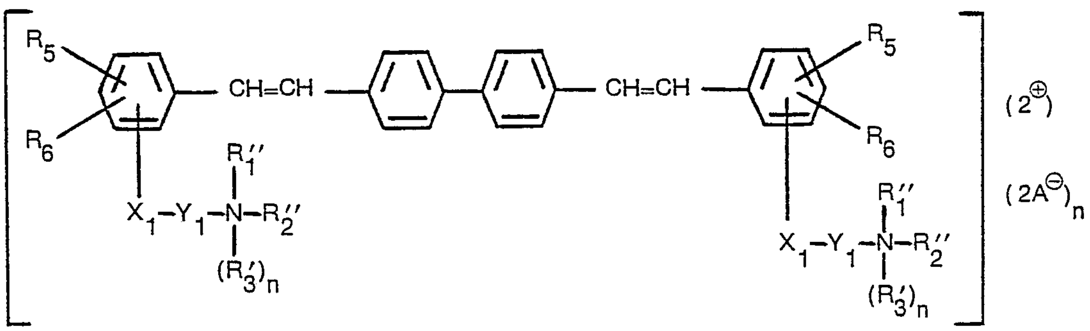

(4)

worin $X_1$ die direkte Bindung, Sauerstoff, Schwefel, —O—$C_{1-3}$-Alkylen-CONH—, —CONH—, —O—$C_{1-3}$-Alkylen-COO—, —OCO— oder —COO—, wobei, wenn n = 0, $X_1$ nicht —CONH— oder —O—$C_{1-3}$-Alkylen-CONH— und wenn n = 1 und $X_1$ —CONH— oder —COO— ist, $A^\ominus$ nicht Phosphit- oder Phosphonatanionen sein dürfen, $Y_1$ $C_{1-4}$-Alkylen oder Hydroxypropylen, $R_1''$ und $R_2''$ unabhängig voneinander $C_{1-4}$-Alkyl oder $R_1''$ und $R_2''$ zusammen mit dem N-Atom, an das sie gebunden sind, einen Pyrrolidin-, Piperidin-, Hexamethylenimin- oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_{2-4}$-Hydroxyalkyl oder $C_{2-4}$-Cyanoalkyl, $R_1''$, $R_2''$ und $R_3'$ zusammen mit dem N-Atom, an das sie gebunden sind, auch den Pyridin- oder Picolinring,

$R_5$ Wasserstoff, Chlor, $C_{1-4}$-Alkyl, $C_{3-4}$-Alkenyl, $C_{1-3}$-Alkoxy oder $R_5$ zusammen mit $R_6$ eine Trimethylen- oder Tetramethylengruppe, $R_6$ Wasserstoff, Chlor, $C_{1-4}$-Alkyl, $C_{1-3}$-Alkoxy oder $R_6$ zusammen mit $R_5$ eine Trimethylen- oder Tetramethylengruppe, n die Zahl 0 oder 1, und $A^\ominus$ ein farbloses Anion bedeuten, und solche der Formel

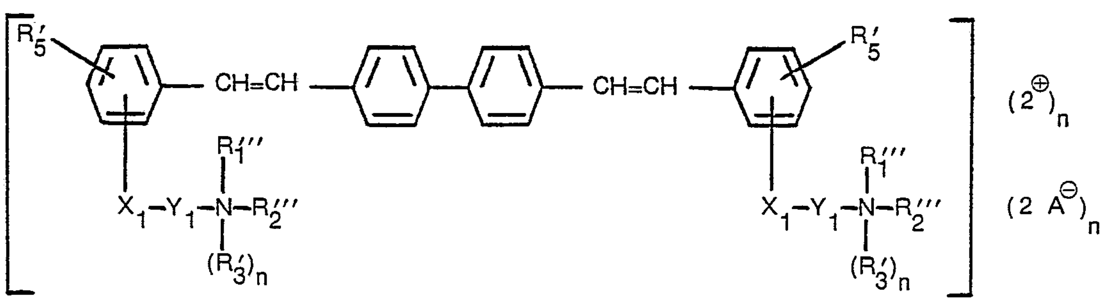

(5)

worin $X_1$ die direkte Bindung, Sauerstoff, Schwefel, —O—$C_{1-3}$-Alkylen-CONH—, —CONH—, —O—$C_{1-3}$-Alkylen-COO—, —OCO— oder —COO—, wobei, wenn n = 0, $X_1$ nicht —CONH— oder —O—$C_{1-3}$-Alkylen-CONH— und wenn n = 1 und $X_1$ —CONH— oder —COO— ist, $A^\ominus$ nicht Phosphit- oder Phosphonatanionen sein dürfen, $Y_1$ $C_{1-4}$-Alkylen oder Hydroxypropylen, $R_1'''$ und $R_2'''$ unabhängig voneinander $C_{1-4}$-Alkyl, $R_1'''$ und $R_2'''$ zusammen mit dem N-Atom, an das sie gebunden sind, einen Pyrrolidin, Piperidin-, oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_{2-4}$-Hydroxyalkyl oder $C_{2-4}$-Cyanoalkyl, $R_1'''$, $R_2'''$ und $R_3'$ zusammen mit dem N-Atom, an das sie gebunden sind, auch den Pyridinring, $R_5'$ Wasserstoff, Chlor, $C_{1-4}$-Alkyl oder $C_{1-3}$-Alkoxy, n die Zahl 0 oder 1, und $A^\ominus$ ein farbloses Anion bedeuten.

In den Formeln (3), (4), (5) bzw. (6) sind $X_1$ und $X_2$ vorzugsweise in 2-Stellung und nicht —$CONH(R_4)$—, —CONH—, —O—$C_{1-3}$-Alkylen-CONH— oder —COO—; $R_3'$ und $R_3''$ $C_{1-3}$-Alkyl; n gleich 1 und $A^{\ominus}$ $CH_3OSO_3^{\ominus}$, $C_2H_5OSO_3^{\ominus}$, $Cl^{\ominus}$, $Br^{\ominus}$, $CH_3COO^{\ominus}$, $HCOO^{\ominus}$ oder

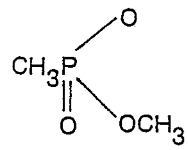

Von Bedeutung sind die Distyrylbiphenyle

A) der Formel

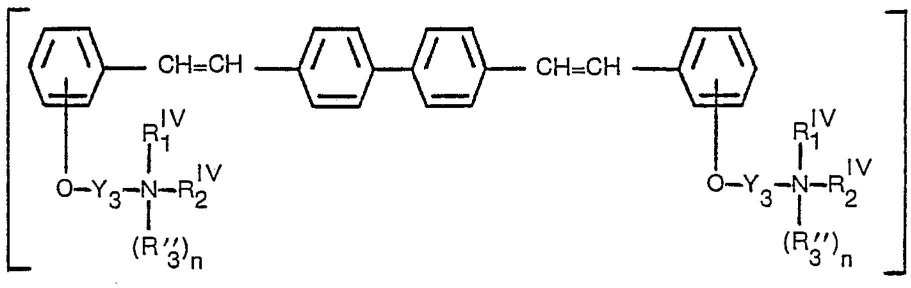

(6)

worin $Y_3$ $C_{2-4}$-Alkylen, $R_1^{IV}$ $C_{1-3}$-Alkyl oder $R_1^{IV}$ zusammen mit $R_2^{IV}$ und dem N-Atom, an das sie gebunden sind einen Pyrrolidin-, Piperidin-, Hexamethylenimin- oder Morpholinring, $R_2^{IV}$ $C_{1-3}$-Alkyl oder $R_2^{IV}$ zusammen mit $R_1^{IV}$ und dem N-Atom, an das sie gebunden sind, einen Pyrrolidin-, Piperidin-, Hexamethylenimin- oder Morpholinring, $R_3''$ Wasserstoff oder $C_{1-3}$-Alkyl, n die Zahl 0 oder 1 und $A^{\ominus}$ ein farbloses Anion bedeuten.

B) der Formel

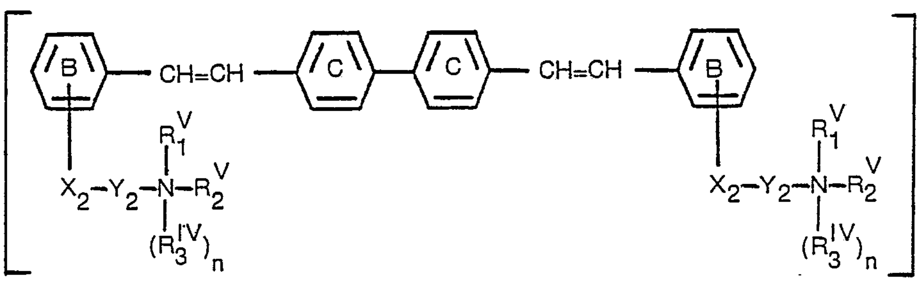

(7)

worin $X_2$ Sauerstoff, Schwefel

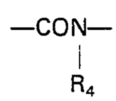

oder —COO—, wobei wenn $A^{\ominus}$ ein Phosphit- oder Phosphonatanion darstellt, $X_2$ nicht —$CON(R_4)$— oder —COO— ist, $Y_2$ $C_{2-20}$-Alkylen, $R_1^V$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_1^V$ zusammen mit $R_2^V$ und dem N-Atom, an das sie gebunden sind, einen 5- bis 7-gliedrigen heterocyclischen Ring, $R_2^V$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_2^V$ zusammen mit $R_1^V$ und dem N-Atom, an das sie gebunden sind, einen 5- bis 7-gliedrigen heterocyclischen Ring, $R_3^{IV}$ Wasserstoff oder unsubstituiertes oder durch Cyano, Hydroxy, Alkoxy, Phenyl oder $C_{1-4}$-Alkoxycarbonyl substituiertes $C_{1-4}$-Alkyl, $R_4$ Wasserstoff oder unsubstituiertes oder durch Cyano, Carbamoyl, Carbalkoxy, Hydroxy, Halogen oder $C_{1-4}$-Alkoxy substituiertes $C_{1-6}$-Alkyl, $A^{\ominus}$ das Aequivalent eines farblosen Anions und n die Zahl 0 oder 1 bedeuten, wobei die Benzolkerne B und C auch nichtchromophor substituiert sein können,

C) der Formel

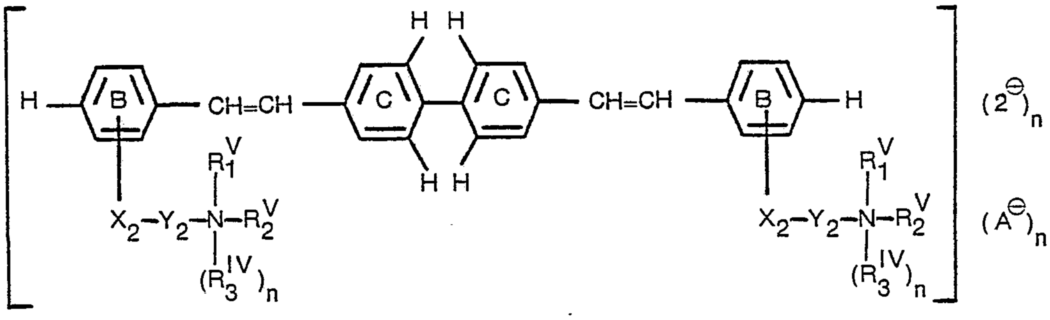

(8)

worin $X_2$, $Y_2$, $R_1^V$, $R_2^V$, $R_3^{IV}$, $A^{\ominus}$, n und die Benzolkerne B und C die oben angegebene Bedeutung haben, und

D) der Formel

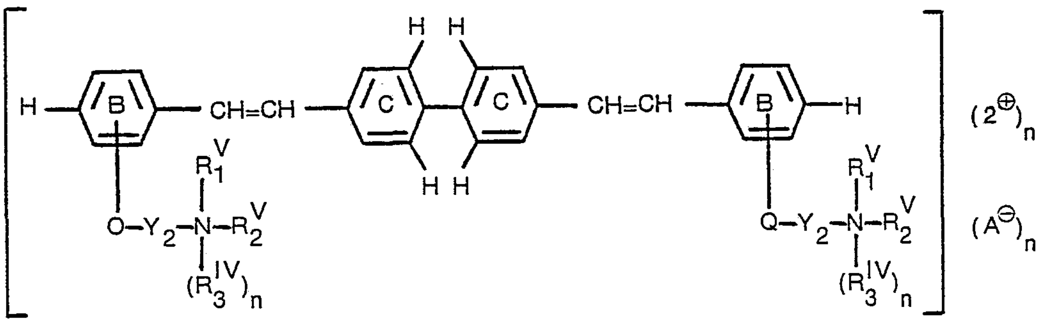

(9)

worin $Y_2$, $R_1^V$, $R_2^V$, $R_3^{IV}$, $A^{\ominus}$ n und die Benzolkerne B und C, die oben angegebene Bedeutung haben.

Besonders bevorzugt sind Distyrylbiphenyle der Formeln

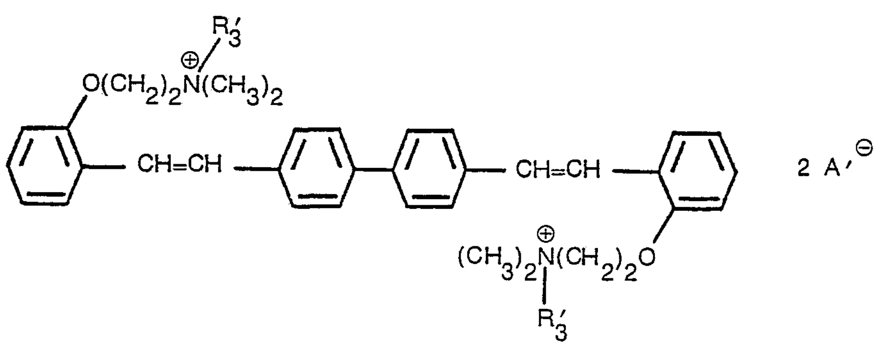

(10)

worin $A'^{\ominus}$ $CH_3OSO_3^{\ominus}$ oder $C_2H_5OSO_3^{\ominus}$ und $R_3'$ Methyl oder Aethyl bedeuten,

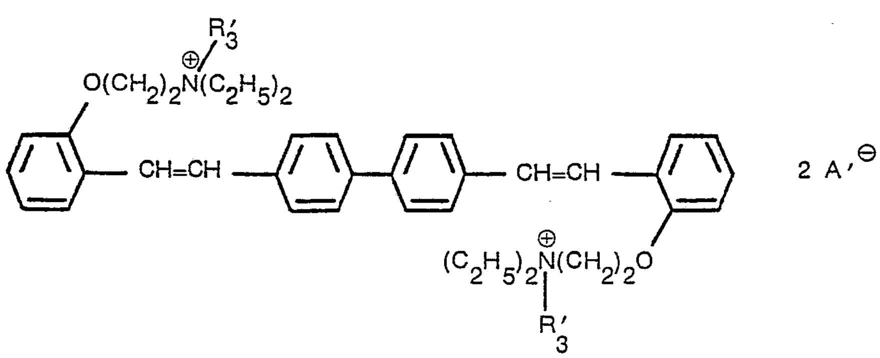

(11)

worin $A'^{\ominus}$ $CH_3OSO_3^{\ominus}$ oder $C_2H_5OSO_3^{\ominus}$ und $R_3'$ Methyl oder Aethyl bedeuten und besonders der Formel

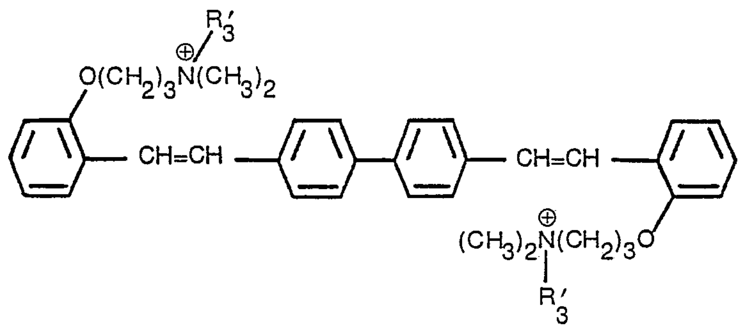 2 $A'^{\ominus}$

(12)

worin $A'^{\ominus}$ $CH_3OSO_3^{\ominus}$ oder $C_2H_5OSO_3^{\ominus}$ und $R_3'$ Methyl oder Aethyl bedeuten.

Die Herstellung der Distyrylbiphenyle der Formel (1) worin X die direkte Bindung, Sauerstoff oder Schwefel und n und n' die Zahl 0 bedeuten, erfolgt in an sich bekannter Weise indem man im Molekularverhältnis 1:2 eine Verbindung der Formel

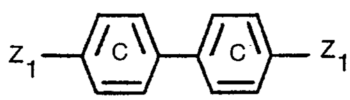

(13)

mit einer der beiden oder einer Mischung der beiden Verbindungen der Formel

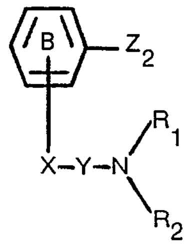 und 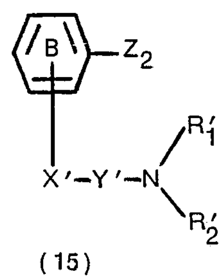

(14) (15)

in Gegenwart einer starken Base umsetzt, in welchen Formeln die Benzolkerne B und C auch nichtchromophor substituiert sein können, X und X' die direkte Bindung, Sauerstoff oder Schwefel und Y, Y', $R_1$, $R_1'$, $R_2$ und $R_2'$ die oben angegebene Bedeutung haben und eines der Symbole $Z_1$ und $Z_2$ eine OCH-Gruppe und das andere eine Gruppierung der Formel

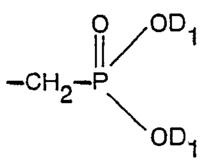 , 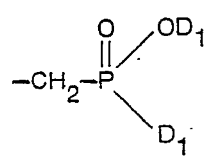 ,

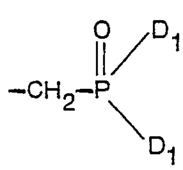 oder 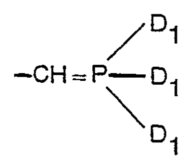

bedeutet, worin $D_1$ einen unsubstituierten oder substituierten Alkyl-, Aryl-, Cycloalkyl- oder Aralkylrest darstellt, und anschliessend die erhaltene Verbindung der Formel

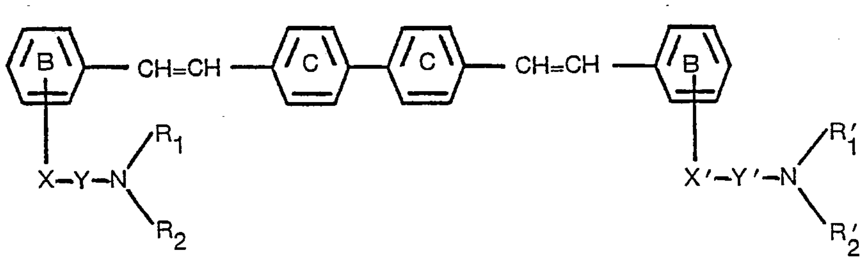

(16)

worin X, X', Y, Y', $R_1$, $R_1'$, $R_2$ und $R_2'$ die oben angegebene Bedeutung haben und die Benzolkerne B und C auch nicht-chromophor substituiert sein können, in an sich bekannter Weise mit 1 bis 2 Moläquivalenten eines Alkylierungsmittels bzw. einer Säure der Formel $R_3$—A, worin $R_3$ und A die oben angegebene Bedeutung haben, quaterniert bzw. protoniert.

Distyrylbiphenyle der Formel (6) werden dadurch hergestellt, dass man im Molekularverhältnis 1:2 eine Verbindung der Formel

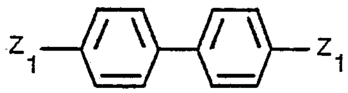

mit solchen der Formel

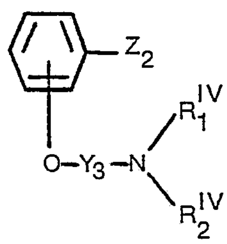

in Gegenwart einer starken Base umsetzt, in welchen Formeln $Y_3$, $R_1^{IV}$ und $R_2^{IV}$ die oben angegebene Bedeutung haben und eines der Symbole $Z_1$ und $Z_2$ eine OCH-Gruppe und das andere eine Gruppierung der Formel

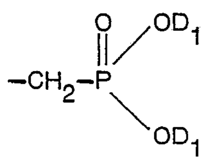 ,

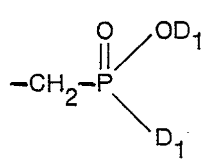 ,

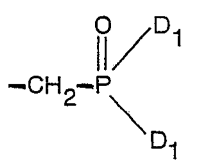

oder

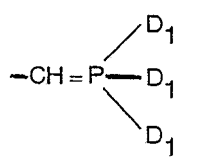

bedeutet, worin $D_1$ einen unsubstituierten oder substituierten Alkyl-, Aryl-, Cycloalkyl oder Aralkylrest darstellt und gewünschtenfalls die erhaltene Verbindung der Formel

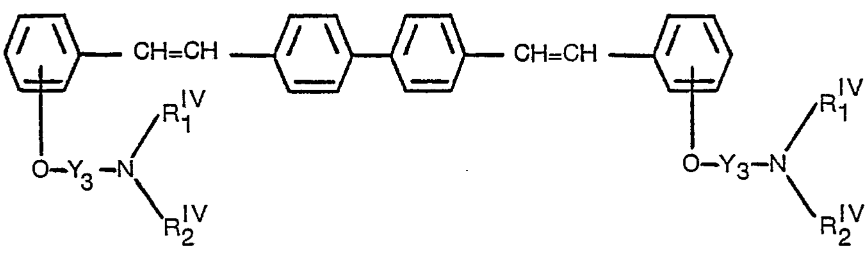

worin $Y_3$, $R_1^{IV}$ und $R_2^{IV}$ die oben angegebene Bedeutung haben mit 2 Moläquivalenten eines Alkylierungsmittels bzw. einer Säure der Formel $R_3''$—A worin $R_3''$ und A die oben angegebene Bedeutung haben, quateriert bzw. protoniert.

Man führt die Kondensation vorteilhaft in indifferenten Lösungsmitteln durch. Als Beispiele hiefür seinen Kohlenwasserstoffe wie Toluol und Xylol oder Alkohole wie Methanol, Aethanol, Isopropanol, Butanol, Glykole, Glykolähter wie 2-Methoxyäthanol, Hexanole, Cyclohexanol und Cyclooctanol, ferner Aether wie Diisopropyläther, Tetrahydrofuran und Dioxan sowie Dimethylsulfoxyd, Formamid und N-Methylpyrrolidon genannt. Besonders geeignet sind polare organische Lösungsmittel wie Dimethylformamid und Dimethylsulfoxyd. Auch in wässriger Lösung lassen sich einige der Umsetzungen durchführen.

Die Temperatur, bei welcher die Umsetzung durchgeführt wird, kann in weiten Grenzen schwanken. Sie wird bestimmt:

α) durch die Beständigkeit des verwendeten Lösungsmittels gegenüber den Reaktionsteilnehmern, insbesondere gegenüber den stark basischen Alkaliverbindungen,

β) durch die Reaktivität der Kondensationspartner und

γ) durch die Wirksamkeit der Kombination Lösungsmittel-Base als Kondensationsmittel.

In der Praxis kommen hiernach im allgemeinen Temperaturen zwischen etwa 10 und 100°C in Betracht. Wenn Dimethylformamid als Lösungsmittel verwendet wird, liegt der Temperatur-Vorzugsbereich bei 20 bis 60°C.

Als stark basische Alkaliverbindungen kommen vor allem die Hydroxyde, Amide und Alkoholate (vorzugsweise 1 bis 4 Kohlenstoffatome enthaltender Alkohole) der Alkalimetalle in Betracht, wobei aus ökonomischen Gründen solche des Lithiums, Natriums und Kaliums von vorwiegendem Interesse sind. Es können jedoch grundsätzlich und in besonderen Fällen auch Alkalisulfide und -carbonate, Arylalkaliverbindungen wie z.B. Phenyl-lithium oder stark basische Amine (einschliesslich Ammoniumbasen) z.B. Trialkylammoniumhydroxyde, mit Erfolg verwendet werden.

Die Ausgangsprodukte der Formel (14) oder (15), worin $Z_2$ eine Alkdehydgruppe bedeutet, erhält man z.B. durch Alkalierung von Hydroxylbenzaldehyden mit Dialkylaminoalkylchloriden in Gegenwart basischer Alkali- oder Erdalkaliverbindungen wie z.B. Natriumalkoholate (siehe Beispiel 1), Kaliumcarbonat, Natriumcarbonat, Calciumcarbonat, Magnesiumoxid, Natriumhydrid, Kaliumhydroxid oder Natriumhydroxid in indifferenten organischen Lösungsmitteln wie Alkoholen, Dialkylamiden aliphatischen Carbonsäuren. Besonders geeignet sind wasserfreie Lösungsmittel, welche auch in der Folgestufe, d.h. zur Herstellung der Verbindungen der Formel (16) brauchbar sind wie z.B. Dimethylformamid, wobei die Isolierung der Verbindungen der Formel (14) oder (15) nicht mehr notwendig ist.

Distyrylbiphenyle der Formel

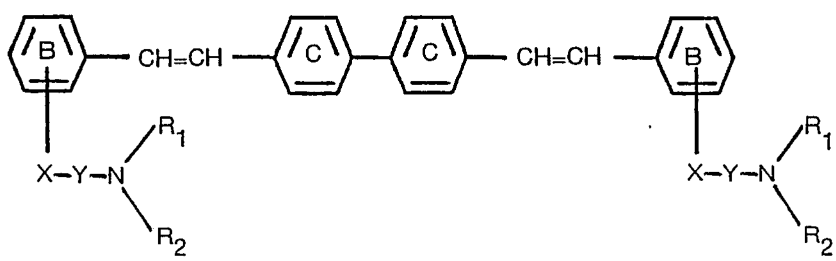

(17)

worin X —O—$C_{1-3}$-Alkylen-CON($R_4$)—, —O—$C_{1-3}$-Alkylen-COO—,

—CON—,
|
$R_4$

oder —COO— bedeutet, werden in an sich bekannter Weise aus den entsprechenden Säuren (vgl. DE—AS 17 93 482; 22 09 128) hergestellt, indem man in an sich bekannter Weise die Säurehalogenide herstellt und diese mit einer Verbindung der Formel HN($R_4$)—Y—N($R_1$)($R_2$) oder HOY—N($R_1$)($R_2$) umsetzt.

Distyrylbiphenyle der Formel

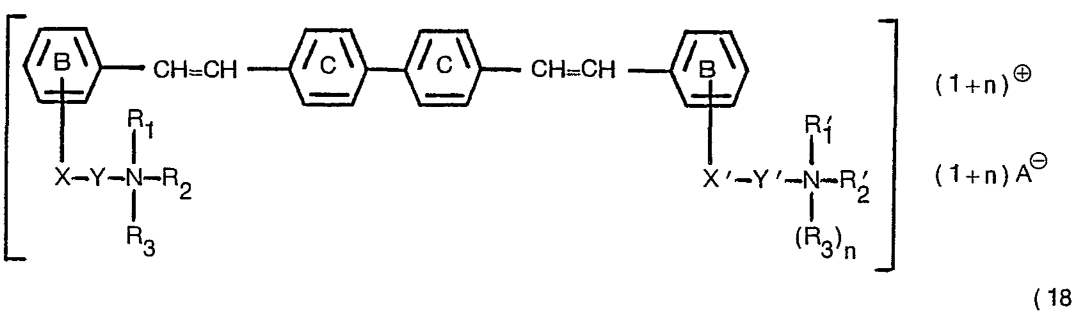

(18)

worin X und X' die direkte Bindung, Sauerstoff, Schwefel, —O—$C_{1-3}$-Alkylen-CON($R_4$)—, —O—$C_{1-3}$-Alkylen-COO—,

—CON—,
|
$R_4$

—OCO oder —COO—, wobei, wenn $A^{\ominus}$ ein Phosphit oder Phosphonatanion darstellt, X nicht —CON($R_4$)—

oder —COO— ist, Y und Y' Alkylen mit 1—20 C-Atomen, $R_1$ und $R_1'$ unsubstituiertes oder substituiertes Alkyl mit 1—8 C-Atomen, Alkenyl mit 2—4 C-Atomen oder zusammen mit $R_2$ bzw. $R_2'$ einen heterocyclischen Ring, $R_2$ und $R_2'$ unsubstituiertes oder substituiertes Alkyl mit 1—8 C-Atomen, Alkenyl mit 2—4 C-Atomen oder zusammen mit $R_1$ bzw. $R_1'$ einen heterocyclischen Ring, $R_3$ Wasserstoff, unsubstituiertes oder substituiertes Alkyl mit 1—4 C-Atomen oder $C_{3-4}$-Alkenyl, $R_4$ Wasserstoff oder unsubstituiertes oder substituiertes Alkyl mit 1—6 C-Atomen, $A^{\ominus}$ ein farbloses Anion und n die Zahl 0 oder 1 bedeuten, wobei die Benzolkerne B und C auch nicht-chromophor substituiert sein können, werden dadurch hergestellt, dass man in an sich bekannter Weise Distyrylbiphenyle der Formeel (16) mit 1 bis 2 Moläquivalenten eines Alkylierungsmittels bzw. einer Säure der Formel $R_3$—A quaterniert bzw. protoniert, wobei in diesen Formeln die Benzolkerne B und C und X, X', Y, Y', $R_1$, $R_1'$, $R_2$, $R_2'$, $R_3$, n und A die oben angegebene Bedeutung haben.

Als Quaternierungs- bzw. Protonierungsmittel $R_3$—A sind beispielsweise folgende Verbindungen geeignet: Alkylhalogenide wie Methyljodid, Aethyljodid, Aethylbromid, Butylbromid oder Benzylchlorid, Dialkylsulfate wie Dimethyl- oder Diäthylsulfat, Sulfonsäureester wie Toluol- oder Benzolsulfonsäuremethyl- oder -äthylester, Alkylenoxide wie Aethylen- oder Propylenoxid oder Epichlorhydrin, die Verbindungen der Formel

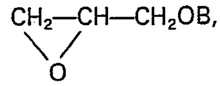

wobei B für Methyl, Aethyl, Propyl, Butyl oder Phenyl steht. Phosphite oder Phosphonate der Formel

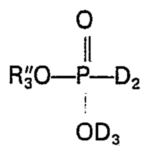

worin $R_3''$ Alkyl mit 1—4 C-Atomen und $D_2$ Wasserstoff oder unsubstituiertes oder durch Hydroxy, Cyano, Alkylcarbonyloxy oder Alkoxycarbonyl mit jeweils 1 bis 4 C-Atomen im Alkylteil substituiertes Alkyl und $D_3$ Alkyl mit 1 bis 4 C-Atomen bedeuten.

Die Quaternierung der Verbindungen der Formel (16) mit Alkylhalogeniden, Dialkylsulfaten oder Sulfosäureester zu den Verbindungen der Formel (1) wird zweckmässigerweise in einem gegenüber dem Alkylierungsmittel inerten Lösungsmittel vorgenommen. Geeignete Lösungsmittel sind z.B. Kohlenwasserstoffe wie Benzol, Toluol und Xylol, halogenierte aliphatische oder aromatische Kohlenwasserstoffe wie Chloroform, Aethylenchlorid, Chlorbenzol und Dichlorbenzol, Alkohole wie Aethanol, Butanol, Aethylenglykol und Aethylenglykolmonomethyläther, Aether wie Aethylenglykoldimethyläther und Dioxan oder Amide wie Dimethylformamid und N-Methylpyrrolidon. Zuweilen erweist es sich auch vorteilhaft, das Quaternierungsmittel als Lösungsmittel zu verwenden. Die Quaternierung mit den genannten Alkylierungsmitteln erfolgt vorteilhaft bei Temperaturen zwischen 0 und 180°C, vorzugsweise bei 30 bis 140°C.

Die Quaternierung der Verbindungen der Formel (16) zu den Verbindungen der Formel (1) mit Alkylenoxiden, Epichlorhydrin sowie dessen Derivaten der Formel

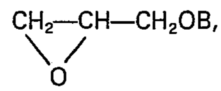

in der B die genannten Bedeutungen hat wird bei den genannten Temperaturen in saurem Medium, vorteilhaft in Gegenwart einer organischen Säure wie Ameisensäure, Essigsäure, Propionsäure oder Milchsäure durchgeführt, jedoch können auch anorganische Säuren wie Schwefelsäure, Phosphorsäure oder Halogenwasserstoffsäuren dafür verwendet werden. Diese anorganischen Säuren können in konzentrierter, handelsüblicher Form als verdünnte wässrige Lösungen oder in Mischung mit den genannten organischen Lösungsmitteln, gegebenenfalls unter Zusatz von Wasser, verwendet werden. Erfolgt die Umsetzung in Gegenwart von organischen Säuren, so wird meistens die konzentrierte Form dieser Säuren angewandt, gegebenenfalls in Mischung mit den genannten organischen Lösungsmitteln.

Bevorzugte Phosphite bzw. Phosphonate sind z.B. Dimethylphosphit, Diäthylphosphit, Dimethylmethanphosphonat, Diäthylmethanphosphonat, Methyl-äthyl-methanphosphonat, Methyl-propyl-methanphosphonat, Methyl-butyl-methanphosphonat, Methyl-hexyl-methanphosphonat, Methyl-octyl-methanphosphonat, Methyl-decyl-methanphosphonat, Methyldodecylmethanphosphonat, Dimethyl-β-hydroxyäthanphosphonat, Dimethyl-β-acetoxyäthanphosphonat, Dimethyl-β-methoxycarbonyläthan-phosphonat, Dimethyl-β-cyanäthan-phosphonat. Die Umsetzung erfolgt in Wasser und/oder organischen Lösungsmitteln wie Methanol, Aethanol, Propanol, Isopropanol, Butanol, Glykol, Glykol-methyläther, Glykol-dimethyläther, Glykolbutyläther, Diglykolmethyläther, Methyläthylketon, Methylbutylketon, Dimethylformamid, Sulfolan, Oxypropionitril, Toluol, Xylol, Benzylalkohol, Phenoxyäthanol, Benzyloxypropionitril

bei vorzugsweise 60 bis 190°C. Bei der Verwendung von flüssigen Quaternierungsmittel kann die Umsetzung auch in Abwesenheit eines zusätzlichen Lösungsmittels vorgenommen werden.

Werden protonierte Verbindungen der Formel (1) gewünscht, d.h. Säure-Additionssalze, so verwendet man als Protonierungsmittel insbesondere Mineralsäuren. Geeignet sind prinzipiell alle starken bis mittelstarken organischen Säuren oder Mineralsäuren.

Als Lösungsmittel, in denen die Protonierung vorgenommen werden kann, eignen sich im allgemeinen alle inerten Lösungsmittel. Bevorzugt sind solche, die das Ausgangsprodukt lösen und aus denen sich das Endprodukt sofort ausscheidet. Beispielsweise seien genannt: Aromatische Kohlenwasserstoffe wie Benzol, Toluol und Xylol; Halogenkohlenwasserstoffe wie Trichloräthan, Tetrachloräthylen, Chlorbenzol oder Dichlorbenzol, ferner auch Nitroverbindungen wie Nitromethan, Nitropropan, Nitrobenzol, Alkanole und offene oder cyclische Aether wie Butanol, Dibutyläther, Aethylenglykol, Aethylenglykolmonomethyläther, Aethylenglykolmonoäthyläther, Anisol oder Dioxan; Ketone wie Cyclohexanon oder Methyläthylketon; Fettsäureamide wie Dimethylformamid oder Dimethylacetamid; Sulfoxyde wie Dimethylsulfoxyd und Carbonsäureester wie Essigester oder Essigsäure-butylester.

Distyrylbiphenyle der Formel (1), worin $X = X'$ die Gruppierung —OCO— und $n = n'$ die Zahl 1 bedeutet, können z.B. aus den entsprechenden Phenolen hergestellt werden, indem man diese mit einem Halogenacylhalogenid umsetzt und das erhaltene Reaktionsprodukt mit einem Trialkylamin bzw. Pyridin der Formel $N(R_1)(R_2)(R_3)$ quaterniert, wobei $Y = Y'$, $R_1 = R_1'$, $R_2 = R_2'$ und $R_3$ die vorangehend angegebene Bedeutung haben. Die Quaternierung erfolgt vorteilhaft in einem inerten Lösungsmittel mit mindestens 2 Mol des Amins bei 50 bis 160°C vorzugsweise bei 80—130°C.

Die vorstehend definierten neuen Verbindungen zeigen in gelöstem oder feinverteiltem Zustande eine mehr oder weniger ausgeprägte Fluoreszenz. Sie können zum optischen Aufhellen der verschiedensten synthetischen, halbsynthethischen oder natürlichen organischen Materialien oder Substanzen, welche solche organische Materialien enthalten, verwendet werden. Als organische Materialien kommen vorzugsweise Polyacrylnitril und Cellulose in Betracht.

Die optisch aufzuhellenden organischen Materialien können den verschiedenartigsten Verarbeitungszuständen (Rohstoffe, Halbfabrikate oder Fertigfabrikate) angehören.

Den erfindungsgemäss anzuwendenden Verbindungen kommt u.a. Bedeutung für die Behandlung von textilen organischen Materialien, insbesondere textilen Geweben, zu.

In Abhängigkeit vom verwendeten Aufheller-Verbindungstyp kann es sich als vorteilhaft erweisen, in neutralem oder alkalischem oder saurem Bade zu arbeiten.

Die neuen optischen Aufhellmittel gemäss vorliegender Erfindung können auch zum optischen Aufhellen von Papiermassen, unter anderem in Gegenwart von z.B. kationischen Retentionsmitteln und anderen Zusatzstoffen eingesetzt werden.

Die neuen optischen Aufhellmittel gemäss vorliegender Erfindung können in folgenden Anwendungsformen eingesetzt werden:

a) Mischungen mit Farbstoffen (Nuancierung) oder Pigmenten (Farb- oder insbesondere z.B. Weisspigmenten) oder als Zusatz zu Färbebädern;

b) in Mischungen mit Netzmitteln, Weichmachern, Quellmitteln oder Antioxydantien;

c) in Kombination mit verschiedenen Textilveredlungsverfahren, wie z.B. Flammfest-, Weichgriff-, Schmutzablöse ("anti-soiling")- oder Antistatisch-Ausrüstungen oder antimikrobiellen Ausrüstungen;

d) Einarbeiten der optischen Aufhellmittel in polymere Trägermaterialien (Polymerisations-, Polykondensations- oder Polyadditionsprodukte) in gelöster oder dispergierter Form für Anwendungen z.B. in Beschichtungs-, Imprägnier- oder Bindemitteln (Lösungen, Dispersionen, Emulsionen) für Textilien, Vliese, Papier, Leder;

e) als Zusätze zu den verschiedensten industriellen Produkten, um dieselben markfähiger zu machen (z.B. Aspektverbesserung von Seifen, Waschmitteln, Weichspül- und Textilbehandlungsmitteln, Pigmenten);

f) in Kombination mit anderen, optisch aufhellend wirkenden Substanzen;

g) in Spinnbadpräparationen, d.h. als Zusätze zu Spinnbädern, wie sie zur Gleitfähigkeitsverbesserung für die Weiterverarbeitung von Synthese-Fasern verwendet werden, oder aus einem speziellen Bad von der Verstreckung der Faser, z.B. als Nachbehandlung von nassversponnenen Polyacrylfasern im sog. Gelzustand;

h) für verschiedene Zwecke photographischer Art, wie z.B. für elektrophotographische Reproduktion oder Supersensibilisierung;

i) je nach Substitution als Laser-Farbstoffe.

Wird das Aufhellverfahren mit Textil-Behandlungs- oder Veredlungsmethoden kombiniert, so kann die kombinierte Behandlung in vielen Fällen vorteilhafterweise mit Hilfe entsprechender Beständiger Präparate erfolgen, welche die optische aufhellenden Verbindungen in solcher Konzentration enthalten, dass der gewünschte Aufhelleffekt erreicht wird.

Die Menge der erfindungsgemäss zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,0001 Gewichtsprozent, kann ein deutlicher und haltbarer Effekt erzielt werden. Es können aber auch Mengen bis zu etwa 0,8 Gewichtsprozent und gegebenenfalls bis zu etwa 2

Gewichtsprozent zur Anwendung gelangen. Für die meisten praktischen Belange sind vorzugsweise Mengen zwischen 0,0005 und 0,5 Gewichtsprozent von Interesse.

Aus verschiedenen Grüdnen ist es oft zweckmässig, die Aufheller nicht als solche, d.h. rein einzusetzen, sondern vermischt mit den verschiedensten Hilfs- und Coupiermitteln.

Die neuen optischen Aufhellmittel eignen sich besonders als Zusätze für Waschbäder oder zu Gewerbe- und Haushaltswasch- und Wäschenachbehandlungsmitteln, wobei sie in verschiedener Weise zugesetzt werden können. Zu Waschbädern werden sie zweckmässig in Form ihrer Lösungen in Wasser oder organischen Lösungsmitteln oder auch in feiner Verteilung als wässrige Dispersionen zugegeben. Zu Haushalt- oder gewerblichen Waschmitteln werden sie vorteilhaft in irgendeiner Phase des Herstellungsprozesses der Waschmittel zugesetzt. Die Zugabe kann sowohl in Form einer Lösung oder Dispersion in Wasser oder anderen Lösungsmitteln als auch ohne Hilfsmittel als trockenes Aufhellerpulver erfolgen. Man kann die Aufhellmittel beispielsweise in den waschaktiven Substanzen lösen oder mit denselben vermischen, verkneten oder vermahlen und so dem fertigen Waschmittel zumischen. Sie können jedoch auch gelöst oder vordispergiert auf das fertige Waschmittel aufgesprüht werden.

Die erfindungsgemässen Verbindungen können auch im Nachspülbad, wie es zur blossen Verleihung von Weichgriff, antistatischen Eigenschaften, Antisoileffekten und Duftnoten üblich ist, eingesetzt werden. Insbesondere eignen sie sich für den Einsatz in Wäschenachbehandlungsmitteln, welche kationische Weichmacher enthalten.

Die vorliegende Erfindung betrifft daheer auch ein Waschmittel, welches vorzugsweise in flüssiger Form vorliegt und welches neben den neuen Distyrylbiphenylen und den üblichen Zusätzen noch nicht-ionogene Tenside und kationische Textilweichmacher enthält.

Als nicht-ionogene Tenside kommen die handelsüblichen in Betracht, z.B. die wasserlöslichen Produkte, die aus der Addition eines Alkylenoxides, bzw. einer äquivalenten Verbindung mit einem rekativen Wasserstoff einer hydrophoben Verbindung erhalten werden. Die hydrophoben organischen Produkte können Heterocyclen und besonders Aliphate oder Aromate sein. Bevorzugt sind höhere aliphatische Alkohole und Alkylphenole, wobei aber auch andere, wie z.B. Carbonsäuren, Carboxamide, Mercaptane, Sulfamide usw. verwendet werden können. Bevorzugte nicht-ionogene Verbindungen sind die Additionsprodukte von Aethylenoxid mit höheren aliphatischen Alkoholen mit 6 bis 50 und mehr C-Atomen. Die Menge Aethylenoxid kann sich in weiteren Grenzen bewegen, aber im allgemeinen werden zumindest 5 Mol Aethylenoxid pro Mol hydrophober Substanz gebraucht. Anstelle von Aethylenoxid können ganz oder teilweise andere niedere Alkylenoxide, wie z.B. Propylenoxid und Butylenoxid verwendet werden. Als weitere nicht-ionogene verwendbare Verbindungen kommen in Betracht:

a) Polyoxyalkylenester organischer Säuren wie höherer Fettsäuren, Harzsäuren, Tallölsäuren und Säuren der Oxydationsprodukte des Erdöls, deren Ester in der Regel im Säureteil 10 bis 22 C-Atome aufweisen und ca. 12 bis ca. 30 Mol Aethylenoxid oder dessen Aequivalent enthalten;

b) Alkylenoxidaddukte höherer Fettsäureamide, wobei der Fettsäureteil in der Regel 8 bis 22 C-Atome aufweist und mit 10 bis 50 Mol Aethylenoxid kondensiert ist. Die entsprechenden Carboxamide und Sulfamide können ebenfalls als weitgehend äquivalent verwendet werden.

In der Herstellung von flüssigen konzentrierten Waschmitteln werden als nicht-ionogene Tenside vorzugsweise oxalkylierte höhere aliphatische Alkohole verwendet, wobei die Fettalkohole mindestens 6 und vorzugsweise mindestens 8 C-Atome vorweisen. Bevorzugte Alkohole sind Lauryl-, Myristyl-, Cetyl-, Stearyl- und Oleylalkohol, welche mit mindestens 6 Mol Aethylenoxid kondensiert werden. Als typisches nicht-ionogenes Produkt sei das Additionsprodukt von einem aliphatischen Alkohol mit 12—13 C-Atomen mit ca. 6,5 Mol Aethylenoxid erwähnt. Die entsprechenden Alkylmercaptane sind, nach Kondensation mit Aethylenoxid, ebenfalls als nicht-ionogene Tenside verwendbar.

Die alkoxylierten höheren aliphatischen Alkohole sind besonders für Haushaltswaschmittel geeignet, da sie leicht biologisch abbaubar sind und gut mit kationischen Tensiden und Textilweichmachern und den übrigen Zusätzen verträglich sind.

Von den kationischen Textilweichmachern eignen sich besonders quaternäre Derivate des Ammoniaks und/oder des Imidazolins mit 2 langkettigen aliphatischen Resten, wie z.B. das 1-Methyl-1-oleylamidoäthyl-2-oleyl-imidazolinium $\cdot X^{\ominus}$, das 1-Methyl-1-talgamidoäthyl-2-talgimidazoliunium $\cdot X^{\ominus}$, das Di-talg-dimethyl-ammonium $\cdot X^{\ominus}$ und eine Verbindung der Formel

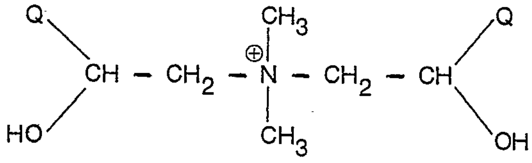

$\cdot X^{\ominus}$

worin Q $C_{14-16}$-Alkyl bedeutet und $X^{\ominus}$ für ein Chlorid-, Bromid-, Methylsulfat-, Aethylsulfat, Methan-, Aethan- oder Toluolsulfonatanion steht.

Die erfindungsgemäss verwendbaren quaternären Textilweichmacher und besonders die vorstehend genannten verleihen dem Gewebe einen weichen und flaumigen Griff und gleichzeitig eine gute Wiederanfeuchtbarkeit. Diese Textilweichmacher sind substantiv zum Gewebe und tragen zur

Verminderung der statischen Aufladung und der Neigung zum Knittern bei, so dass das Gewebe leichter gebügelt werden kann und angenehmer zu tragen ist.

Das flüssige Medium für die erfindungsgemässen Waschmittel ist wässerig und kann aus Wasser allein oder aus Wasser und zusätzlichen Lösungsmitteln für gewisse Zusätze bestehen. Die zusätzlichen Lösungsmittel können bis zu 20, vorzugsweise bis 15% des gesamten Lösungsmittelanteils ausmachen. Als solche kommen in Betracht: niedere Alkanole oder ein niederes Diol oder Polyol, wie z.B. Aethanol, Isopropanol, Aethylenglykol, Propylenglykol und Glycerin. Auch verätherte Polyole, wie Diäthylenglykol, Aethylenglykoldimethyläther und Aethylenglykolmonoäthyläther können als zusätzliche Lösungsmittel verwendet werden.

Das erfindungsgemässe flüssige Waschmittel kann verschiedene ausgewählte verträgliche Zusätze enthalten, wie Schmutzsuspendiermittel oder Vergrauungsinhibitoren, z.B. Polyvinylalkohol, Hydroxypropylmethylcellulose; Schauminhibitoren, Konservierungsmittel, z.B. Natriumbenzoat; UV-Absorber und Parfüme. Diese werden selbstverständlich so gewählt, dass sie mit den Hauptkomponenten des Waschmittels verträglich sind.

Die nicht-ionogenen Tenside werden in Mengen von 10 bis 70 Gewichts-%, vorzugsweise 60 Gewichts-% eingesetzt. Die Konzentration des Textilweichmachers beträgt 1 bis 30 Gewichts-%, vorzugsweise 21 Gewichts-%. Das wässerige Lösungsmittel, vorzugsweise Wasser, das noch mono-, di- und mehrwertige Alkohole und ähnliche Lösungsmittel enthalten kann, beträgt 5 bis 60 Gewichts-%. Das flüssige oder pulverförmige, fertige Waschmittel enthält die erfindungsgemässen Verbindungen in Mengen von 0,005 bis 3 Gewichts-%. Der Gehalt an den übrigen Hilfsmitteln beträgt vorzugsweise weniger als 5 Gewichts-% des Waschmittels, da die Verwendung von grösseren Mengen die Eigenschaften flüssiger Waschmittel beeinflussen kann. Obschon die bevorzugte anmeldungsgemässe waschaktive Zubereitung eine stabile, klare Flüssigkeit ist, kann man ihr ein verträgliches Trübungsmittel hinzugeben, um einen opaken Aspekt hervorzurufen.

Das erfindungsgemässe Waschmittel kann in weichem oder angemessen hartem Wasser bei höherer Temperatur zur Anwendung gelangen. Dieses Waschmittel kann auch zum Waschen von Textilien in sehr hartem Wasser bei tieferer Temperatur verwendet werden. Die Wasserhärte kann deshalb von 0 bis über 300 ppm, berechnet als Calciumcarbonat schwanken und die Waschtemperatur kann 4 bis 60°C betragen.

Das erfindungsgemässe Waschmittel löst sich sehr leicht in kaltem oder warmem Waschwasser, reinigt gründlich, eliminiert die statische Aufladung und macht die Wäsche weich, ohne sie zu hydrophobieren. Das bevorzugte Waschmittel liegt als eine klare, stabile Flüssigkeit vor, die ihre Aktivität und Uniformität über eine längere Zeitspanne behält. Zur Herstellung von klaren flüssigen Waschmitteln kann die Konzentration der Aktivsubstanzen nur innerhalb gewisser Grenzen variiert werden. So soll z.B. die Konzentration des Textilweichmachers nicht viel höher sein als 30%, wenn man ein klar flüssiges Waschmittel erhalten will.

Die erfindungsgemässen Verbindungen werden in Mengen von 0,005 bis 1% oder mehr, bezogen auf das Gewicht des flüssigen oder pulverförmigen, fertigen Waschmittels resp. Textilbehandlungsmittels, zugesetzt. Wasch-/Behandlungsflotten, die die angegebenen Mengen der beanspruchten Aufheller enthalten, verleihen beim Waschen von Textilien aus Cellulosefasern, Polyamidfasern, hochveredelten Cellulosefasern, Polyesterfasern, Wolle etc. diesen einen brillanten Aspekt am Tageslicht.

Die Waschbehandlung wird beispielsweise wie folgt durchgeführt: Die angegebenen Textilien werden während 1 bis 30 Minuten bei 20 bis 100°C in einem Waschbad behandelt, das 0,1 bis 10 g/kg eines aufgebauten, zusammengesetzten Waschmittels und 0,05 bis 1%, bezogen auf das Waschmittelgewicht, der beanspruchten Aufhellmittel enthält. Das Flottenverhältnis kann 1:3 bis 1:50 betragen. Nach dem Waschen wird wie üblich gespült und getrocknet.

In den Beispielen sind Teile, soweit nicht anders angegeben, immer Gewichtsteile und Prozente immer Gewichtsprozente. Schmelz- und Siedepunkte sind, sofern nicht anders vermerkt, unkorrigiert und oft unscharf, besonders bei den quaternierten Verbindungen.

Beispiel 1

Zu einer Lösung von 20,6 g 4,4'-Bis-(dimethoxy-phosphonomethyl)-biphenyl (Gehalt 96,9%) und 24,2 g der Verbindung der Formel

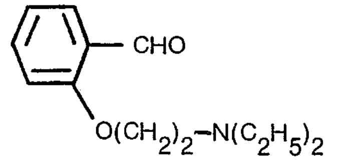

(100)

(Gehalt 96%) in 230 ml Dimethylformamid tropft man nach Verdrängen der Luft durch Stickstoff unter Rühren 22,2 g einer methanolischen 30,4%igen Natriummethylatlösung, so dass die Temperatur nicht über 40°C ansteigt. Man hält die Temperatur 2 Stunden bei 40—45°C, kühlt mit Eiswasser ab und versetzt die Reaktionsmischung mit 23 ml Wasser. Das ausgefallene Produkt wird abgenutscht, wiederholt mit Methanol und Wasser gewaschen und im Vakuum bei 90° getrocknet. Man erhält 27,6 g der Verbindung der Formel

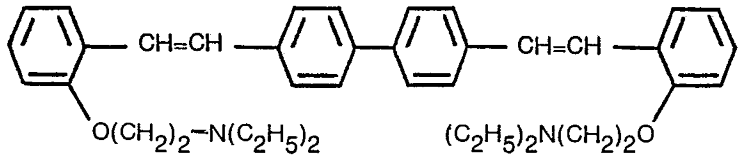

(101)

in Form leuchtend, hellgelber Kristalle vom Smp. 127—128°C (nach Umkristallisation aus Nonan und n-Propanol).

Die Verbindung der Formel (100) kann wie folgt hergestellt werden: Zu einer Suspension von 244 g Salicylaldehyd und 344 g 2-Diäthylamino-äthylchlorid-hydrochlorid in 2000 ml Chlorbenzol tropft man nach Verdrängen der Luft durch Stickstoff unter Rühren 732 g einer methanolischen 30,7%igen Natriummethylat-Lösung, wobei sich die Suspension gelb färbt und dick wird. Man steigert die Temperatur allmählich bis etwa 131°C und destilliert dabei das Methanol ab. Man belässt noch 4 Stunden bei dieser Temperatur, lässt abkühlen, fügt 500 ml Wasser hinzu, trennt die beiden Schichten und trocknet die organische Phase mit Natriumsulfat. Das Lösungsmittel wird alsdann im Wasserstrahlvakuum abgedampft und der Rückstand im Hochvakuum fraktioniert destilliert. Nach Abtrennung eines geringen Vorlaufes erhält man 327,5 g einer leicht rötlichen Flüssigkeit vom Kp. 105—118°/0,06 mbar mit einem Gehalt von 96%.

In analoger Weise erhält man durch Umsetzung von 4,4'-Bis(di-methoxyphosphonomethyl)-biphenyl mit entsprechenden Aldehyden die in Tabelle I aufgeführten Verbindungen der Formel

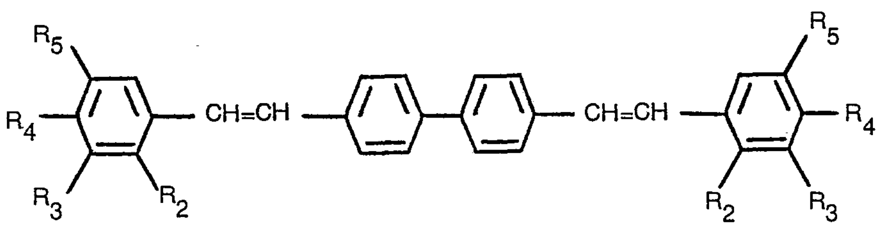

(102)

TABELLE I

| Formel | $R_2$ | $R_3$ | $R_4$ | $R_5$ | Smp. (°C) |
|---|---|---|---|---|---|
| (103) | $—O(CH_2)_3—N(CH_3)_2$ | H | H | H | 131° |
| (104) | $—O(CH_2)_2—N(CH_3)_2$ | H | H | H | 165° |
| (105) | 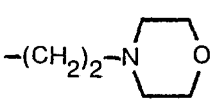 | H | H | H | 158° |
| (106) | 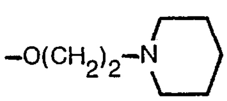 | H | H | H | 142° |
| (107) | 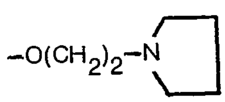 | H | H | H | 148° |
| (108) | $—O(CH_2)_3—N(C_2H_5)_2$ | H | H | H | 135° |
| (109) | $—O(CH_2)_3—N(CH_3)_2$ | $—OCH_3$ | H | H | 93° |
| (110) | $—O(CH_2)_3—N(CH_3)_2$ | H | H | Cl | 132° |
| (111) | $—O(CH_2)_3—N(CH_3)_2$ | H | H | $CH_3$ | 108° |
| (112) | H | $—O(CH_2)_2—N(C_2H_5)_2$ | H | H | 174° |

TABELLE 1 (Fortsetzung)

| Formel | $R_2$ | $R_3$ | $R_4$ | $R_5$ | Smp. (°C) |
|---|---|---|---|---|---|
| (113) | H | H | $—O(CH_2)_2—N(C_2H_5)_2$ | H | 291° |
| (114) | $—S(CH_2)_2—N(CH_3)_2$ | H | H | H | 145° |
| (115) | 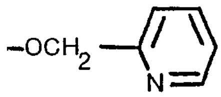 | H | H | H | 239° |
| (116) | $—CH_2N(CH_3)_2$ | H | H | H | 164° |

Der zur Herstellung der Verbindung der Formel (114) benötigte Aldehyd der Formel

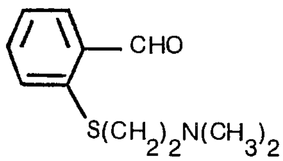

(117)

kann wie folgt hergestellt werden: Zu einer Mischung von 61,7 g Kaliumhydroxid 88%ig in 54,3 g Wasser und 60 ml Dimethylsulfoxid fügt man nach Verdrängen der Luft durch Stickstoff unter heftigem Rühren und Kühlen 100 g Dimethylamino-äthanthiol-hydrochlorid ca 85%ig hinzu, so dass die Temperatur nicht über 20°C ansteigt. Alsdann lässt man 57,2 g o-Chlorbenzaldehyd eintropfen und hält die Temperatur je 2 Stunden bei 60°C und dann bei 80°C. Nach dem Abkühlen auf Raumtemperatur versetzt man mit 300 ml Methylenchlorid und 300 ml Wasser, mischt durch, lässt die beiden Schichten abtrennen und trocknet die organische Phase mit Natriumsulfat. Das Lösungsmittel wird abgedampft und der Rückstand im Hochvakuum fraktioniert destilliert. Nach Abtrennung eines Vorlaufes erhält man 18,6 g einer gelblichen Flüssigkeit vom Kp. 97—106°C/0,03 mbar mit einem Gehalt von 79,3%.

Beispiel 2

Verwendet man in Beispiel 1 anstelle eines einheitlichen Aldehyds eine Mischung (200) der beiden isomeren Aldehyde

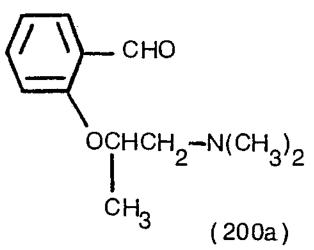

(200a)

und

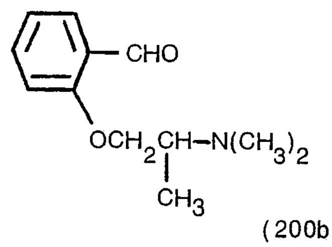

(200b)

so erhält man eine Isomerenmischung (201) der beiden symmetrischen Verbindungen der Formeln (201a) und (201b) und der asymmetrischen Verbindung der Formel (201c) von Smp. 122—130°C (nach Umkristallisation aus Nonan):

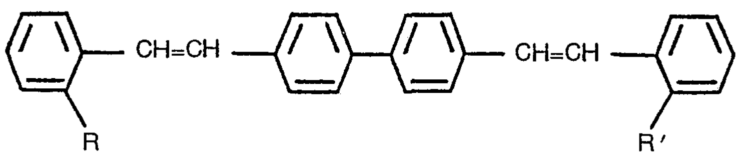

R = R' = $—OCHCH_2—N(CH_3)_2$ (mit $CH_3$ am CH) (201a)

R = R' = $—OCH_2CH—N(CH_3)_2$ (mit $CH_3$ am CH) (201b)

$R = —OCHCH_2—N(CH_3)_2$ (201c) $R' = —OCH_2CH—N(CH_3)_2$

$CH_3$ $CH_3$

Die Isomerenmischung der Aldehyde der Formel (200) erhält man durch Umsetzung von Salicylaldehyd mit 2-Chlor-1-dimethylaminopropanhydrochlorid analog Beispiel 1, Formel (100).

Beispiel 3

20,6 4,4'-Bis-(dimethoxyphosphonomethyl)-biphenyl (Gehalt 96,9%) und 20,9 g der Verbindung der Formel

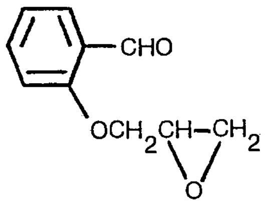

(300)

(Gehalt 89,9%) werden gemäss Beispiel 1 in 150 ml Dimethylformamid umgesetzt. Nach Umkristallisation aus Xylol erhält man 22,5 g der Verbindung der Formel

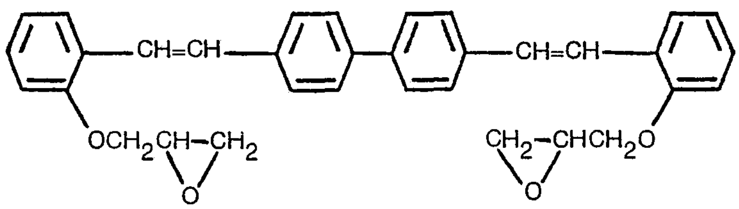

(301)

in Form hellgelber Kristalle von Smp. 208—210°C.

8,05 g dieser Verbindung werden in 30 ml Dimethylformamid bei 115°C unter Rühren gelöst und zur Lösung 30 ml alkoholisches 33%iges Dimethylamin zugetropft, wobei man die Temperatur auf 80°C sinken lässt. Man rührt noch 2 Stunden bei 80°C nach, kühlt ab und versetzt mit 60 ml Wasser. Das ausgefallene Produkt wird abgenutscht, wiederholt mit Wasser gewaschen und bei 100°C im Vakuum getrocknet. Man erhält 9,3 g der Verbindung der Formel

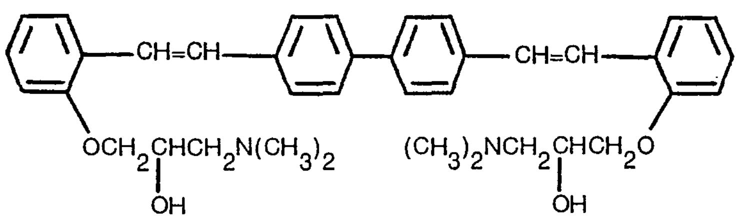

(302)

in Form blass-gelber Kristalle von Smp. 140—142°C (nach Umkristallisation aus Nonan).

Beispiel 4

16,3 g der Verbindung der Formel

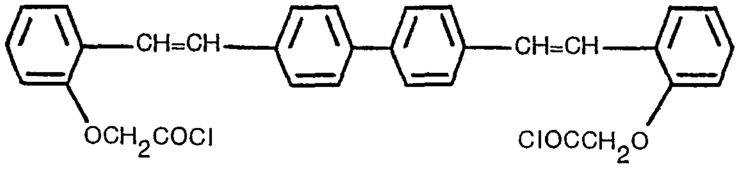

(400)

(DE—OS 2,209,128) und 25,5 g 3-Dimethylaminopropyl-amin-(1) werden in 250 ml Chlorbenzol bei Rückflusstemperatur verrührt. Die Lösung wird mit Bleicherde heiss klärfiltriert, eingeengt und abkühlen gelassen.

Das ausgeschiedene Produkt wird abgenutscht, mit Chlorbenzol gewaschen und getrocknet. Man erhält 17,4 g der aus US—A—3 927 119 bekannten Verbindung der Formel

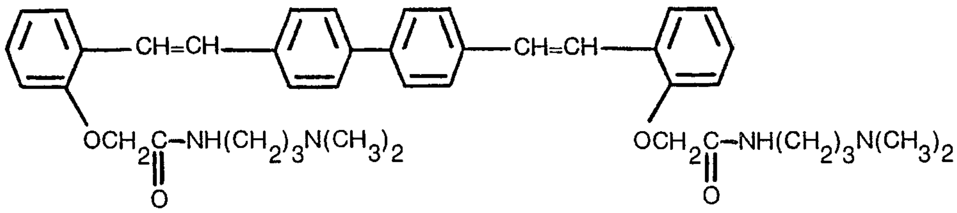

(401)

von Smp. 226—227°C.

Setzt man die Verbindung der Formel (400) mit 2-Dimethylaminoäthanol gemäss Beispiel 14 um, so erhält man die Verbindung der Formel

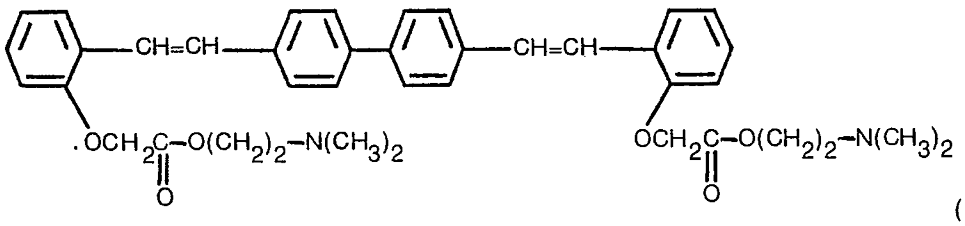

(402)

die in Dimethylformamid gelöst violettblaue Tageslichtfluoreszenz zeigt.

Beispiel 5

In ähnlicher Weise wie in Beispiel 1 beschreiben, erhält man die in Tabelle II aufgeführten Verbindungen der Formel

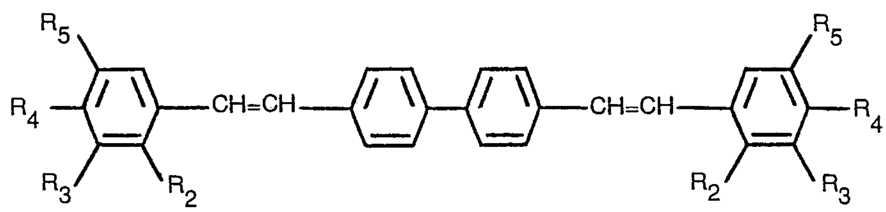

(500)

TABELLE II

| Formel | $R_1$ | $R_2$ | $R_3$ | $R_4$ | $R_5$ |
|---|---|---|---|---|---|
| (501) | H | —Cl | $—O(CH_2)_2—N(C_2H_5)_2$ | H | H |
| (502) | H | —Cl | H | $—O(CH_2)_2—N(C_2H_5)_2$ | H |
| (503) | H | $—O(CH_2)_2—N(C_2H_5)_2$ | —Cl | H | —Cl |
| (504) | H | $—O(CH_2)_2—N(C_2H_5)_2$ | H | $—(CH_2)_3—$ | |
| (505) | H | $—O(CH_2)_2—N(C_2H_5)_2$ | H | $—(CH_2)_4—$ | |
| (506) | H | H | H | $—O(CH_2)_3—N(CH_3)_2$ | H |
| (507) | H | $—O(CH_2)_2—N(C_2H_5)$ | $—CH_2CH=CH_2$ | H | H |
| (508) | H | $CH_3$ | H | $—O(CH_2)_2—N(C_2H_5)_2$ | $—CH(CH_3)_2$ |
| (509) | H | $—O(CH_2)_2—N(C_2H_5)_2$ | $—CH_3$ | H | $—CH_3$ |
| (510) | H | $—O(CH_2)_2—N(C_2H_5)_2$ | H | $CH_3$ | —Cl |

Beispiel 6

Zu einer Lösung von 5,9 g der Verbindung der Formel (101) in 50 ml Methyläthylketon tropft man unter Rühren bei etwa 70°C 2,5 ml Dimethylsulfat. Man erhitzt noch 1 Stunde bei Rückflusstemperatur, lässt Abkühlen, nutscht das ausgefallene Produkt ab und wäscht wiederholt mit Methyläthylketon. Nach dem Trocknen im Vakuum bei 100°C erhält man 8,2 g der Verbindung der Formel

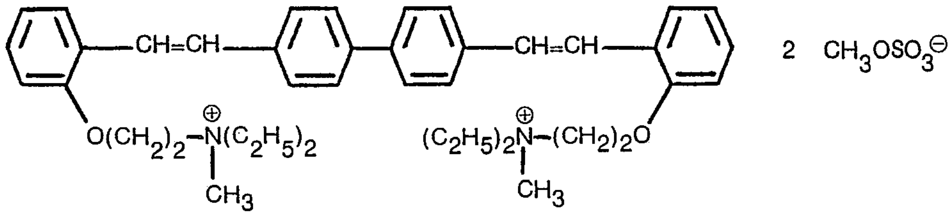

(600)

die noch etwa 1/2 Mol Kristallwasser enthält, in From blassgelber Kristalle vom Smp. 235—238°C.

Anstelle von Methyläthylketon kann auch Chlorbenzol als Lösungsmittel verwendet werden.

In analoger Weise erhält man aus den in Tabelle I beschriebenen Verbindungen und denjenigen der Formel (302) und (401) die in folgender Tabelle III aufgeführten Verbindungen der Formel

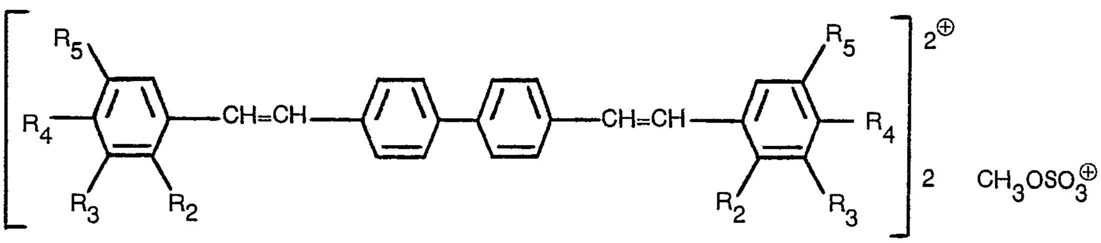

(601)

TABELLE III

| Formel | $R_2$ | $R_3$ | $R_4$ | $R_5$ | Smp. (°C) |
|---|---|---|---|---|---|
| (602) | $-O(CH_2)_3-N(CH_3)_3$ | H | H | H | 263° |
| (603) | $-O(CH_2)_2-N(CH_3)_3$ | H | H | H | 285° |
| (604) | 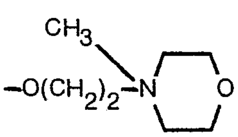 | H | H | H | 227° |
| (605) | 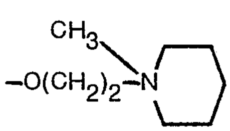 | H | H | H | 197° |
| (606) | 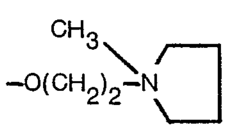 | H | H | H | 244° |
| (607) | $CH_3$<br>$-O(CH_2)_3-N(C_2H_5)_2$ | H | H | H | 229° |

TABELLE III (Fortsetzung)

| Formel | $R_2$ | $R_3$ | $R_4$ | $R_5$ | Smp. (°C) |
|---|---|---|---|---|---|
| (608) | OH<br>\|<br>$—OCH_2CHCH_2N(CH_3)_3$ | H | H | H | 234° |
| (609) | $—O(CH_2)_3—N(CH_3)_3$ | $—OCH_3$ | H | H | 264° |
| (610) | $—O(CH_2)_3—N(CH_3)_3$ | H | H | Cl | 289° |
| (611) | $—O(CH_2)_3—N(CH_3)_3$ | H | H | $CH_3$ | 236° |
| (612) | H | $CH_3$<br>\|<br>$—O(CH_2)_2—N(C_2H_5)_2$ | H | H | 210° |
| (613) | H | H | $CH_3$<br>\|<br>$—O(CH_2)_2—N(C_2H_5)_2$ | H | 235° |
| (614) | $—S(CH_2)_2—N(CH_3)_3$ | H | H | H | 283° |
| (615) | 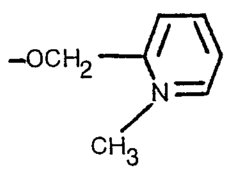 | H | H | H | 247° |
| (616) | $—CH_2N(CH_3)_3$ | H | H | H | 274° |
| (617) | O<br>‖<br>$—OCH_2C—NH(CH_2)_3—N(CH_3)_3$ | H | H | H | 228° |

Zur Herstellung der Verbindungen der Formeln (613) und (617) verwendet man anstelle von Methyläthylketon zum Lösen der Ausgangsprodukte Chloroform bzw. Dioxan und arbeitet bei Rückflusstemperatur.

## Beispiel 7

Quaterniert man gemäss Beispiel 6 die Isomerenmischung der Formel (201), so erhält man die Isomerenmischung der Formel (700), die sich aus den beiden symmetrischen Verbindungen (700a) und (700b), sowie der asymmetrischen Verbindung der Formel (700c) zusammensetzt

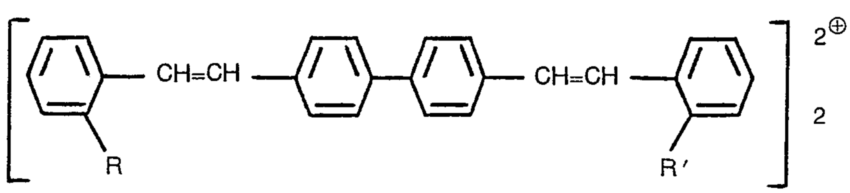

2 $CH_3OSO_3^{\ominus}$

$R = R' = —OCHCH_2—N(CH_3)_3$ (mit $CH_3$ an CH) (700a)

$R = R' = —OCH_2CH—N(CH_3)_3$ (mit $CH_3$ an CH) (700b)

$R = —OCHCH_2—N(CH_3)_3$ (mit $CH_3$ an CH) (700c) $R' = —OCH_2CH—N(CH_3)_3$ (mit $CH_3$ an CH)

und etwa 1 Mol Kristallwasser enthält. Smp. 272—285°C.

Beispiel 8

Zu einer Lösung von 5,6 g der Verbindung der Formel (103) in 50 ml Methyläthylketon tropft man unter Rühren bei 70°C 1,0 ml Dimethylsulfat. Man erhitzt noch 1 Stunde bei Rückflusstemperatur, nutscht heiss und wäscht dreimal mit heissem Methyläthylketon. Man erhält eine Mischung der symmetrischen Verbindung der Formel (602) und der asymmetrischen Verbindung der Formel

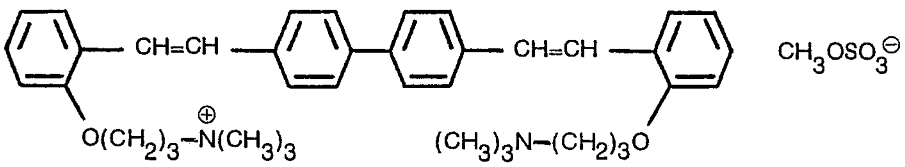

(800)

Smp. 233—240°C.

In ähnlicher Weise erhält man aus (114) eine Mischung der Verbindungen der Formeln (614) und

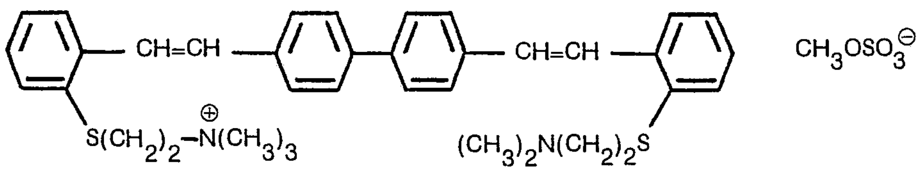

(801)

Smp. 249—252°C.

Beispiel 9

11,2 g der Verbindung der Formel (103) werden in 25 g Dimethylmethanphosphonat 2 Stunden bei 120°C verrührt, wobei das Endprodukt auskristallisiert. Man verdünnt mit 25 ml Methyläthylketon, lässt abkühlen, nutscht und wäscht den Rückstand wiederholt mit Methyläthylketon. Nach dem Trocknen im Vakuum bei 100°C erhält man 13,1 g der Verbindung der Formel

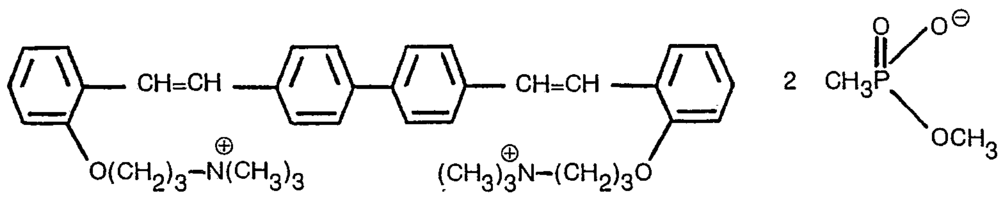

,

(900)

welche 1/2 Mol Kristallwasser enthält, vom Smp. 243°C. (Zers.).

In ähnlicher Weise erhält man aus (104) bzw. (101) die Verbindung der Formel

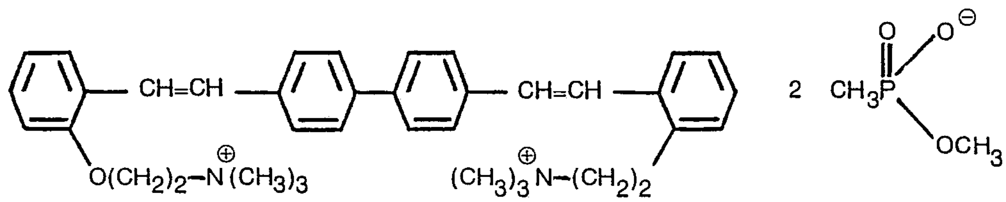

(901)

die mit etwa 2 Kristallwasser kristallisiert. Smp. 222—235°C, und

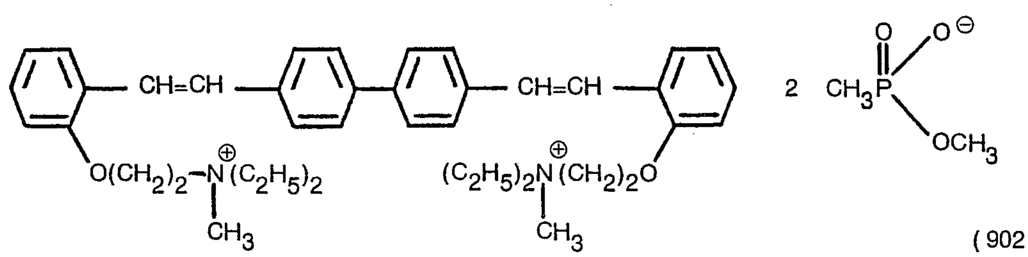

(902)

die mit etwa 1 1/2 Kristallwasser kristallisiert. Smp. 165—178°C.

Beispiel 10

In eine Lösung von 5,4 g der Verbindung der Formel

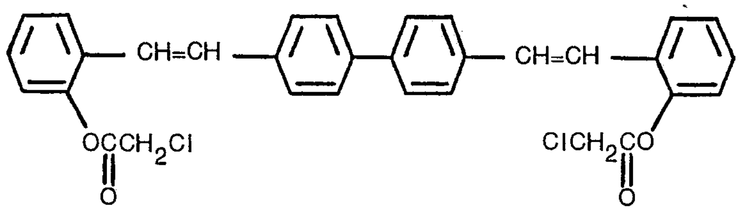

(1000)

in 100 ml Chlorbenzol leitet man bei 120°C Trimethylamin bis zur Sättigung ein (ca. 10 Minuten). Man lässt auf Raumtemperatur abkühlen, nutscht das ausgefallene Produkt ab, wäscht wiederholt mit Chlorbenzol und Methyläthylketon und trocknet im Vakuum bei 100°C. Man erhält 6,5 g der Verbindung der Formel

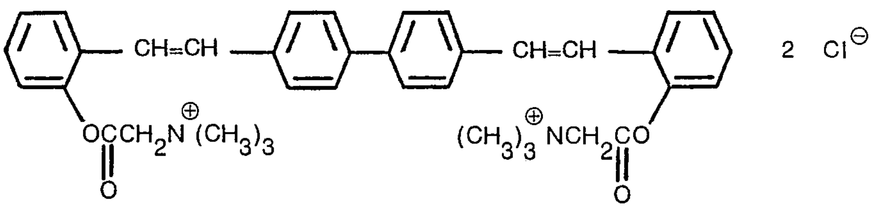

(1001)

vom Smp. 175°C. (Zers.)

Das Ausgangsprodukt der Formel (1000) erhält man wie folgt:

Zu einer Lösung von 42,0 g Biphenyl-4,4'-dialdehyd und 197,7 g der Verbindung der Formel

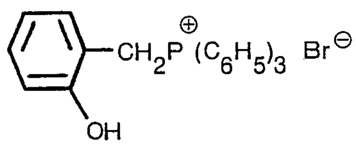

(1002)

in 600 ml Dimethylformamid tropft man nach Verdrängen der Luft mit Stickstoff unter Rühren 159,3 g einer methanolischen 30,5%igen Natriummethylatlösung, so dass die Temperatur nicht über 40°C ansteigt. Man hält die Temperatur 2 Stunden bei 40—45°C, lässt abkühlen und giesst die Reaktionsmischung unter Rühren in 3000 ml Wasser. Durch Einleiten von Kohlendioxid bis zur Sättigung oder durch Zugabe von Trockeneis neutralisiert man auf pH 8, wobei das Reaktionsprodukt harzig ausfällt. Man dekantiert die wässerige Phase, verrührt das Reaktionsprodukt mit 3000 ml Wasser, nutscht und wäscht wiederholt mit Wasser. Der feuchte Rückstand wird in 1000 ml siedendem Methyläthylketon aufgenommen, die Lösung mit 0,4 g Bleicherde heiss klärfiltriert und auf die Hälfte eingedampft. Man kühlt auf 0°C ab, nutscht das auskristallisierte Produkt ab, wäscht zweimal mit je 50 ml gekühltem Methyläthylketon und trocknet im Vakuum bei 100°C. Man erhält 34,7 g der Verbindung der Formel

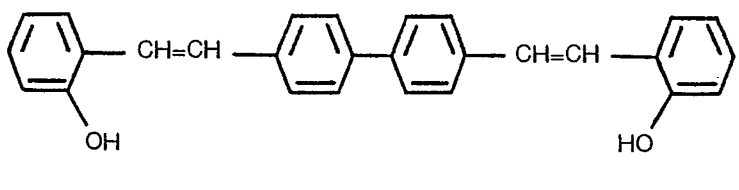

(1003)

Smp. 280—287° (nach Umkristallisation aus Methyläthylketon.

15,6 g der Verbindung der Formel (1003) werden in 40 ml Chloracetylchlorid über Nacht unter Rückfluss verrührt, wobei zuletzt vollständige Lösung eintritt. Man versetzt mit 40 ml Cyclohexan, lässt abkühlen, nutscht das ausgefallene Produkt ab, wäscht es wiederholt mit Cyclohexan und trocknet es im Vakuum bei 100°C. Nach Umkristallisation aus Perchloräthylen erhält man 13,4 g der Verbindung der Formel (1000) in Form blassgelber Kristalle. Smp. 182—185°C (nach Umkristallisation aus Xylol).

Beispiel 11

Ersetzt man in Beispiel 10 die Verbindung der Formel (1000) durch das Isomere der Formel

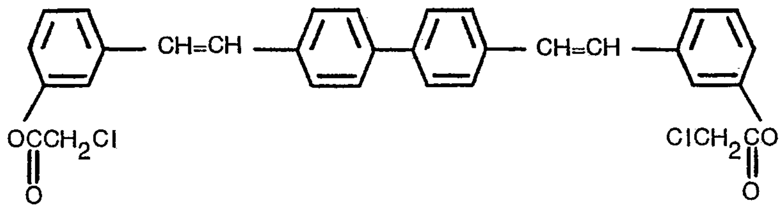

Smp. 240–241°

(aus Chlorbenzol)

(1100)

so erhält man die Verbindung der Formel

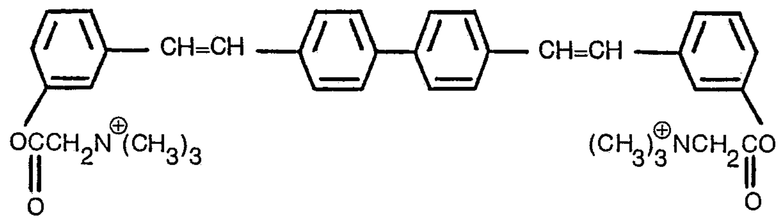

2 $Cl^{\ominus}$

Smp. 259–275 °C

(1101)

Verwendet man in diesem Beispiel anstelle von Trimethylamin Pyridin (3,2 ml), so erhält man nach einer Reaktionszeit von 2 1/2 Stunden 4,5 g der Verbindung der Formel

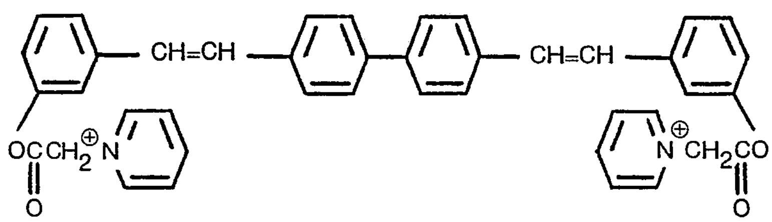

2 $Cl^{\ominus}$

Smp. 260–288 °C

(1102)

Das Ausgangsprodukt der Formel (1100) erhält man wie folgt:

Zu einer Lösung von 418 g 4,4'-Bis-(dimethoxyphosphonomethyl)biphenyl (Gehalt 96,9%) und 256,2 g 3-Hydroxybenzaldehyd in 1000 ml Dimethylformamid tropft man nach Verdrängen der Luft durch Stickstoff unter Rühren 797 g einer methanolischen 30,5%igen Natriummethylatlösung, so dass die Temperatur nicht über 40°C ansteigt. Man hält die Temperatur 4 Stunden bei 40—45°C, kühlt ab und fügt 2000 ml Wasser hinzu. Durch Einleiten von Kohlendioxid bis zur Sättigung oder durch Zugabe von Trockeneis neutralisiert man auf etwa pH 8, nutscht das ausgefallene Produkt ab, wäscht wiederholt mit Wasser und Isopropanol und trocknet im Vakuum bei 100°C (317,9 g). Nach Umkristallisation aus Dioxan erhält man die Verbindung der Formel

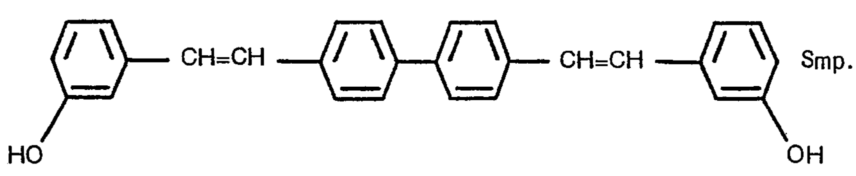

Smp. 315–325 °C

(1103)

in Form blassgelber Kristalle.

Diese wird gemäss Beispiel 10, Formel (1000) in die Verbindung der Formel (1100) übergeführt.

Beispiel 12

In ähnlicher Weise wie in den Beispielen 6, 9 oder 10 beschrieben, erhält man die in Tabelle IV aufgeführten Verbindungen der Formel

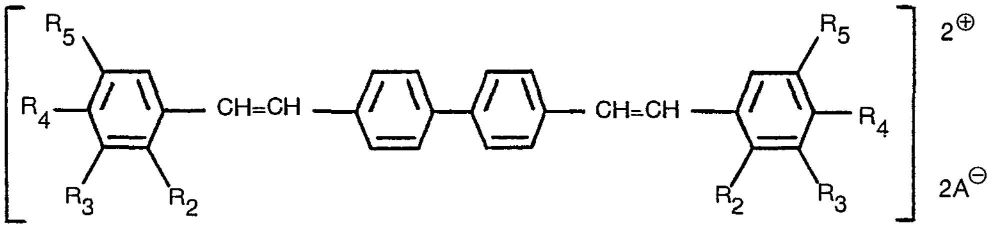

(1200)

TABELLE IV

| Formel | $R_2$ | $R_3$ | $R_4$ | $R_5$ | A |
|---|---|---|---|---|---|
| (1201) | Cl | $-O(CH_2)_2-\underset{CH_3}{\underset{\vert}{N}}(C_2H_5)_2$ | H | H | $CH_3OSO_3$ |
| (1202) | Cl | H | $-O(CH_2)_2-\underset{CH_3}{\underset{\vert}{N}}(C_2H_5)_2$ | H | " |
| (1203) | $-O(CH_2)_2-\overset{CH_3}{\overset{\vert}{N}}(C_2H_5)_2$ | $-Cl$ | H | $-Cl$ | " |
| (1204) | $-O(CH_2)_2-\overset{CH_3}{\overset{\vert}{N}}(C_2H_5)_2$ | H | $-(CH_2)_3$ | | " |
| (1205) | $-O(CH_2)_2-\underset{CH_3}{\underset{\vert}{N}}(C_2H_5)_2$ | H | $-(CH_2)_4$ | | " |
| (1206) | H | H | $-O(CH_2)_3-N(CH_3)_3$ | H | " |
| (1207) | $-O(CH_2)_2-\overset{CH_3}{\overset{\vert}{N}}(C_2H_5)_2$ | $-CH_2CH{=}CH_2$ | H | H | " |
| (1208) | $-CH_3$ | H | $-O(CH_2)_2-\overset{CH_3}{\overset{\vert}{N}}(C_2H_5)_2$ | $-CH\begin{smallmatrix}CH_3\\CH_3\end{smallmatrix}$ | " |
| (1209) | $-O(CH)_2-\overset{CH_3}{\overset{\vert}{N}}(C_2H_5)_2$ | $-CH_3$ | H | $-CH_3$ | " |
| (1210) | $-O(CH_2)_2-\overset{CH_3}{\overset{\vert}{N}}(C_2H_5)_2$ | H | $-CH_3$ | $-Cl$ | " |

TABELLE IV (Fortsetzung)

| Formel | $R_2$ | $R_3$ | $R_4$ | $R_5$ | A |
|---|---|---|---|---|---|
| (1211) | $-OCH_2\overset{O}{\overset{\|}{C}}O(CH_2)_2-N(CH_3)_3$ | H | H | H | $CH_3OSO_3$ |
| (1212) | $-O(CH_2)_2-N(C_2H_5)_3$ | H | H | H | Br |
| (1213) | $-O(CH_2)_2-N(C_2H_5)_2$<br>\|<br>$CH_2C_6H_5$ | H | H | H | Cl |
| (1214) | $-O(CH_2)_2-N(C_2H_5)_2$<br>\|<br>$CH_2COOCH_3$ | H | H | H | Cl |
| (1215) | $-O(CH_2)_2-N(C_2H_5)_2$<br>\|<br>$CH_3$ | H | H | H | 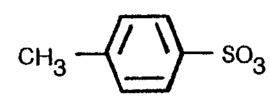 |
| (1216) | $-O(CH_2)_2-N(C_2H_5)_2$<br>\|<br>$CH_2CN$ | H | H | H | Cl |
| (1217) | $-O(CH_2)_3-N(CH_3)_2$<br>\|<br>$C_2H_5$ | H | H | H | $C_2H_5OSO_3$ |
| (1218) | H | H | $-CH_2N(CH_3)_3$ | H | Cl |
| (1219) | $-O(CH_2)_3-N(CH_3)_2$<br>\|<br>$CH_2CH{=}CH_2$ | H | H | H | Cl |
| (1220) | $-O(CH_2)_3-N(CH_3)_2$<br>\|<br>$CH_2CH_2OH$ | H | H | H | $CH_3COO$ |
| (1221) | $-O(CH_2)_3N(CH_3)_2$<br>\|<br>$CH_2CH-CH_3$<br>\|<br>OH | H | H | H | HCOO |

Beispiel 13

6,1 g der Verbindung der Formel

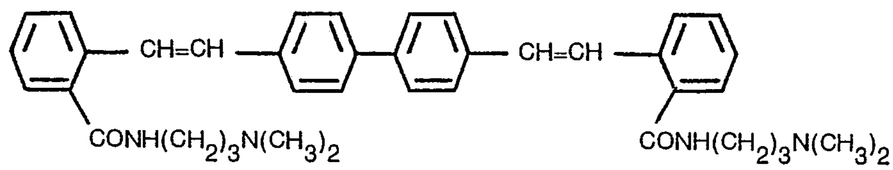

(1300)

werden unter Rühren bei 45°C in 250 ml Methyläthylketon suspendiert. Nach Zugabe von 2,5 ml Dimethylsulfat wird auf Siedetemperatur erhitzt und das Reaktionsgemisch während 1 1/2 Stunden unter Rückfluss gekocht. Nach dem Abkühlen auf Raumtemperatur wird das auskristallisierte Produkt abgenutscht, mit Methyläthylketon gewaschen und bei 60—70°C unter Vakuum getrocknet. Man erhält so 8,1 g der Verbindung der Formel

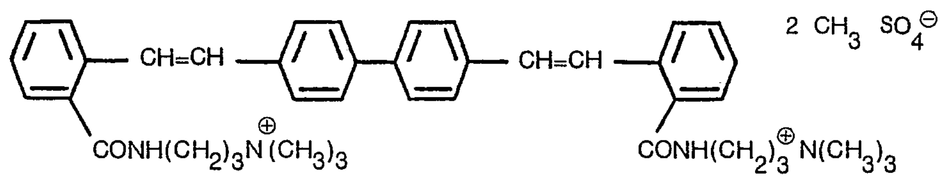

(1301)

vom Schmelzpunkt 196—216°C.

Beispiel 14

102 g der Verbindung der Formel

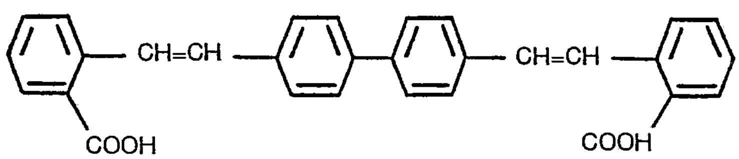

(1400)

werden bei Raumtemperatur in 1000 ml Chlorbenzol und 2 ml Dimethylformamid unter Rühren suspendiert. Innerhalb 10 Minuten werden 125 ml Thionylchlorid zugetropft, auf 80°C erhitzt und das Reaktionsgemisch während 3 Stunden bei 80—85°C gerührt. Nach dem Abkühlen auf Raumtemperatur wird das auskristallisierte Produkt abgenutscht, mit Hexan gewaschen und unter Vakuum bei 80—85°C getrocknet. Man erhält so 105,7 g der Verbindung der Formel

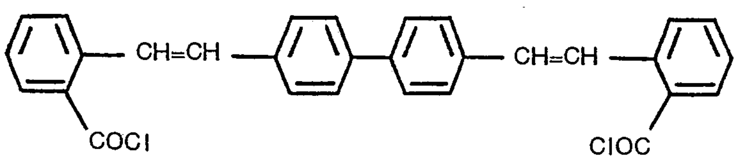

(1401)

vom Schmelzpunkt: 212°C (Zers.).

24,2 g der Verbindung der Formel (1401) werden bei 2°C unter Rühren in 250 ml 1,2-Dimethoxyäthan suspendiert. Innerhalb 15 Minuten werden dann 35,7 g 2-Dimethylamino-äthanol zugetropft und während 23 Stunden bei 2—5°C gerührt. Dann wird die Temperatur der Suspension innerhalb 45 Minuten auf 20°C ansteigen gelassen und noch 4 Stunden bei 20—25°C gerührt. Das Reaktionsgemisch wird auf 2500 ml Wasser gegossen, das Produkt abgenutscht und bei 50—60°C unter Vakuum getrocknet.

Das so erhaltene Rohprodukt wird zweimal aus einer Mischung von 175 ml Aethanol und 75 ml Wasser unter Zuhilfenahme von Aktivkohle umkristallisiert. Man erhält so 19,1 g der Verbindung der Formel

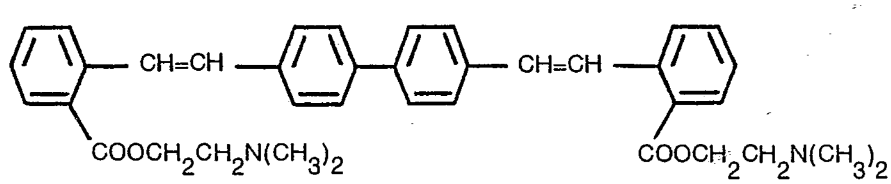

(1402)

von Schmelzpunkt: 104—105°C.

Beispiel 15

5,9 g der Verbindung der Formel (1402) werden bei 45°C unter Rühren in 250 ml Methyläthylketon gelöst. Nach Zugabe von 2,5 ml Dimethylsulfat wird zum Siedepunkt erhitzt und während 3 1/2 Stunden unter Rückfluss gekocht. Nach dem Abkühlen auf Raumtemperatur wird das Produkt abgenutscht, mit Methyläthylketon gewaschen und unter Vakuum bei 60—70°C getrocknet. Man erhält so 7,6 g der Verbindung der Formel

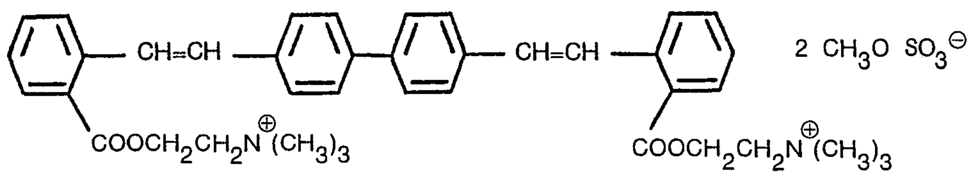

(1500)

von Schmelzpunkt: 280—285°C.

Beispiel 16

Ein gebleichtes Baumwoll-Gewebe wird auf einem Färbeapparat bei einem Flottenverhältnis von 1:20 mit einem wässrigen Bad behandelt, das 0,1% des Aufhellers der Formel (600), (602) bis (606), (608), (617), (700 a—c), (800), (900) oder (901), bezogen auf das Gewicht der Baumwolle, und 5 g/l Natriumsulfat enthält.

Die Applikation erfolgt gemäss folgendem Temperaturprogramm:

20—50°C/15 Minuten

50°C/15 Minuten

Anschliessend wird das Baumwoll-Gewebe während 20 Sektunden in fliessendem enthärtetem Wasser gespült und bei 70°C im Trockenschrank getrocknet. Das so behandelte Baumwoll-Gewebe weist einen guten Aufhelleffekt auf.

Beispiel 17

Man foulardiert bei Raumtemperatur ein gebleichtes Baumwoll-Gewebe mit einer wässrigen Flotte, die 1 g/l des Aufhellers der Formel (600), (602) bis (606), (608), (617), (700 a-c), (800), (900) oder (901) enthält. Der Abquetscheffekt beträgt 75%.

Anschliessend wird während 30 Sekunden bei 70°C auf einem Thermofixiergerät getrocknet.

Das so behandelte Baumwollgewebe weist einen guten Aufhelleffekt auf.

Beispiel 18

Ein Polyacrylnitril-Gewebe (Orlon 75) wird auf einem Färbeapparat bei einem Flottenverhältnis von 1:20 mit einem wässrigen Bad, das

0,1% des Aufhellers der Formel (101), (103)—(107), (600), (602)—(606), (610), (700 a—c), (800) oder (901), bezogen auf das Warengewicht,

1 g/l eines Anlagerungsproduktes von 35 Mol Aethylenoxid an 1 Mol Stearylalkohol und

1,5 ml/l Ameisensäure 85%

enthält, behandelt.

Die Applikation erfolgt gemäss folgendem Temperaturprogamm:

40—97°C/30 Minuten

97°C/30 Minuten

97—40°C/15 Minuten.

Anschliessend wird das Polyacrylnitril-Gewebe während 20 Sekunden in fliessendem enthärtetem Wasser gespült und bei 70°C im Trockenschrank getrocknet. Das so behandelte Gewebe weist einen guten Aufhelleffekt auf.

Beispiel 19

Ein gebleichtes Baumwoll-Gewebe wird im Flottenverhältnis 1:20 während 15 Minuten in einer 30°C warmen, wässrigen Weichspülerflotte behandelt, die pro Liter

0,2 g quaternäres Dimethyldistearyl-ammoniumchlorid und

0,01 g des Aufhellers der Formel (600), (602)—(608), (700 a—c), (900) oder (901).

Anschliessend wird das Baumwoll-Gewebe während 5 Sekunden in fliessendem Trinkwasser gespült und bei 70°C im Trockenschrank getrockent. Das so behandelte Baumwoll-Gewebe weist einen guten Aufhelleffekt auf.

Beispiel 20

Ein gebleichtes Baumwoll-Gewebe wird im Flottenverhältnis 1:20 während 15 Minuten in einer 40°C warmen, wässrigen Flotte gewaschen, die pro Liter

0,5 g eines Anlagerungsproduktes von 10 Mol Aethylenoxid an einem Mol Stearylalkohol und

0,01 g des Aufhellers der Formel (600), (602)—(609), (617), (700 a—c), (800), (900) oder (901) enthält.

Anschliessend wird das Baumwoll-Gewebe während 20 Sekunden in fliessendem Trinkwasser gespült

und bei 70°C im Trockenschrank getrockent. Das so behandelte Baumwoll-Gewebe weist einen guten Aufhelleffekt auf.

Beispiel 21

Ein modifiziertes Polyacrylnitrilgewebe (Courtelle®) wird auf einem Färbeapparat bei einem Flottenverhältnis von 1:20 mit einem wässerigen Bad, das

0,1% des Aufhellers der Formel (101), (103)—(107), (600), (602)—(606), (610), (700 a—c), (800) oder (901), bezogen auf das Warengewicht

1 g/l Oxalsäure

0,25 g/l eines Polyphosphates als Komplexbildner und

0,125 g/l Natriummetabisulfit

enthält, behandelt. Die Applikation erfolgt gemäss folgendem Temperaturprogramm:

40—97°C/30 Minuten

97°C/30 Minuten

97—40°C/15 Minuten.

Anschliessend wird das Polyacrylnitril-Gewebe während 30 Sekunden in fliessendem enthärtetem Wasser gespült und bei 70°C im Trockenschrank getrocknet. Das so behandelte Gewebe weist einen guten Aufhelleffekt auf.

Beispiel 22

Ein Polyamid-6-Gewebe wird auf einem Färbeapparat bei einem Flottenverhältnis von 1:20 mit einem wässrigen Bad behandelt, das

0,3%, bezogen auf das Gewebegewicht, einer Verbindung der Formel (101), (103)—(107), (109), (200) oder (302)

1 g/l eines Anlagerungsproduktes von 1 Mol Stearylalkohol mit 35 Mol Aethylenoxid,

1 g/l eines Anlagerungsproduktes von 1 Mol p.-tert.-Octylphenol mit 8 Mol Aethylenoxid und

0,5 g/l Natriumphosphatpuffer

enthält. Die Applikation erfolgt gemäss folgendem Temperaturprogramm:

50—100°C innerhalb 10 Minuten,

100°C während 20 Minuten

100—50°C innerhalb 5 Minuten.

Anschliessend wird das Gewebe in enthärtetem kalten Wasser gespült und bei 60°C getrocknet. Das so behandelte Gewebe weist einen guten Aufhelleffekt auf.

Beispiel 23

Ein Polyamid-6-Gewebe wird auf einem Färbeapparat bei einem Flottenverhältnis von 1:20 mit einem wässrigen Bad behandelt, das

0,3%, bezogen auf das Gewebegewicht, einer Verbindung der Formel (600), (602)—(607), (700 a—c), (800), (900) oder (901)

1 g/l eines Anlagerungsproduktes von 1 Mol Stearylalkohol mit 35 Mol Aethylenoxid,

1 g/l eines Anlagerungsproduktes von 1 Mol p.-tert.-Octylphenol mit 8 Mol Aethylenoxid und

0,5 g/l Natriumphosphatpuffer

enthält. Die Applikation erfolgt gemäss folgendem Temperaturprogramm.

30—60°C innerhalb 10 Minuten,

60°C während 20 Minuten

anschliessend wird das Gewebe in enthärtetem kaltem Wasser gespült und bei 60°C getrocknet. Das so behandelte Gewebe weist einen guten Aufhelleffekt auf.

Beispiel 24

5 g Faserstoff (bestehend aus gebleichter Sulfitzellulose und gebleichter Buchenzellulose 1:1) in 50 ml Wasser werden in einem Mixer mit 150 ml Aufhellerlösung, enthaltend 2,5 mg entsprechend einer Konzentration von 0,05% des Aufhellers der Formel (600), (602)—(606), (900) oder (901) während 15 Minuten gemischt. Anschliessend werden 1,5 Gew.% Leim, z.B. Bewoidleim® und 2,5 Gew.% Aluminiumsulfat (bezogen auf das Fasertrockengewicht) zugegeben und mit Wasser von ca. 10° dH auf 1000 ml verdünnt. Mit dieser Fasersuspension wird ein Papierblatt gebildet, das einen guten Aufhelleffekt aufweist.

Beispiel 25

5 g Faserstoff (bestehend aus gebleichter Sulfitzellulose und gebleichter Buchenzellulose 1:1) in 150 ml Wasser enthaltend 5 mg eines kationaktiven Polyätheramins werden in einem Mixer mit 50 ml Aufhellerlösung, enthaltend 2,5 mg entsprechend einer Konzentration von 0,05% des Aufhellers der Formel (600), (602)—(606), (900) oder (901) während 15 Minuten gemischt. Anschliessend werden 1,5 Gew.% Leim, z.B. Bewoidleim®, 2,5 Gew.% Aluminiumsulfat und 0,1% eines kationaktiven Polyätheramins (bezogen auf das Fasertrockengewicht) zugegeben und mit Wasser von ca. 10° dH auf 1000 ml verdünnt. Mit dieser Fasersuspension wird ein Papierblatt gebildet, das einen guten Aufhelleffekt aufweist.

Beispiel 26

5 g Faserstoff (bestehend aus gebleichter Sulfitzellulose und gebleichter Buchenzellulose 1:1) in 150 ml Wasser enthaltend 5 mg eines Polyäthylenimins werden in einem Mixer mit 50 ml Aufhellerlösung, enthaltend 2,5 mg entsprechend einer Konzentration von 0,05% des Aufhellers der Formel (600), (602)—(606), (900) oder (901) während 15 Minuten gemischt. Anschliessend werden 1,5 Gew.% Leim z.B. Bewoidleim®, 2,5 Gew.% Aluminiumsulfat und 0,1% eines Polyäthylenimins (bezogen auf das Fasertrockengewicht) zugegeben und mit Wasser von ca. 10° dH auf 1000 ml verdünnt. Mit dieser Fasersuspension wird ein Papierblatt gebildet, das einen guten Aufhelleffekt aufweist.

Beispiel 27

Eine innige Mischung aus 100 Teilen Polyvinylchlorid, 3 Teilen Stabilisator (Advastat BD 100®; Ba/Cd-Komplex), 2 Teilen Titandioxyd, 59 Teilen Dioctylphthalat und 0,01 bis 0,2 Teilen einer Verbindung der Formel (101), (103)—(107) oder (201) wird auf einem Kalander bei 150 bis 155°C zu einer Folie ausgewalzt. Die so gewonnene opake. Polyvinylchloridfolie besitzt einen wesentlich höheren Weissgehalt als eine Folie, welche den optischen Aufheller nicht enthält.

Beispiel 28

Ein konzentriertes flüssiges Waschmittel wird durch Mischen folgender Komponenten hergestellt:

| | Gew.% |
|---|---|
| Aethoxylierte Alkohole ($C_{12}$—$C_{13}$ Alkohol mit 6,5 Mol Aethylenoxid) | 60,0 |
| 1-Methyl-1-oleylamidoäthyl-2-oleyl-imidazoliniummethosulfat | 26,7 |
| Verbindung der Formel (600), (602)—(609), (700), (800), (900) oder (901) | 0,3 |
| Wasser | 12,0 |
| Uebliche Zusätze | 1,0 |

2 kg gebleichtes Baumwollgewebe werden während 10 Minuten bei 60°C in 60 Litern Wasser von 100 ppm Härte gewaschen, das 50 bis 60 g des obigen Waschmittels enthält. Nach dem Spülen und Trocknen weist das Gewebe einen starken Aufhelleffekt und einen weichen Griff auf. Fügt man zur vorangehend beschriebenen Waschflotte noch 0,2 g/l Aktivchlor in Form von Natriumhypochlorit hinzu und verfährt, wie beschrieben, so erhält man ebenso hohe Effekte

Aehnliche Resultate werden erhalten, wenn anstelle des oben angegebenen, ein flüssiges Waschmittel folgender Zusammensetzung

| | Gew.% |
|---|---|
| Aethoxylierte Alkohole ($C_{12}$—$C_{13}$) Alkohol mit 6,5 Mol Aethylenoxid) | 55,0 |
| 1-Methyl-1-talgamidoäthyl-2-talg-imidazoliniummethosulfat | 26,0 |
| Verbindung der Formel (600), (602)—(609), (700), (800), (900) oder (901) | 0,3 |
| Wasser | 13,0 |
| Isopropanol | 5,0 |
| Uebliche Zusätze | 0,7 |

oder ein anderes nicht-ionische Tenside und kationische Substanzen enthaltendes flüssiges Waschmittel, wie z.B. das sich im Handel befindliche "Perwoll® flüssig" oder Samtess® verwendet wird, denen die erfindungsgemässen Aufheller beigemischt werden.

Beispiel 29

Ein flüssiges Waschmittel wird durch Mischen folgender Komponenten hergestellt:

| | Gew.% |
|---|---|
| Aethoxylierte Alkohole ($C_{14}$—$C_{15}$ Alkohol mit 7 Mol Aethylenoxid) | 12,0 |
| Aethoxylierte Alkohole ($C_{12}$—$C_{13}$ Alkohol mit 6,5 Mol Aethylenoxid) | 12,0 |
| Nicht-gehärtetes Di-talg-dimethyl-ammonium chlorid | 6,4 |
| Aethanol | 15,0 |
| Natriumbicarbonat | 0,25 |
| Verbindung der Formel (600), (602)—(609), (700), (800), (900) oder (901) | 0,41 |
| Uebliche Zusätze | 0,41 |
| Wasser | 53,53 |

Ein wie im Beispiel 28 beschrieben, behandeltes Baumwollgewebe weist einen starken Aufhelleffekt und einen weichen Griff auf.

**Patentansprüche für die Vertragsstaaten: BE CH DE FR GB IT LI NL SE**

1. Distyrylbiphenyle der Formel

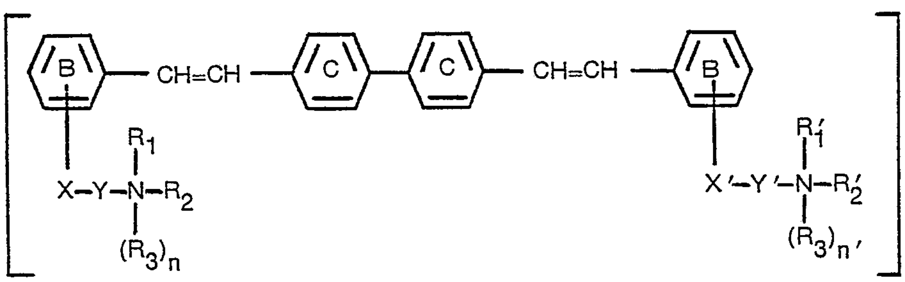

$(n+n')^{\oplus}$

$(n+n')A^{\ominus}$

worin X und X' unabhängig voneinander die direkte Bindung, Sauerstoff, Schwefel, —O—$C_{1-3}$-Alkylen-CON($R_4$)—, —CON($R_4$)—, —O—$C_{1-3}$-Alkylen-COO—, —OCO— oder —COO—, wobei, wenn n + n' = 0, X und X' nicht —CON($R_4$)— oder —O—$C_{1-3}$-Alkylen-CON($R_4$)— und wenn n + n' = 2 und X und X' —CON($R_4$)— oder —COO—, $A^{\ominus}$ nicht Phosphit- oder Phosphonatanion sein dürfen, Y und Y' unabhängig voneinander $C_{1-20}$-Alkylen oder durch eine Hydroxylgruppe substituiertes $C_{1-20}$-Alkylen, $R_1$ und $R_1'$ unabhängig voneinander unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_1$ bzw. $R_1'$ zusammen mit $R_2$ bzw. $R_2'$ und dem N-Atom, an das sie gebunden sind, einen 5- bis 7-gliedrigen heterocyclischen Ring, $R_2$ und $R_2'$ unabhängig voneinander unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_2$ bzw. $R_2'$ zusammen mit $R_1$ bzw. $R_1'$ und dem N-Atom, an das sie gebunden sind, einen 5- bis 7-gliedrigen heterocyclischen Ring, $R_3$ Wasserstoff, unsubstituiertes oder durch Cyano, Hydroxy, Alkoxy, Phenyl oder $C_{1-4}$-Alkoxycarbonyl substituiertes $C_{1-4}$-Alkyl oder $C_{3-4}$-Alkenyl, $R_1$, $R_2$ und $R_3$ zusammen mit dem N-Atom, an das sie gebunden sind, bzw. $R_1'$, $R_2'$ und $R_3$ zusammen mit dem N-Atom, an das sie gebunden sind, auch den Pyridin- oder Picolinring, $R_4$ Wasserstoff oder unsubstituiertes oder durch Cyano, Carbamoyl, Carbalkoxy, Hydroxy, Halogen oder $C_{1-4}$-Alkoxy substituiertes $C_{1-6}$-Alkyl, $A^{\ominus}$ ein farbloses Anion und n und n' unabhängig voneinander die Zahl 0 oder 1 bedeuten, wobei die Benzolkerne B und C auch nichtchromophor substituiert sein können, und worin die Gruppierungen

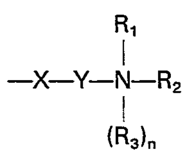 sowie 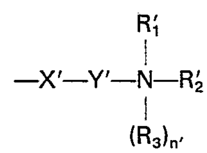

im Falle, dass die Ringe B und C keine nicht-chromophoren Substituenten tragen, auch beide jeweils

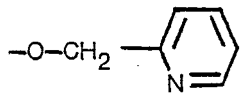

oder beide jeweils

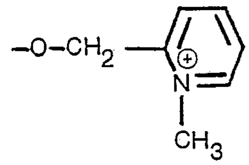

in den ortho-Stellungen zu den beiden Gruppen —CH=CH— sein könen.

2. Distyrylbiphenyle gemäss Anspruch 1, der Formel

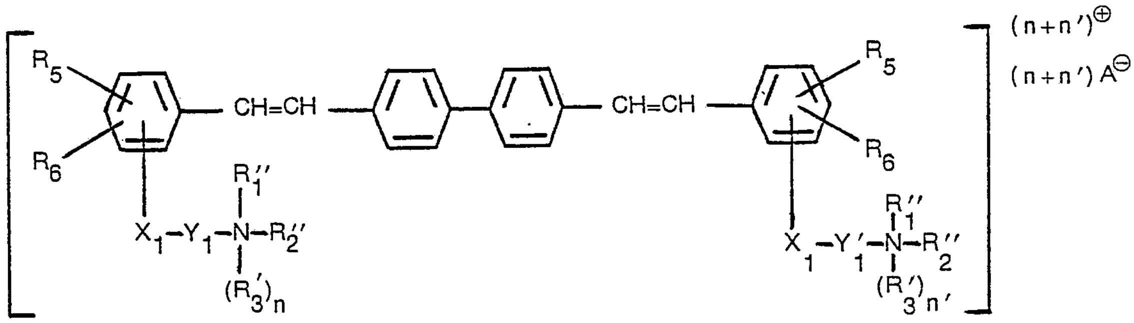

worin $X_1$ die direkte Bindung, Sauerstoff, Schwefel, —O—$C_{1-3}$-Alkylen-CONH—, —CONH—, —O—$C_{1-3}$-Alkylen-COO—, —OCO— oder —COO—, wobei, wenn $n + n' = 0$, $X_1$ nicht —CONH— oder —O—$C_{1-3}$-Alkylen-CONH— und wenn $n + n' = 2$ und $X_1$ —CONH— oder —COO—, $A^{\ominus}$ nicht Phosphit- oder Phosphonatanionen sein dürfen, $Y_1$ und $Y_1'$ unabhängig voneinander $C_{1-4}$-Alkylen oder Hydroxypropylen, $R_1''$ und $R_2''$ unabhängig voneinander $C_{1-4}$-Alkyl oder $R_1''$ und $R_2''$ zusammen mit dem N-Atom, an das die gebunden sind, einen Pyrrolidin-, Piperidin-, Hexamethylenimin- oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_{2-4}$-Hydroxyalkyl oder $C_{2-4}$-Cyanoalkyl, $R_1''$, $R_2''$ und $R_3'$ zusammen mit dem N-Atom, an das die gebunden sind, auch den Pyridin- oder Picolinring, $R_5$ Wasserstoff, Chlor, $C_{1-4}$-Alkyl, $C_{3-4}$-Alkenyl, $C_{1-3}$-Alkoxy oder $R_5$ zusammen mit $R_6$ eine Trimethylen- oder Tetramethylengruppe, $R_6$ Wasserstoff, Chlor, $C_{1-4}$-Alkyl, $C_{1-3}$-Alkoxy oder $R_6$ zusammen mit $R_5$ eine Trimethylen- oder Tetramethylengruppe, n und n' unabhängig voneinander die Zahl 0 oder 1 und $A^{\ominus}$ ein farbloses Anion bedeuten.

3. Distyrylbiphenyle gemäss Anspruch 1 der Formel

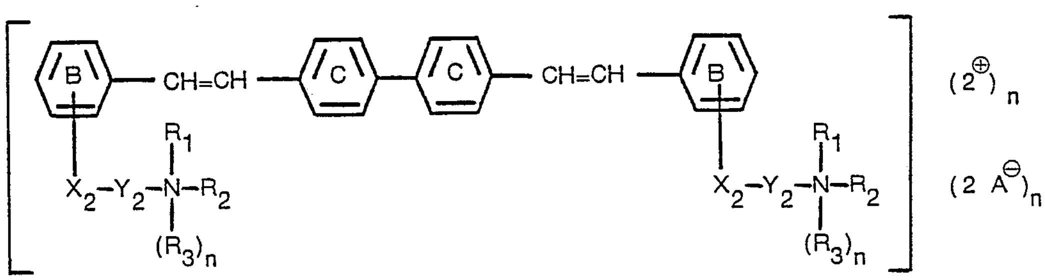

worin $X_2$ Sauerstoff, Schwefel, —CON($R_4$)— —OCO— oder —COO— wobei, wenn $n = 0$, $X_2$ nicht —CON($R_4$)— und wenn $n = 1$ und $X_2$ —CON($R_4$)— oder —COO— ist, $A^{\ominus}$ nicht Phosphit- oder Phosphonatanionen sein dürfen, $Y_2$ $C_{1-20}$-Alkylen oder durch eine Hydroxylgruppe substituiertes $C_{1-6}$-Alkylen, $R_1$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl, oder $R_1$ zusammen mit $R_2$ und dem N-Atom, an das sie gebunden sind, einen 5- bis 7-gliedrigen heterocyclischen Ring, $R_2$ unsubstituiertes oder durch Cyano, Hydroxy, Alkoxy, Phenyl, $C_{1-4}$-Akoxycarbonyl oder Chlor substituiertes $C_{1-8}$-Alkyl, $C_{3-4}$-Alkenyl oder $R_2$ zusammen mit $R_1$ und dem N-Atom, an das sie gebunden sind, einen 5- bis 7-gliedrigen heterocyclischen Ring, $R_3$ Wasserstoff, unsubstituiertes oder durch Cyano, Hydroxy, Alkoxy, Phenyl oder $C_{1-4}$-Alkoxycarbonyl substituiertes $C_{1-4}$-Alkyl, $R_1$, $R_2$ und $R_3$ zusammen mit dem N-Atom, an das sie gebunden sind, auch den Pyridin-

oder Picolinring, $R_4$ Wasserstoff oder unsubstituiertes oder durch Cyano, Carbamoyl, Carbalkoxy, Hydroxy, Halogen oder $C_{1-4}$-Alkoxy substituiertes $C_{1-6}$-Alkyl, n die Zahl 0 oder 1 und $A^{\ominus}$ ein farbloses Anion bedeuten, wobei die Benzolkerne B und C auch nicht-chromophor substituiert sein können.

4. Distyrylbiphenyle gemäss Anspruch 2 der Formel

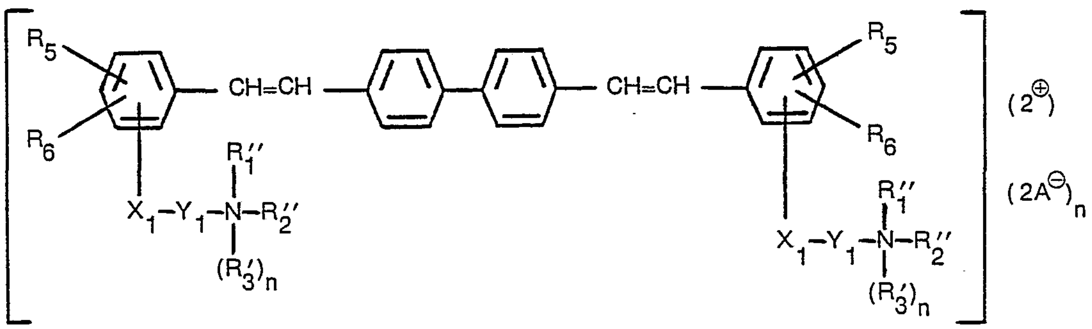

worin $X_1$ die direkte Bindung, Sauerstoff, Schwefel, —O—$C_{1-3}$-Alkylen-CONH—, —CONH—, —O—$C_{1-3}$-Alkylen-COO—, —OCO— oder —COO—, wobei, wenn n = 0, $X_1$ nicht —CONH— oder —O—$C_{1-3}$-Alkylen-CONH— und wenn n = 1 und $X_1$ —CONH— oder —COO— ist, $A^{\ominus}$ nicht Phosphit- oder Phosphonatanionen sein dürfen, $Y_1$ $C_{1-4}$-Alkylen oder Hydroxypropylen, $R_1''$ und $R_2''$ unabhängig voneinander $C_{1-4}$-Alkyl oder $R_1''$ und $R_2''$ zusammen mit dem N-Atom, an das sie bebunden sind, eine Pyrollidin-, Piperidin-, Hexamethylenimin- oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_{2-4}$-Hydroxyalkyl oder $C_{2-4}$-Cyanoalkyl, $R_1''$, $R_2''$ und $R_3'$ zusammen mit dem N-Atom, an das sie gebunden sind, auch den Pyridin- oder Picolinring, $R_5$ Wasserstoff, Chlor, $C_{1-4}$-Alkyl, $C_{3-4}$-Alkenyl, $C_{1-3}$-Alkoxy oder $R_5$ zusammen mit $R_6$ eine Trimethylen- oder Tetramethylengruppe, $R_6$ Wasserstoff, Chlor, $C_{1-4}$-Alkyl, $C_{1-3}$-Alkoxy oder $R_6$ zusammen mit $R_5$ eine Trimethylen- oder Tetramethylengruppe, n die Zahl 0 oder 1 und $A^{\ominus}$ ein farbloses Anion bedeuten.

5. Distyrylbiphenyle gemäss Anspruch 4, der Formel

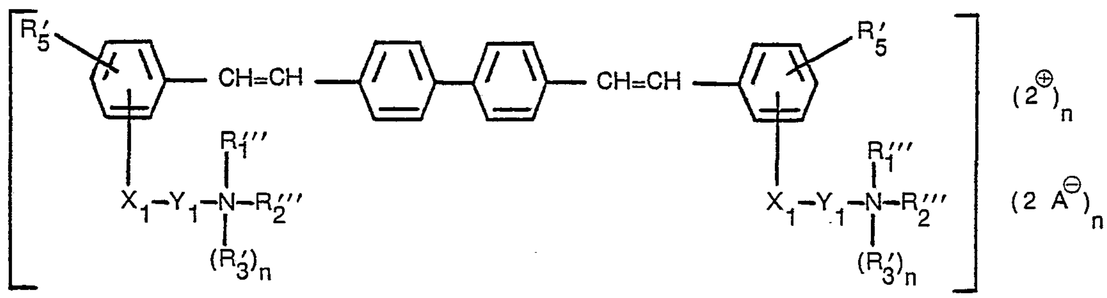

worin $X_1$ die direkte Bindung, Sauerstoff, Schwefel, —O—$C_{1-3}$-Alkylen-CONH—, —CONH—, —O—$C_{1-3}$-Alkylen-COO—, —OCO— oder —COO—, wobei, wenn n = 0, $X_1$ nicht —CONH— oder —O—$C_{1-3}$-Alkylen-CONH— und wenn n = 1 und $X_1$ —CONH— oder —COO— ist, $A^{\ominus}$ nicht Phosphit- oder Phosphonatanionen sein dürfen, $Y_1$ $C_{1-4}$-Alkylen oder Hydroxypropylen, $R_1'''$ und $R_2'''$ unabhängig voneinander $C_{1-4}$-Alkyl, $R_1'''$ und $R_2'''$ zusammen mit dem N-Atom, an das sie gebunden sind, einen Pyrrolidin-, Piperidin- oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_{2-4}$-Hydroxyalkyl oder $C_{2-4}$-Cyanoalkyl, $R_1'''$, $R_2'''$ und $R_3'$ zusammen mit dem N-Atom, an das die gebunden sind, auch den Pyridinring, $R_5'$ Wasserstoff, Chlor, $C_{1-4}$-Alkyl oder $C_{1-3}$-Alkoxy, n die Zahl 0 oder 1 und $A^{\ominus}$ ein farbloses Anion bedeuten.

6. Distyrylbiphenyle gemäss Anspruch 4 der Formel

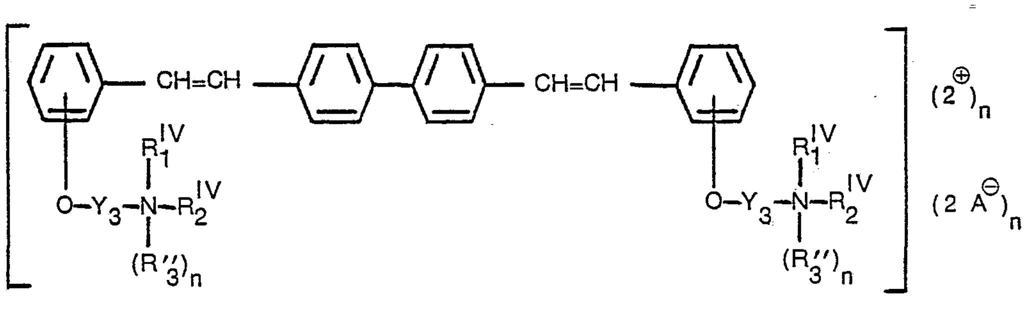

worin $Y_3$ $C_{2-4}$-Alkylen, $R_1^{IV}$ $C_{1-3}$-Alkyl oder $R_1^{IV}$ zusammen mit $R_2^{IV}$ und dem N-Atom, an das sie gebunden

sind, einen Pyrrolidin-, Piperidin-, Hexamethylenimin- oder Morpholinring, $R_2^{IV}$ $C_{1-3}$-Alkyl oder $R_2^{IV}$ zusammen mit $R_1^{IV}$ und dem N-Atom, an das sie gebunden sind, einen Pyrrolidin-, Piperidin-, Hexamethylenimin- oder Morpholingring, $R_3''$ Wasserstoff oder $C_{1-3}$-Alkyl, n die Zahl 0 oder 1 und $A^{\ominus}$ ein farbloses Anion bedeuten.

7. Distyrylbiphenyle gemäss Anspruch 6 der Formel

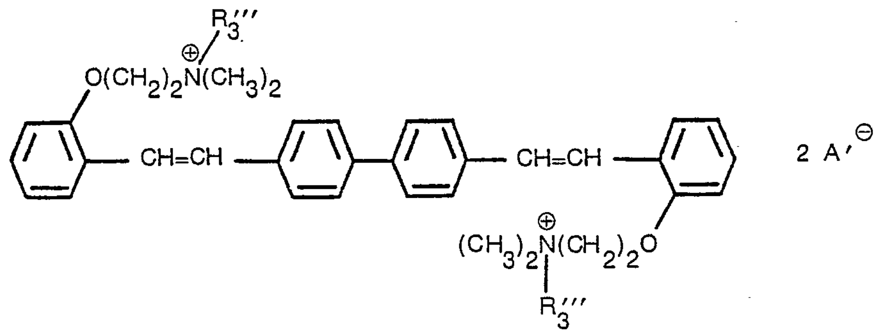

worin $A'^{\ominus}$ $CH_3OSO_3^{\ominus}$ oder $C_2H_5OSO_3^{\ominus}$ und $R_3'''$ Methyl oder Aethyl bedeuten.

8. Distyrylbiphenyl gemäss Anspruch 6 der Formel

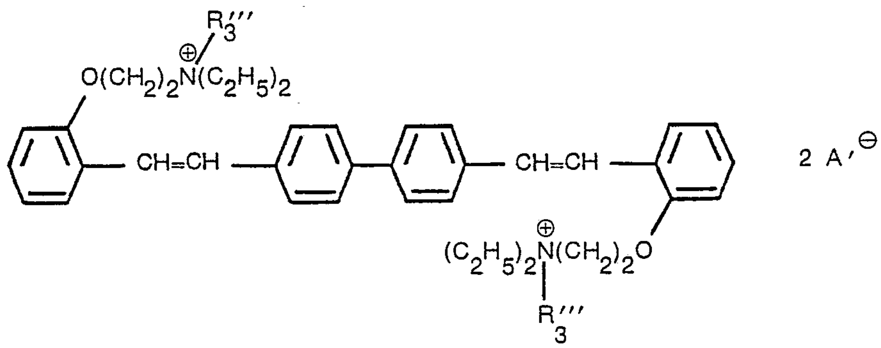

worin $A'^{\ominus}$ $CH_3OSO_3^{\ominus}$ oder $C_2H_5OSO_3^{\ominus}$ und $R_3'''$ Methyl oder Aethyl bedeuten.

9. Distyrylbiphenyle gemäss Anspruch 6 der Formel

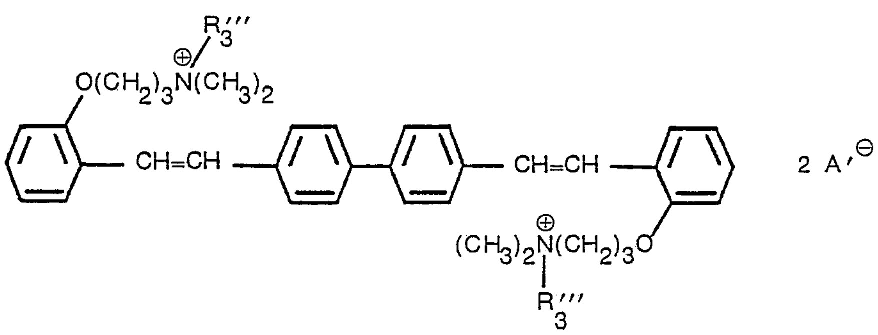

worin $A'^{\ominus}$ $CH_3OSO_3^{\ominus}$ oder $C_2H_5OSO_3^{\ominus}$ und $R_3'''$ Methyl oder Aethyl bedeuten.

10. Distyrylbiphenyle gemäss Anspruch 1 der Formel

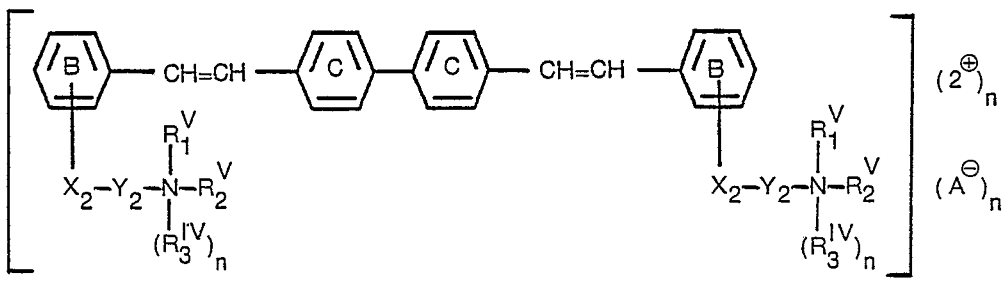

worin $X_2$ Sauerstoff, Schwefel

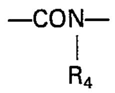

oder —COO—, wobei wenn $A^{\ominus}$ ein Phosphit- oder Phosphonatanion darstellt, $X_2$ nicht —CON($R_4$)— oder —COO— ist, $Y_2$ $C_{2-20}$-Alkylen, $R_1^V$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_1^V$ zusammen mit $R_2^V$ und dem N-Atom, an das die gebunden sind, einen 5- bis 7-gliedrigen heterocyclischen Ring, $R_2^V$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_2^V$ zusammen mit $R_1^V$ und dem N-Atom, an das die gebunden sind, einen 5- bis 7-gliedrigen heterocyclischen Ring, $R_3^{IV}$ Wasserstoff oder unsubstituiertes oder durch Cyano, Hydroxy, Alkoxy, Phenyl oder $C_{1-4}$-Alkoxycarbonyl substituiertes $C_{1-4}$-Alkyl, $R_4$ Wasserstoff oder unsubstituiertes oder durch Cyano, Carbamoyl, Carbalkoxy, Hydroxy, Halogen oder $C_{1-4}$-Alkoxy substituiertes $C_{1-6}$-Alkyl, $A^{\ominus}$ das Aequivalent eines farblosen Anions und n die Zahl 0 oder 1 bedeuten, wobei die Benzolkerne B und C auch nicht-chromophor substituiert sein können.

11. Distyrylbiphenyle gemäss Anspruch 10 der Formel

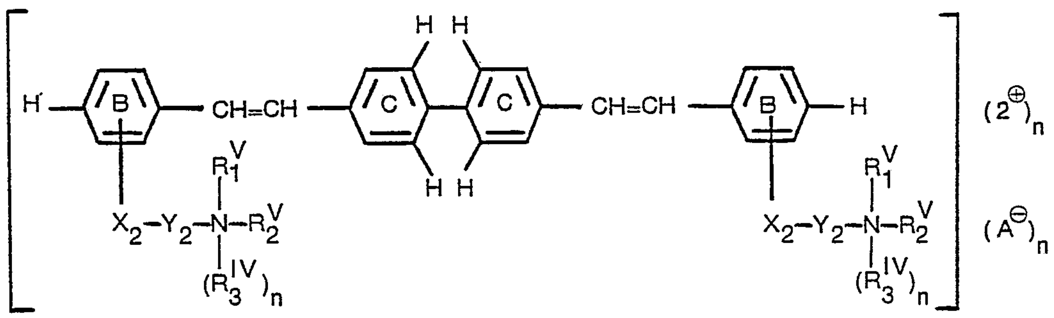

worin $X_2$, $Y_2$, $R_1^V$, $R_2^V$, $R_3^{IV}$, $A^{\ominus}$ und n die in Anspruch 10 angegebene Bedeutung haben und die Benzolkerne B und C auch nicht-chromophor substituiert sein können.

12. Distyrylbiphenyl gemäss Anspruch 11 der Formel

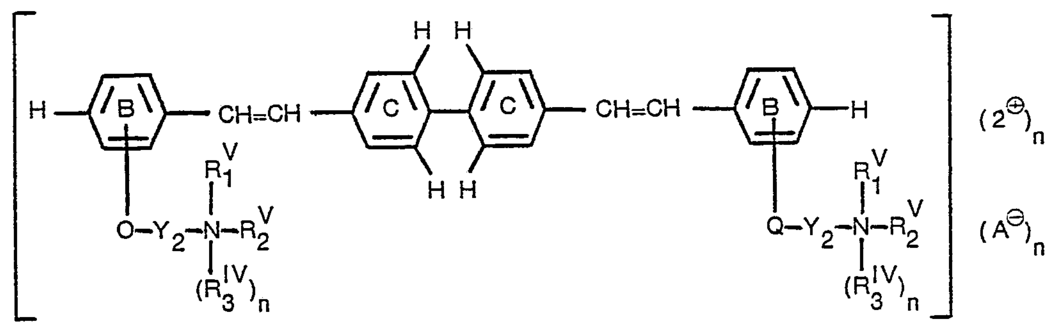

worin $Y_2C_{2-20}$-Alkylen, $R_1^V$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_1^V$ zusammen mit $R_2^V$ und dem N-Atom, an das sie gebunden sind, einen 5- bis 7-gliedrigen heterocyclischen Ring, $R_2^V$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_2^V$ zusammen mit $R_1^V$ und dem N-Atom, an das sie gebunden sind, einen 5- bis 7-gliedrigen heterocyclischen Ring, $R_3^{IV}$ Wasserstoff oder unsubstituiertes oder durch Cyano, Hydroxy, Alkoxy, Phenyl oder $C_{1-4}$-Alkoxycarbonyl substituiertes $C_{1-4}$-Alkyl, $R_4$ Wasserstoff oder unsubstituiertes oder durch Cyano, Carbamoyl, Carbalkoxy, Hydroxy, Halogen oder $C_{1-4}$-Alkoxy substituiertes $C_{1-6}$-Alkyl, $A^{\ominus}$ das Aequivalent eines farblosen Anions und n die Zahl 0 oder 1 bedeuten, wobei die Benzolkerne B und C auch nicht-chromophor substituiert sein können.

13. Verfahren zur Herstellung von Distyrylbiphenylen der Formel

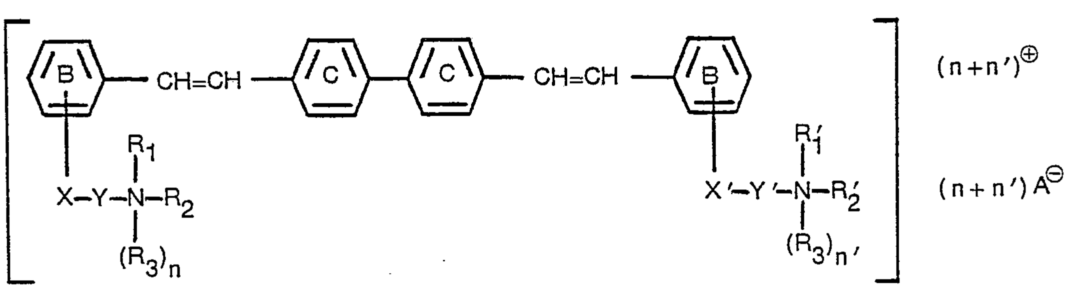

worin X und X' unabhängig voneinander die direkte Bindung, Sauerstoff, Schwefel, —O—$C_{1-3}$-Alkylen-CON($R_4$)—, —CON($R_4$)—, —O—$C_{1-3}$-Alkylen-COO—, —OCO— oder —COO—, wobei wenn n + n' = 0, X und X' nicht —CON($R_4$)— oder —O—$C_{1-3}$-Alkylen-CON($R_4$)— und wenn n + n' = 2 und X und X' —CON($R_4$)— oder —COO—, $A^{\ominus}$ nicht Phosphit- oder Phosphonatanion sein dürfen, Y und Y' unabhängig voneinander $C_{1-20}$-Alkylen oder durch eine Hydroxylgruppe substituiertes $C_{1-20}$-Alkylen, $R_1$ und $R_1'$ unabhängig voneinander unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_1$ bzw. $R_1'$ zusammen mit $R_2$ bzw. $R_2'$ und dem N-Atom, an das die gebunden sind, einen 5- bis 7-gliedrigen heterocyclischen Ring, $R_2$ und $R_2'$ unabhängig voneinander unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_2$ bzw. $R_2'$ zusammen mit $R_1$ bzw. $R_1'$ und dem N-Atom, an das sie gebunden sind, einen 5- bis 7-gliedrigen heterocyclischen Ring, $R_3$ Wasserstoff, unsubstituiertes oder durch Cyano, Hydroxy, Alkoxy, Phenyl oder $C_{1-4}$-Alkoxycarbonyl substituiertes $C_{1-4}$-Alkyl oder $C_{3-4}$-Alkenyl, $R_1$, $R_2$ und $R_3$ zusammen mit dem N-Atom, an das die gebunden sind, bzw. $R_1'$, $R_2'$ und $R_3'$ zusammen mit dem N-Atom, an das sie gebunden sind, auch den Pyridin- oder Picolinring, $R_4$ Wasserstoff oder unsubstituiertes oder durch Cyano, Carbamoyl, Carbalkoxy, Hydroxy, Halogen oder $C_{1-4}$-Alkoxy substituiertes $C_{1-6}$-Alkyl, $A^{\ominus}$ ein farbloses Anion, n und n' anabhängig voneinander die Zahl 0 oder 1 bedeuten, wobei die Benzolkerne B und C auch nicht-chromophor substituiert sein können, und worin die Gruppierungen

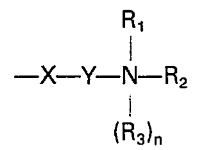

sowie

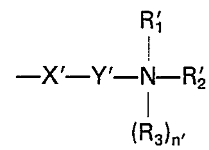

im Falle, dass die Ringe B und C keine nicht-chromophoren Substituenten tragen, auch beide jeweils

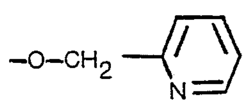

oder beide jeweils

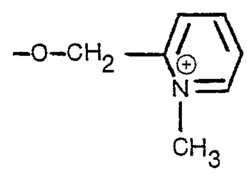

in den ortho-Stellungen zu den beiden Gruppen —CH=CH— sein können, dadurch gekennzeichnet, dass man 1 Moläquivalent eines Distyrylbiphenyls der Formel

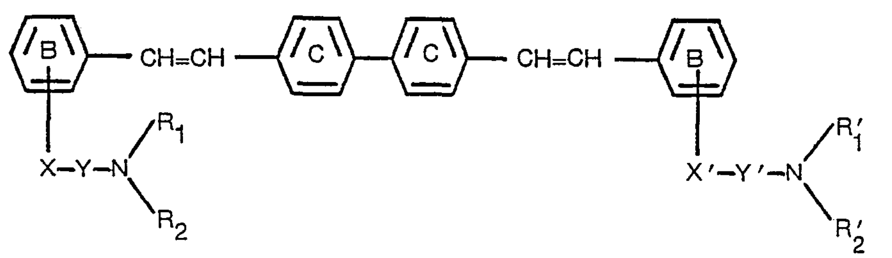

worin die Benzolringe B und C und X, X', Y, Y', $R_1$, $R_1'$, $R_2$ und $R_2'$ die oben angegenbene Bedeutung haben, mit 1 Moläquivalent oder mit 2 Moläquivalenten eines Alkylierungsmittels bzw. einer Säure der Formel $R_3$—A quaterniert bzw. protoniert, worin $R_3$ und $A^{\ominus}$ die oben angegebene Bedeutung haben.

14. Verfahren zur Herstellung von Distyrylbiphenylen der Formel

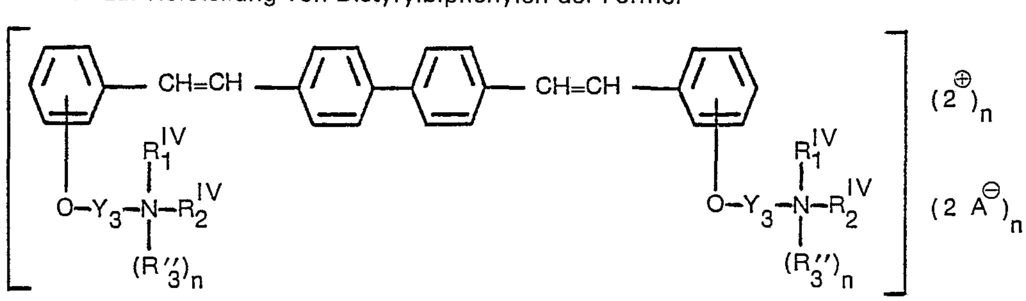

worin $Y_3$ $C_{1-4}$-Alkylen, $R_1^{IV}$ $C_{1-3}$-Alkyl oder $R_1^{IV}$ zusammen mit $R_2^{IV}$ un dem N-Atom, an das sie gebunden sind einen Pyrollidin-, Piperidin-, Hexamethylenimin- oder Morpholinring, $R_2^{IV}$ $C_{1-3}$-Alkyl oder $R_2^{IV}$ zusammen mit $R_1^{IV}$ und dem N-Atom, an das sie gebunden sind, einen Pyrrolidin-, Piperidin-, Hexamethylenimin- oder Morpholingring, $R_3''$ Wasserstoff oder $C_{1-3}$-Alkyl, n die Zahl 0 oder 1 und $A^{\ominus}$ ein farbloses Anion bedeuten, dadurch gekennzeichnet, dass man im Molekularverhältnis 1:2 eine Verbindung der Formel

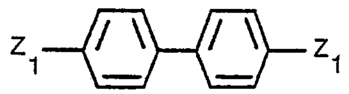

mit solchen der Formel

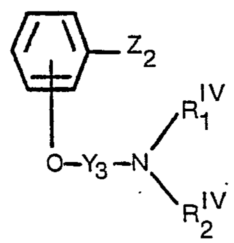

in Gegenwart einer starken Base umsetzt, in welchen Formeln $Y_3$, $R_1^{IV}$ und $R_2^{IV}$ die oben angegebene Bedeutung haben und eines der Symbole $Z_1$ und $Z_2$ eine OCH-Gruppe und das andere eine Gruppierung der Formel

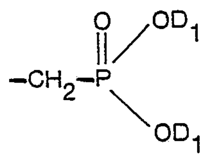 , 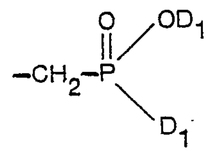 ,

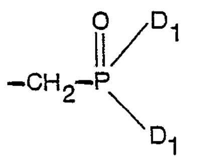 oder 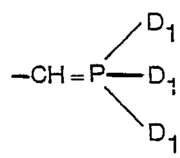

bedeutet, worin $D_1$ eine unsubstituierten oder substituierten Alkyl-, Aryl-, Cycloalkyl- oder Aralkylrest darstellt, und gewünschtenfalls die erhaltene Verbindung der Formel

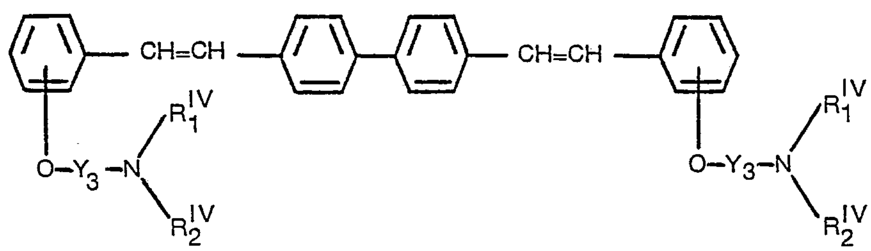

worin $Y_3$, $R_1^{IV}$ und $R_2^{IV}$ die oben angegebene Bedeuten haben, mit 2 Moläquivalenten eines Alkylierungsmittels bzw. einer Säure der Formel $R_3''$—A worin $R_3''$ und A die oben angegebene Bedeutung haben, quaterniert bzw. protoniert.

15. Verfahren zum optischen Aufhellen von organischen Materialien, dadurch gekennzeichnet, dass man Distyrylbiphenyle der im Anspruch 1 definierten Formel diesen Materialien einverleibt oder auf deren Oberfläche aufbringt.

16. Verfahren gemäss Anspruch 15 zum optischen Aufhellen von Polyacrylnitril oder Cellulose als organischem Material.

17. Waschmittel enthaltend ein oder mehrere Distyrylbiphenyle der im Anspruch 1 definierten Formel.

18. Waschmittel gemäss Anspruch 17, welches 10 bis 70 Gew.% eines nicht-ionogenen Tensides und 1 bis 30% eines kationischen Textilweichmachers enthält.

19. Waschmittel gemäss Anspruch 18, welches 10 bis 70 Gew.% eines nicht-ionogenen Tensides und 1 bis 30 Gew.% eines quartären Derivates des Ammoniaks und/oder des Imidazolins mit je 2 langkettigen

aliphatischen Resten als kationischen Textilweichmacher und zur Verleihung der flüssigen Form ein Lösungsmittel enthält.

20. Waschmittel gemäss Anspruch 19, welches 1-Methyl-1-oleylamidoäthyl-2-oleyl-imidazolinium·$X^{\ominus}$, 1-Methyl-1-talgamidoäthyl-2-talg-imidazolinium·$X^{\ominus}$, Di-talg-dimethyl-ammonium·$X^{\ominus}$ oder eine Verbindung der Formel

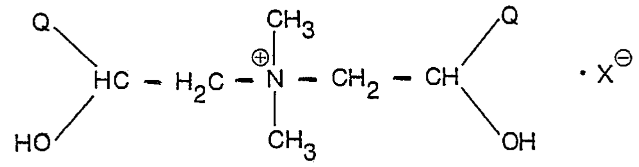

worin Q $C_{14-16}$-Alkyl bedeutet und $X^{\ominus}$ für ein Chlorid-, Bromid-, Methylsulfat-, Aethylsulfat-, Methansulfonat-, Aethansulfonat- oder Toluolsulfonat-Anion steht, als kationischen Textilweichmacher enthält.

21. Textilbehandlungsmittel, enthaltend ein oder mehrere Distyrylbiphenyle der im Anspruch 1 definierten Formel.

22. Wäschenachbehandlungsmittel, enthaltend eine oder mehrere Distyrylbiphenyle der im Anspruch 1 definierten Formel und einen kationischen Textilweichmacher.

**Patentansprüche für den Vertragsstaat: AT**

1. Aufhellmittel enthaltend eine Verbindung der Formel

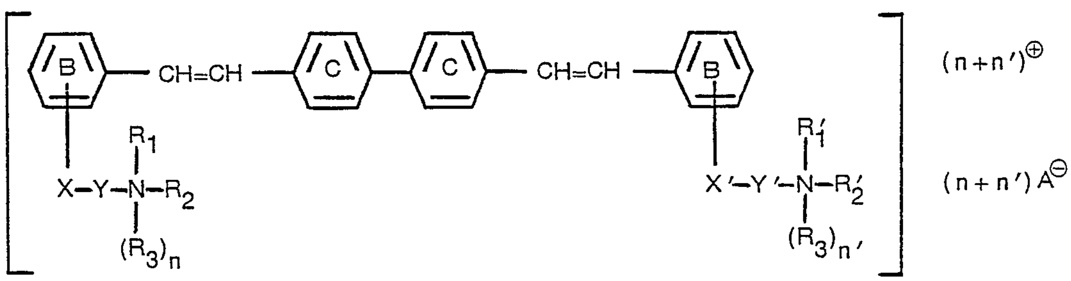

worin X und X' unabhängig voneinander die direkte Bindung, Sauerstoff, Schwefel, —O—$C_{1-3}$-Alkylen-CON($R_4$)—, —CON($R_4$)—, —O—$C_{1-3}$-Alkylen-COO—, —OCO— oder —COO—, wobei, wenn n + n' = 0, X und X' nicht —CON($R_4$)— oder —O—$C_{1-3}$-Alkylen-CON($R_4$)— und wenn n + n' = 2 und X und X' —CON($R_4$)— oder —COO—, $A^{\ominus}$ nicht Phosphit- oder Phosphanatanion sein dürfen, Y und Y' unabhängig voneinander $C_{1-20}$-Alkylen oder durch eine Hydroxylgruppe substituiertes $C_{1-20}$-Alkylen, $R_1$ und $R_1'$ unabhängig voneinander unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_1$ bzw. $R_1'$ zusammen mit $R_2$ bzw. $R_2'$ und dem N-Atom, an das sie gebunden sind, einen 5- bis 7-gliedrigen heterocyclischen Ring, $R_2$ und $R_2'$ unabhängig voneinander unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_2$ bzw. $R_2'$ zusammen mit $R_1$ bzw. $R_1'$ und dem N-Atom, an das sie gebunden sind, einen 5- bis 7-gliedrigen heterocyclischen Ring, $R_3$ Wasserstoff, unsubstituiertes oder durch Cyano, Hydroxy, Alkoxy, Phenyl oder $C_{1-4}$-Alkoxycarbonyl substituiertes $C_{1-4}$-Alkyl oder $C_{3-4}$-Alkenyl, $R_1$, $R_2$ und $R_3$ zusammen mit dem N-Atom, an das sie gebunden sind, bzw. $R_1'$, $R_2'$ und $R_3$ zusammen mit dem N-Atom, an das sie gebunden sind, auch den Pyridin- oder Picolinring, $R_4$ Wasserstoff oder unsubstituiertes oder durch Cyano, Carbamoyl, Carbalkoxy, Hydroxy, Halogen oder $C_{1-4}$-Alkoxy substituiertes $C_{1-6}$-Alkyl, $A^{\ominus}$ ein farbloses Anion und n und n' unabhängig voneinander die Zahl 0 oder 1 bedeuten, wobei die Benzokerne B und C auch nicht-chromophor substituiert sein können, und worin die Gruppierungen

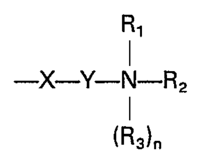

sowie

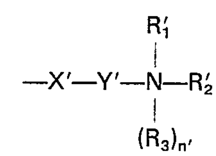

im Falle, dass die Ringe B und C keine nicht-chromophoren Substituenten tragen, auch beide jeweils

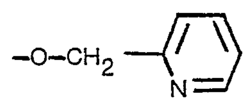

oder beide jeweils

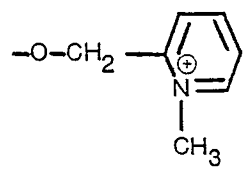

in den ortho-Stellungen zu den beiden Gruppen —CH=CH— sein könen, zusammen mit für Aufhellmittel geeigneten Zusätzen.

2. Aufhellmittel nach Anspruch 1 enthaltend eine Verbindung der Formel

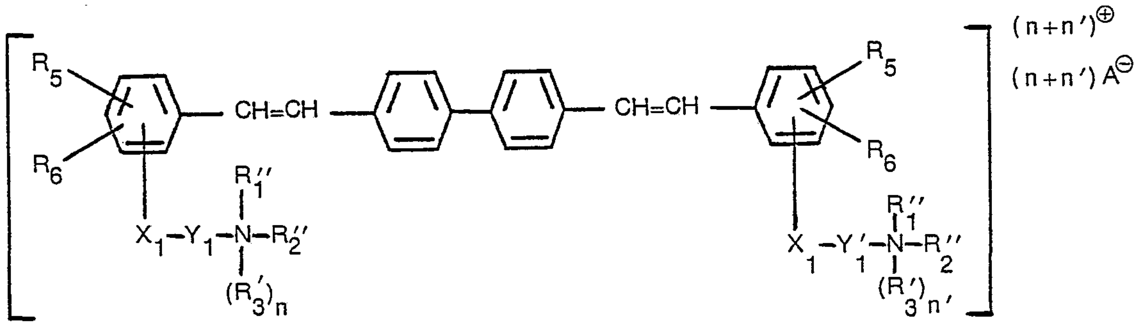

worin $X_1$ die direkte Bindung, Sauerstoff, Schwefel, —O—$C_{1-3}$-Alkylen-CONH—, —CONH—, —O—$C_{1-3}$-Alkylen-COO—, —OCO— oder —COO—, wobei, wenn $n + n' = 0$, $X_1$ nicht —CONH— oder —O—$C_{1-3}$-Alkylen-CONH— und wenn $n + n' = 2$ und $X_1$ —CONH— oder —COO—, $A^{\ominus}$ nicht Phosphit- oder Phosphonatanionen sein dürfen, $Y_1$ und $Y_1'$ unabhängig voneinander $C_{1-4}$-Alkylen oder Hydroxypropylen, $R_1''$ und $R_2''$ unabhängig voneinander $C_{1-4}$-Alkyl oder $R_1''$ und $R_2''$ zusammen mit dem N-Atom, an das sie gebunden sind, einen Pyrrolidin-, Piperidin-, Hexamethylenimin- oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_{2-4}$-Hydroxyalkyl oder $C_{2-4}$-Cyanoalkyl, $R_1''$, $R_2''$ und $R_3'$ zusammen mit dem N-Atom, an das sie gebunden sind, auch den Pyridin- oder Picolinring, $R_5$ Wasserstoff, Chlor, $C_{1-4}$-Alkyl, $C_{3-4}$-Alkenyl, $C_{1-3}$-Alkoxy oder $R_5$ zusammen mit $R_6$ eine Trimethylen- oder Tetramethylengruppe, $R_6$ Wasserstoff, Chlor, $C_{1-4}$-Alkyl, $C_{1-3}$-Alkoxy oder $R_6$ zusammen mit $R_5$ eine Trimethylen- oder Tetramethylengruppe, n und n' unabhängig voneinander die Zahl 0 oder 1 und $A^{\ominus}$ ein farbloses Anion bedeuten.

3. Aufhellmittel nach Anspruch 1 enthaltend eine Verbindung der Formel

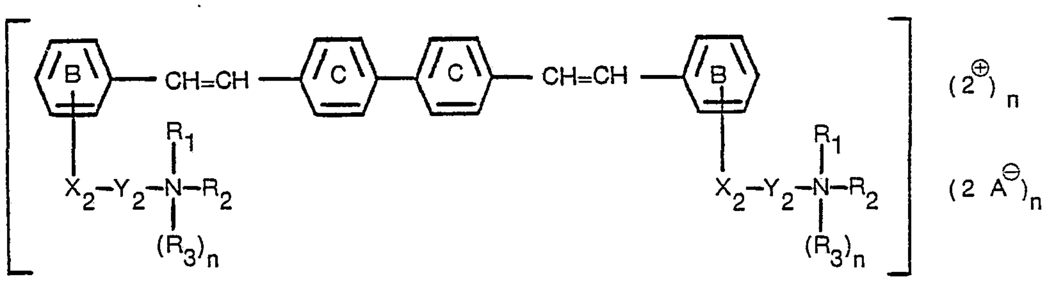

worin $X_2$ Sauerstoff, Schwefel, —CON($R_4$)— —OCO— oder —COO— wobei, wenn $n = 0$, $X_2$ nicht —CON($R_4$)— und wenn $n = 1$ und $X_2$ —CON($R_4$)— oder —COO— ist, $A^{\ominus}$ nicht Phosphit- oder Phosphonatanionen sein dürfen, $Y_2$ $C_{1-20}$-Alkylen oder durch eine Hydroxylgruppe substituiertes $C_{1-20}$-Alkylen, $R_1$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl, oder $R_1$ zusammen mit $R_2$ und dem N-Atom, an das sie gebunden sind, einen 5- bis 7-gliedrigen heterocyclischen Ring. $R_2$ unsubstituiertes oder durch Cyano, Hydroxy, Alkoxy, Phenyl, $C_{1-4}$-Akoxycarbonyl oder Chlor substituiertes $C_{1-8}$-Alkyl, $C_{3-4}$-Alkenyl oder $R_2$ zusammen mit $R_1$ und dem N-Atom, an das sie gebunden sind, einen 5- bis 7-gliedrigen heterocyclischen Ring, $R_3$ Wasserstoff, unsubstituiertes oder durch Cyano, Hydroxy, Alkoxy, Phenyl oder $C_{1-4}$-Alkoxycarbonyl substituiertes $C_{1-4}$-Alkyl, $R_1$, $R_2$ und $R_3$ zusammen mit dem N-Atom, an das sie gebunden sind, auch den Pyridin- oder Picolinring, $R_4$ Wasserstoff oder unsubstituiertes oder durch Cyano, Carbamoyl, Carbalkoxy, Hydroxy, Halogen oder $C_{1-4}$-Alkoxy substituiertes $C_{1-6}$-Alkyl, n die Zahl 0 oder 1 und $A^{\ominus}$ ein farbloses Anion bedeuten, wobei die Benzolkerne B und C auch nicht-chromophor substituiert sein können.

4. Aufhellmittel nach Anspruch 1 enthaltend eine Verbindung der Formel

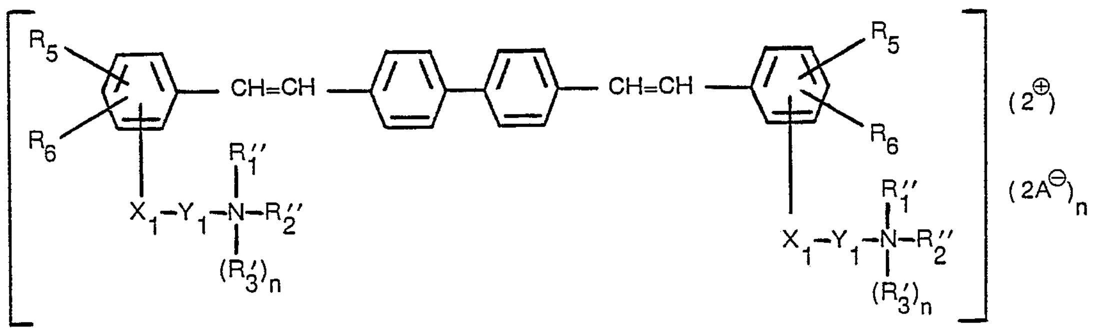

worin $X_1$ die direkte Bindung, Sauerstoff, Schwefel, —O—$C_{1-3}$-Alkylen-CONH—, —CONH—, —O—$C_{1-3}$-Alkylen-COO—, —OCO— oder —COO—, wobei, wenn n = 0, $X_1$ nicht —CONH— oder —O—$C_{1-3}$-Alkylen-CONH— und wenn n = 1 und $X_1$ —CONH— oder —COO— ist, $A^{\ominus}$ nicht Phosphit- oder Phosphonatanionen sein dürfen, $Y_1$ $C_{1-4}$-Alkylen oder Hydroxypropylen, $R_1''$ und $R_2''$ unabhängig voneinander $C_{1-4}$-Alkyl oder $R_1''$ und $R_2''$ zusammen mit dem N-Atom, an das sie bebunden sind, eine Pyrollidin-, Piperidin-, Hexamethylenimin- oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_{2-4}$-Hydroxyalkyl oder $C_{2-4}$-Cyanoalkyl, $R_1''$, $R_2''$ und $R_3'$ zusammen mit dem N-Atom, an das sie gebunden sind, auch den Pyridin- oder Picolinring, $R_5$ Wasserstoff, Chlor, $C_{1-4}$-Alkyl, $C_{3-4}$-Alkenyl, $C_{1-3}$-Alkoxy oder $R_5$ zusammen mit $R_6$ eine Trimethylen- oder Tetramethylengruppe, $R_6$ Wasserstoff, Chlor, $C_{1-4}$-Alkyl, $C_{1-3}$-Alkoxy oder $R_6$ zusammen mit $R_5$ eine Trimethylen- oder Tetramethylengruppe, n die Zahl 0 oder 1 und $A^{\ominus}$ ein farbloses Anion bedeuten.

5. Aufhellmittel nach Anspruch 4 enthaltend eine Verbindung der Formel

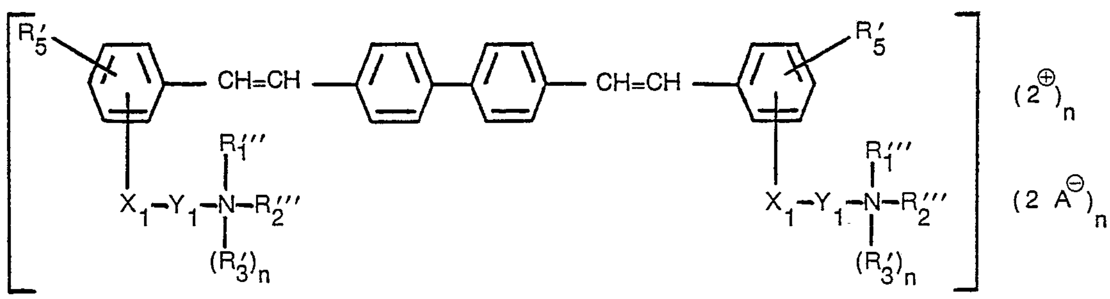

worin $X_1$ die direkte Bindung, Sauerstoff, Schwefel, —O—$C_{1-3}$-Alkylen-CONH—, —CONH—, —O—$C_{1-3}$-Alkylen-COO—, —OCO— oder —COO—, wobei, wenn n = 0, $X_1$ nicht —CONH— oder —O—$C_{1-3}$-Alkylen-CONH— und wenn n = 1 und $X_1$ —CONH— oder —COO— ist, $A^{\ominus}$ nicht Phosphit- oder Phosphanatanionen sein dürfen, $Y_1$ $C_{1-4}$-Alkylen oder Hydroxypropylen, $R_1'''$ und $R_2'''$ unabhängig voneinander $C_{1-4}$-Alkyl, $R_1'''$ und $R_2'''$ zusammen mit dem N-Atom, an das sie gebunden sind, einen Pyrrolidin-, Piperidin- oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_{2-4}$-Hydroxyalkyl oder $C_{2-4}$-Cyanoalkyl, $R_1'''$, $R_2'''$ und $R_3'$ zusammen mit dem N-Atom, an das die gebunden sind, auch den Pyridinring, $R_5'$ Wasserstoff, Chlor, $C_{1-4}$-Alkyl oder $C_{1-3}$-Alkoxy, n die Zahl 0 oder 1 und $A^{\ominus}$ ein farbloses Anion bedeuten.

6. Aufhellmittel nach Anspruch 4 enthaltend eine Verbindung der Formel

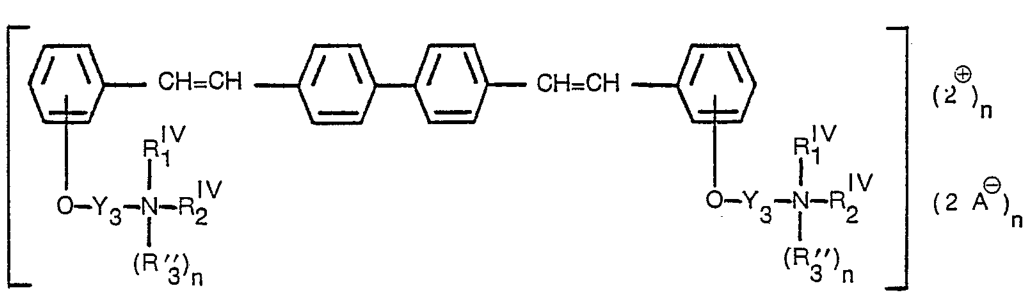

worin $Y_3$ $C_{2-4}$-Alkylen, $R_1^{IV}$ $C_{1-3}$-Alkyl oder $R_1^{IV}$ zusammen mit $R_2^{IV}$ und dem N-Atom, an das sie gebunden sind, einen Pyrrolidin-, Piperidin-, Hexamethylenimin- oder Morpholinring, $R_2^{IV}$ $C_{1-3}$-Alkyl oder $R_2^{IV}$ zusammen mit $R_1^{IV}$ und dem N-Atom, an das sie gebunden sind, einen Pyrrolidin-, Piperidin-, Hexamethylenimin- oder Morpholingring, $R_3''$ Wasserstoff oder $C_{1-3}$-Alkyl, n die Zahl 0 oder 1 und $A^{\ominus}$ ein farbloses Anion bedeuten.

7. Aufhellmittel nach Anspruch 6 enthaltend eine Verbindung der Formel

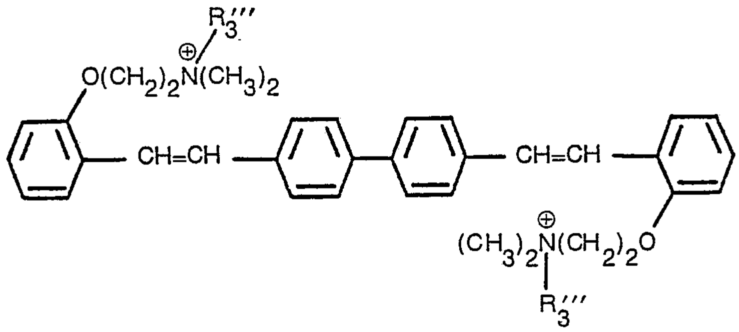

2 A'$^{\ominus}$

worin A'$^{\ominus}$ $CH_3OSO_3^{\ominus}$ oder $C_2H_5OSO_3^{\ominus}$ und $R_3'''$ Methyl oder Aethyl bedeuten.

8. Aufhellmittel nach Anspruch 6 enthaltend eine Verbindung der Formel

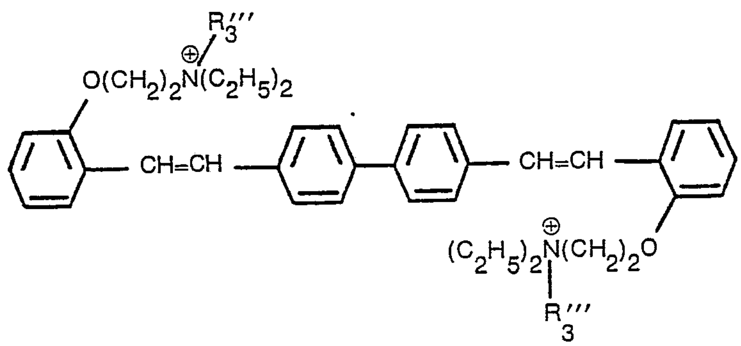

2 A'$^{\ominus}$

worin A'$^{\ominus}$ $CH_3OSO_3^{\ominus}$ oder $C_2H_5OSO_3^{\ominus}$ und $R_3'''$ Methyl oder Aethyl bedeuten.

9. Aufhellmittel nach Anspruch 6 enthaltend eine Verbindung der Formel

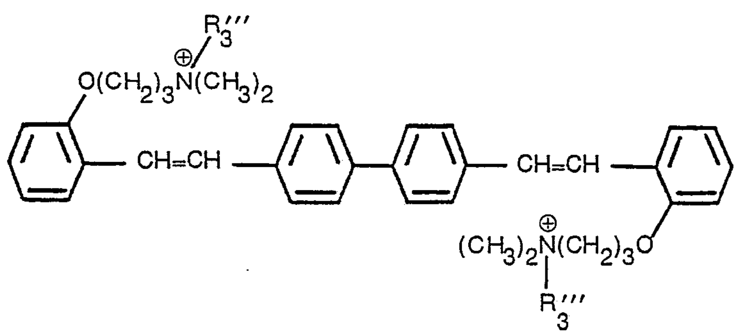

2 A'$^{\ominus}$

worin A'$^{\ominus}$$CH_3OSO_3^{\ominus}$ oder $C_2H_5OSO_3^{\ominus}$ und $R_3'''$ Methyl oder Aethyl bedeten.

10. Aufhellmittel nach Anspruch 1 enthaltend eine Verbindung der Formel

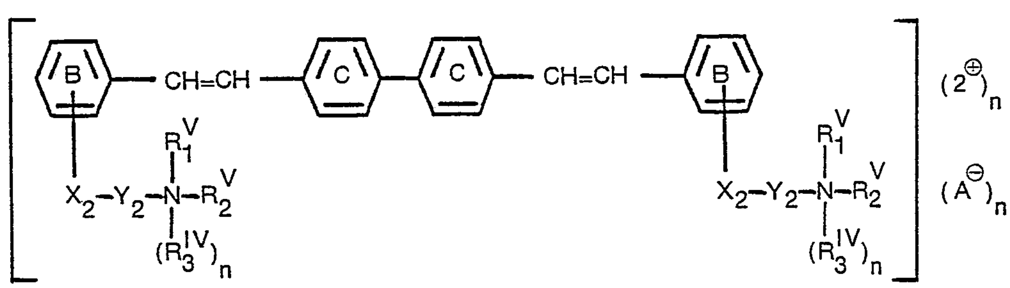

worin $X_2$ Sauerstoff, Schwefel

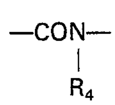

oder —COO—, wobei wenn $A^{\ominus}$ ein Phosphit- oder Phosphonatanion darstellt, $X_2$ nicht —CON($R_4$)— oder —COO— ist, $Y_2$ $C_{2-20}$-Alkylen, $R_1^V$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_1^V$ zusammen mit $R_2^V$ und dem N-Atom, an das sie gebunden sind, einen 5- bis 7-gliedrigen heterocyclischen Ring, $R_2^V$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_2^V$ zusammen mit $R_1^V$ und dem N-Atom, an das sie gebunden sind, einen 5- bis 7-gliedrigen heterocyclischen Ring, $R_3^{IV}$ Wasserstoff oder unsubstituiertes oder durch Cyano, Hydroxy, Alkoxy, Phenyl oder $C_{1-4}$-Alkoxycarbonyl substituiertes $C_{1-4}$-Alkyl, $R_4$ Wasserstoff oder unsubtituiertes oder durch Cyano, Carbamoyl, Carbalkoxy, Hydroxy, Halogen oder $C_{1-4}$-Alkoxy substituiertes $C_{1-6}$-Alkyl, $A^{\ominus}$ das Aequivalent eines farblosen Anions und n die Zahl 0 oder 1 bedeuten, wobei die Benzolkerne B und C auch nicht-chromophor substituiert sein können.

11. Aufhellmittel nach Anspruch 10 enthaltend eine Verbindung der Formel

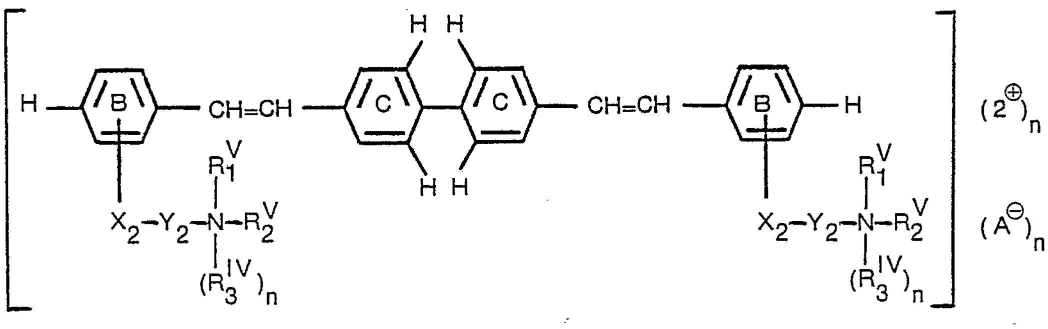

worin $X_2$, $Y_2$, $R_1^V$, $R_2^V$, $R_3^{IV}$, A und n die in Anspruch 10 angegebene Bedeutung haben und die Benzolkerne B und C auch nicht-chromophor substituiert sein können.

12. Aufhellmittel nach Anspruch 11 enthaltend eine Verbindung der Formel

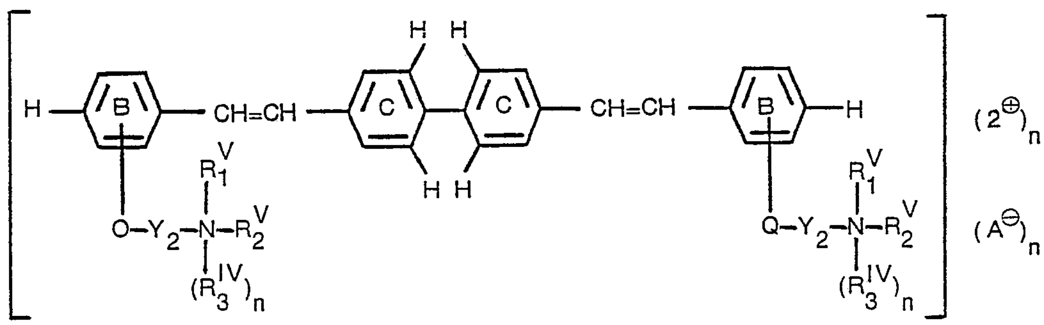

worin $Y_2C_{2-20}$-Alkylen, $R_1^V$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_1^V$ zusammen mit $R_2^V$ und dem N-Atom, an das sie gebunden sind, einen 5- bis 7-gliedrigen heterocyclischen Ring, $R_2^V$ unsubstituiertes oder durch Cyano, Hydroxy, Alkoxy, Phenyl, $C_{1-4}$-Alkoxycarbonyl oder Chlor substituiertes $C_{1-8}$-Alkyl, $C_{3-4}$-Alkenyl oder $R_2^V$ zusammen mit $R_1^V$ und dem N-Atom, an das sie gebunden sind, einen 5- bis 7-gliedrigen heterocyclischen Ring, $R_3^{IV}$ Wasserstoff oder unsubstituiertes oder durch Cyano, Hydroxy, Alkoxy, Phenyl oder $C_{1-4}$-Alkoxycarbonyl substituiertes $C_{1-4}$-Alkyl, $R_4$ Wasserstoff oder unsubstituiertes oder durch Cyano, Carbamoyl, Carbalkoxy, Hydroxy, Halogen oder $C_{1-4}$-Alkoxy substituiertes $C_{1-6}$-Alkyl, $A^{\ominus}$ das Aequivalent eines frablosen Anions und n die Zahl 0 oder 1 bedeuten, wobei die Benzolkerne B und C auch nicht-chromophor substituiert sein können.

13. Verfahren zur Herstellung von in Anspruch 1 definierten Verbindung, dadurch gekennzeichnet, dass man 1 moläquivalent eines Distyrylbiphenyls der Formel

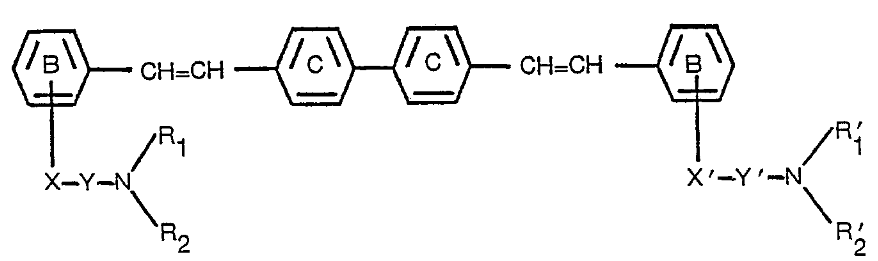

worin die Benzolringe B und C und X, X', Y, Y', $R_1$, $R_1'$, $R_2$ und $R_2'$ die entsprechende in Anspruch 1 angegebene Bedeutung haben, mit 1 Moläquivalent oder mit 2 Moläquivalenten eines Alkylierungsmittels bzw. einer Säure der Formel $R_3$—A quaterniert bzw. protoniert, worin $R_3$ und A die oben angegebene Bedeutung haben.

14. Verfahren zur Herstellung von in Anspruch 6 definierten Verbindung, dadurch gekennzeichnet, dass man im Molekularverhältnis 1:2 eine Verbindung der Formel

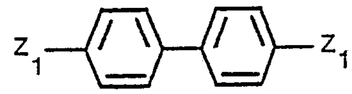

mit solchen der Formel

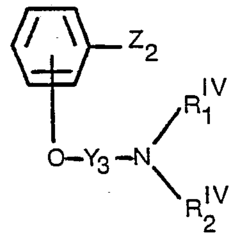

in Gegenwart einer starken Base umsetzt, in welchen Formeln $Y_3$, $R_1^{IV}$ und $R_2^{IV}$ die oben angegebene Bedeutung haben und eines der Symbole $Z_1$ und $Z_2$ eine OCH-Gruppe und das andere eine Gruppierung der Formel

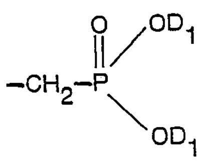 , 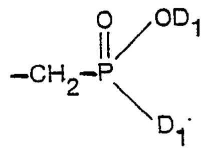 ,

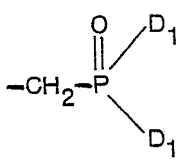 oder 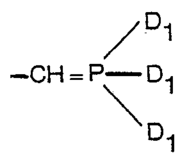

bedeutet, worin $D_1$ eine unsubstituierten oder substituierten Alkyl-, Aryl-, Cycloalkyl- oder Aralkylrest darstellt, und gewünschtenfalls die erhaltene Verbindung der Formel

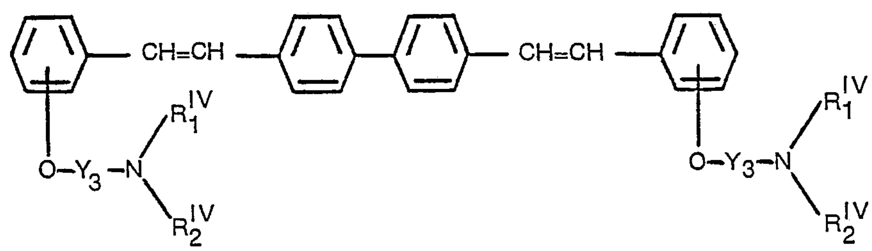

worin $Y_3$, $R_1^{IV}$ und $R_2^{IV}$ die oben angegebene Bedeutung haben, mit 2 Moläquivalenten eines Alkylierungsmittels bzw. einer Säure der Formel $R_3''$—A worin $R_3''$ und A die oben angegebene Bedeutung haben, quaterniert bzw. protoniert.

15. Verwendung von einer oder mehreren der in einem der Ansprüche 1 bis 12 definierten Verbindung zum optischen Aufhellen von organischen Materialien.

16. Verwendung nach Anspruch 15 zum optischen Aufhellen von Polyacrylnitril oder Cellulose als organischem Material.

17. Waschmittel enthaltend ein oder mehrere Distylrylbiphenyle der im Anspruch 1 definierten Formel.

18. Waschmittel gemäss Anspruch 17, welches 10 bis 70 Gew.% eines nicht-ionogenen Tensides und 1 bis 30% eines kationischen Textilweichmachers enthält.

19. Waschmittle gemäss Anspruch 18, welches 10 bis 70 Gew.% eines nicht-ionogenen Tensides und 1 bis 30 Gew.% eines quartären Derivates des Ammoniaks und/oder des Imidazolins mit je 2 langkettigen

aliphatischen Resten als kationischen Textilweichmacher und zur Verleihung der flüssigen Form ein Lösungsmittel enthält.

20. Waschmittel gemäss Anspruch 19, welches 1-Methyl-1-oleylamidoäthyl-2-oleyl-imidazolinium·$X^{\ominus}$, 1-Methyl-1-talgamidoäthyl-2-talg-imidazolinium·$X^{\ominus}$, Di-talg-dimethyl-ammonium·$X^{\ominus}$ oder eine Verbindung der Formel

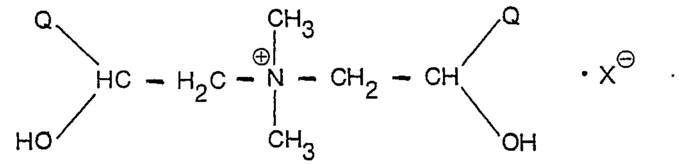

worin Q $C_{14-16}$-Alkyl bedeutet und $X^{\ominus}$ für ein Chlorid-, Bromid-, Methylsulfat-, Aethylsulfat-, Methansulfonat-, Aethansulfonat- oder Toluolsulfonatanion steht, als kationischen Textilweichmacher enthält.

21. Textilbehandlungsmittel, enthaltend ein oder mehrere Distyrylbiphenyle der im Anspruch 1 definierten Formel.

22. Wäschenachbehandlungsmittel, enthaltend eine oder mehrere Distyrylbiphenyle der im Anspruch 1 definierten Formel und einen kationischen Textilweichmacher.

**Claims for the Contracting States: DE GB FR CH LI IT NL BE SE**

1. A distyrylbiphenyl of the formula

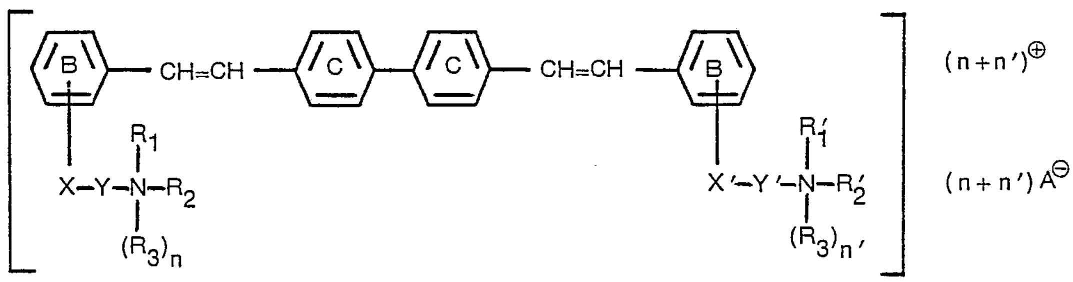

in which X and X' independently of each other are a direct bond, oxygen, sulfur, —O—$C_{1-3}$alkylene-CON($R_4$)—, —CON($R_4$)—, —O—$C_{1-3}$alkylene-COO—, —OCO— or —COO—, with the proviso that if n +n' = O, X and X' may not be —CON($R_4$)— or —O—$C_{1-3}$— alkylene—CON($R_4$)—, and if n + n' = 2 and X and X' is —(CON($R_4$)— or —COO, $A^{\ominus}$ may not be a phosphite or phosphonate anion, Y and Y' independently of each other are $C_{1-20}$— alkylene or $C_{1-0}$alkylene which is substituted by a hydroxyl group, $R_1$ and $R_1'$ independently of each other are $C_{1-8}$alkyl which is unsubstituted or substituted by cyano, hydroxy, alkoxy, phenyl, $C_{1-4}$alkoxycarbonyl or chlorine, or are $C_{3-4}$alkenyl, or $R_1$ together with $R_2$ and the nitrogen atom to which they are attached is a 5- to 7-membered heterocyclic ring and $R_1'$ together with $R_2'$ and the nitrogen atom to which they are attached is a 5- to 7-membered heterocyclic ring, $R_2$ and $R_2'$ independently of each other are $C_{1-8}$alkyl which is unsubstituted or substituted by cyano, hydroxy, alkoxy, phenyl, $C_{1-4}$alkoxy-carbonyl or chlorine, or are $C_{3-4}$alkenyl, or $R_2$ together with $R_1$ and the nitrogen atom to which they are attached is a 5- to 7-membered heterocyclic ring and $R_2'$ together with $R_1'$ and the nitrogen atom to which they are attached is a 5- to 7-membered heterocyclic ring, $R_3$ is hydrogen, $C_{1-4}$alkyl which is unsubstituted or substituted by cyano, hydroxy, alkoxy, phenyl or $C_{1-4}$alkoxycarbonyl, or is $C_{3-4}$alkenyl, or $R_1$, $R_2$ and $R_3$ together with the nitrogen atom to which they are attached are a pyridine or picoline ring and $R_1'$, $R_2'$ $R_3'$ together with the nitrogen atom to which they are attached are a pyridine or picoline ring, $R_4$ is hydrogen or $C_{1-6}$alkyl which is unsubstituted or substituted by cyano, carbamoyl, carbalkoxy, hydroxy, halogen or $C_{1-4}$alkoxy, $A^{\ominus}$ is a colourless anion and n and n' independently of each other are 0 or 1, and the benzene nuclei B and C may also be substituted by non-chromophoric substituents, and in which, if the nuclei B and C do not carry non-chromophoric substituents, the

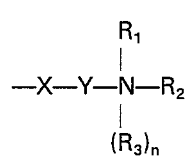 and 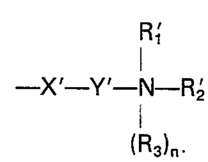

groups, as well as each of the two

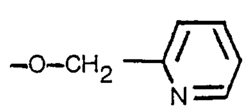

groups or each of the two

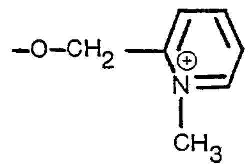

groups, may be ortho to the two —CH=CH— groups.

2. A distyrylbiphenyl according to claim 1 of the formula

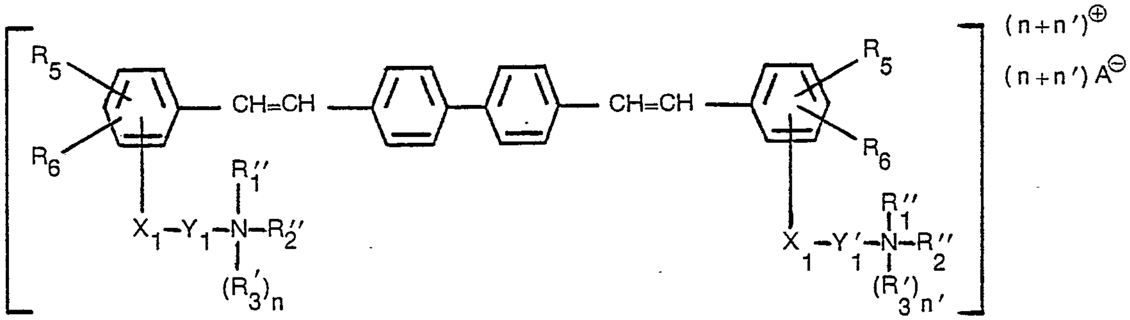

in which $X_1$ is a direct bond, oxygen, sulfur, —O—$C_{1-3}$alkylene-CONH—, —CONH—, —O—$_{1-3}$alkylene-COO—, —OCO— or —COO—, with the proviso that if $n + n' = 0$, $X_1$ may not be —CONH— or —O—$C_{1-3}$alkylene-CONH—, and if $n + n' = 2$ and $X_1$ is —CONH— or —COO—, $A^{\ominus}$ may not be phosphite or phosphonate anions, and $Y_1$ and $Y_1'$ independently of each other are $C_{1-4}$alkylene or hydroxypropylene, $R_1''$ and $R_2''$ independently of each other are $C_{1-4}$alkyl, or $R_1''$ and $R_2''$ together with the nitrogen atom to which they are attached are a pyrrolidine, piperidine, hexamethyleneimine or morpholine ring, $R_3'$ is hydrogen, $C_{1-4}$alkyl, $C_{3-4}$alkenyl, $C_{1-4}$alkoxycarbonylmethyl, benzyl, $C_{2-4}$hydroxyalkyl or $C_{2-4}$cyanoalkyl, or $R_1''$, $R_2''$ and $R_3'$ together with the nitrogen atom to which they are attached are a pyridine or picoline ring, $R_5$ is hydrogen, chlorine, $C_{1-4}$alkyl, $C_{3-4}$alkenyl or $C_{1-3}$alkoxy, or $R_5$ together with $R_6$ is a trimethylene or tetramethylene gorup, $R_6$ is hydrogen, chlorine, $C_{1-4}$alkyl or $C_{1-3}$alkoxy, or $R_6$ together with $R_6$ is a trimethylene or tetramethylene group, n and n' independently of each other are 0 or 1 and $A^{\ominus}$ is a colourless anion.

3. A distyrylbiphenyl according to claim 1 of the formula

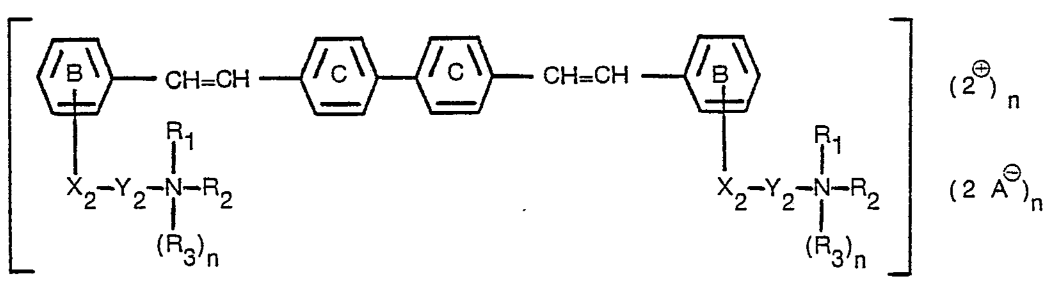

in which $X_2$ is oxygen, sulfur, —CON($R_4$)—, —OCO—, or —COO—, with the proviso that if $n = 0$, $X_2$ may not be —CON($R_4$)—, and if $n = 1$ and $X_2$ is —CON($R_4$)— or —COO—, $A^{\ominus}$ may not be phosphite or phosphonate anions, and $Y_2$ is $C_{1-20}$alkylene or $C_{1-20}$alkylene which is substituted by a hydroxyl group, $R_1$ is $C_{1-8}$alkyl which is unsubstituted or substituted by cyano, hydroxy, alkoxy, phenyl, $C_{1-4}$alkoxycarbonyl or chlorine, or is $C_{3-4}$alkenyl, or $R_1$ together with $R_2$ and the nitrogen atom to which they are attached is a 5- to 7-membered heterocyclic ring, $R_2$ is $C_{1-8}$alkyl which is unsubstituted or substituted by cyano, hydroxy, alkoxy, phenyl, $C_{1-4}$alkoxycarbonyl or chlorine, or is $C_{3-4}$alkenyl, or $R_2$ together with $R_1$ and the nitrogen atom to which they are attached is a 5- to 7-membered heterocyclic ring, $R_3$ is hydrogen or $C_{1-4}$alkyl which is unsubstituted or substituted by cyano, hydroxy, alkoxy, phenyl or $C_{1-4}$alkoxycarbonyl, or $R_1$, $R_2$ and $R_3$ together with the nitrogen to which they are attached are a pyridine or picoline ring, $R_4$ is hydrogen or $C_{1-6}$alkyl is unsubstituted or substituted by cyano, carbamoyl, carbalkoxy, hydroxy, halogen or $C_{1-4}$alkoxy, n is 0 or 1 and $A^{\ominus}$ is a colourless anion, and the benzene nuclei B and C may also be substituted by non-chromophoric substituents.

4. A distyrylbiphenyl according to claim 2 of the formula

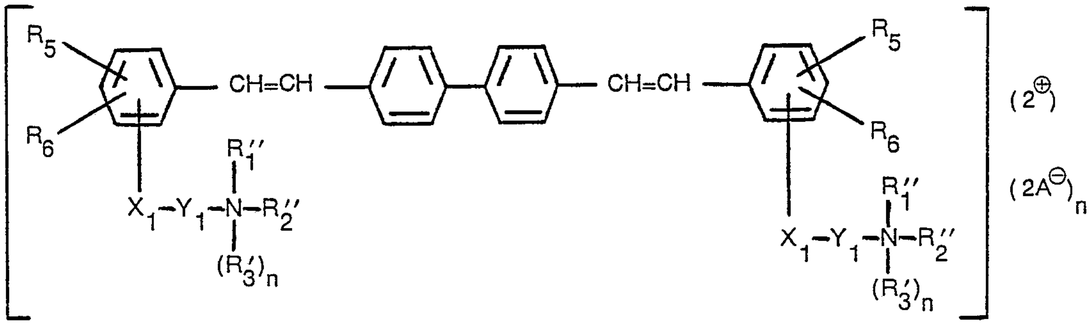

in which $X_1$ is a direct bond, oxygen, sulfur, —O—$C_{1-3}$alkylene-CONH—, —CONH—, —O—$C_{1-3}$alkylene-COO—, —OCO— or —COO—, with the proviso that if n = 0, $X_1$ may not be —CONH— or —O—$C_{1-3}$—alkylene- CONH—, and if n = 1 and $X_1$ is —CONH— r —COO—, $A^{\ominus}$ may not be phosphite or phosphonate anions, and $Y_1$ is $C_{1-4}$alkelene or hydroxypropylene, $R_1''$ and $R_2''$ independently of each other are $C_{1-4}$alkyl, or $R_1''$ and $R_2''$ together with the nitrogen atom to which they are attached are a pyrrolidine, piperidine, hexamethyleneimine or morpholine ring, $R_3'$ is hydrogen, $C_{1-4}$alkyl, $C_{3-4}$alkenyl, $C_{1-3}$alkoxycarbonylmethyl, benzyl, $C_{2-4}$hydroxyalkyl or $C_{2-4}$cyanoalkyl, or $R_1''$, $R_2''$ and $R_3'$ together with the nitrogen atom to which they are attached are a pyridine or picoline ring, $R_6$ is hydrogen, chlorine, $C_{1-4}$-alkyl, $C_{3-4}$alkenyl or $C_{1-3}$alkoxy, or $R_6$ together with $R_6$ is a trimethylene or tetramethylene group, $R_6$ is hydrogen, chlorine, $C_{1-4}$alkyl or $C_{1-3}$alkoxy, or $R_6$ together with $R_5$ is a trimethylene or tetramethylene group, n is 0 or 1 and $A^{\ominus}$ is a colourless anion.

5. A distyrylbiphenyl according to claim 4 of the formula

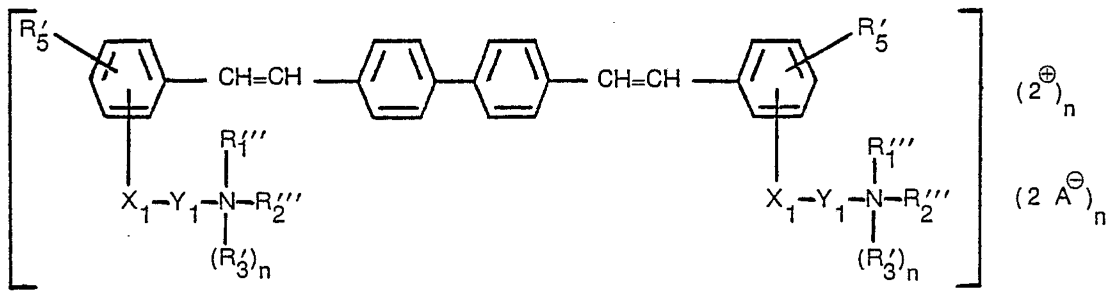

in which $X_1$ is a direct bond, oxygen, sulfur, —O—$C_{1-3}$alkylene-CONH—, —CONH—, —O—$C_{1-3}$alkylene-COO—, —OCO— or —COO—, with the proviso that if n = 0, $X_1$ may not be —CONH— or —O—$C_{1-3}$alkylene-CONH—, and if n = 1 and $X_1$ is —CONH— or —COO—, $A^{\ominus}$ may not be phosphite or phosphonate anions, and $Y_1$ is $C_{1-4}$— alkylene or hydroxypropylene, $R_1'''$ and $R_2'''$ independently of each other are $C_{1-4}$alkyl, $R_1'''$ and $R_2'''$ together with the nitrogen atom to which they are attached are a pyrrolidine, piperidine or morpholine ring, $R_3'$ is hydrogen, $C_{1-4}$alkyl, $C_{3-4}$alkenyl, $C_{1-3}$alkoxycarbonylmethyl, benzyl, $C_{2-4}$hydroxyalkyl or $C_{2-4}$cyanoalkyl, or $R_2'''$, $R_2'''$ and $R_3'$ together with the nitrogen atom to which they are attached are a pyridine ring, $R_5'$ is hydrogen, chlorine, $C_{1-4}$alkyl or $C_{1-3}$alkoxy, n is 0 or 1 and $A^{\ominus}$ is a colourless anion.

6. A distyrylbiphenyl according to claim 4 of the formula

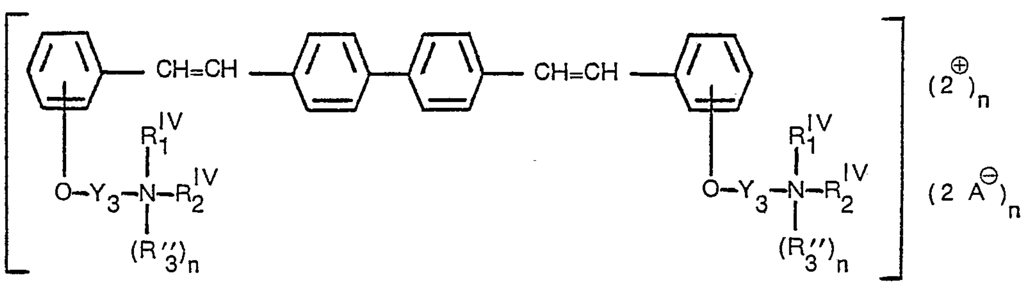

in which $Y_3$ is $C_{2-4}$alkylene, $R_1^{IV}$ is $C_{1-3}$alkyl, or $R_1^{IV}$ together with $R_2^{IV}$ and the nitrogen atom to which they are attached is a pyrrolidine, piperidine, hexamethyleneimine or morpholine ring, $R_1 8^{V}$ is $C_{2-3}$alkyl, or $R_2^{IV}$ together with $R_1^{IV}$ and the nitrogen atom to which they are attached is a pyrrolidine, piperidine, hexamethyleneimine or morpholine ring, $R_3''$ is hydrogen or $C_{1-3}$, n is 0 or 1 and $A^{\ominus}$ is a colourless anion.

7. A distyrylbiphenyl according to claim 6 of the formula

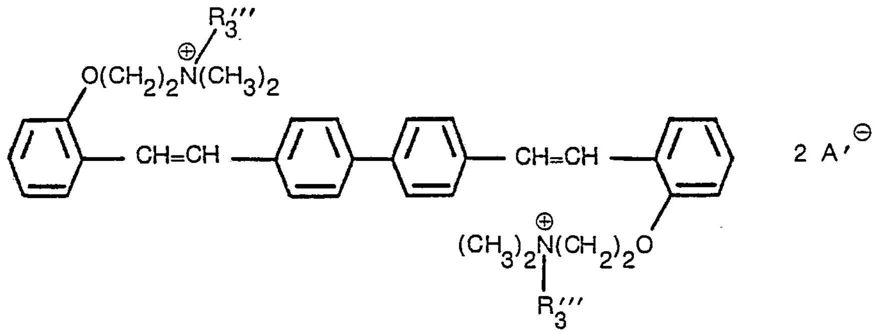

in which $A'^{\ominus}$ is $CH_3OSO_3^{\ominus}$ or $C_2H_5OSO_3^{\ominus}$ and $R_3'''$ is methyl or ethyl.

8. A distyrylbiphenyl according to claim 6 of the formula

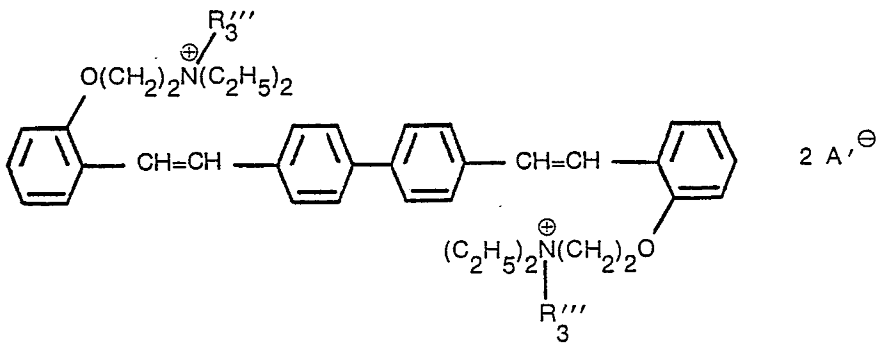

in which $A'^{\ominus}$ is $CH_3OSO_3^{\ominus}$ or $C_2H_5OSO_3^{\ominus}$ and $R_3'''$ is methyl or ethyl.

9. A distyrylbiphenyl according to claim 6 of the formula

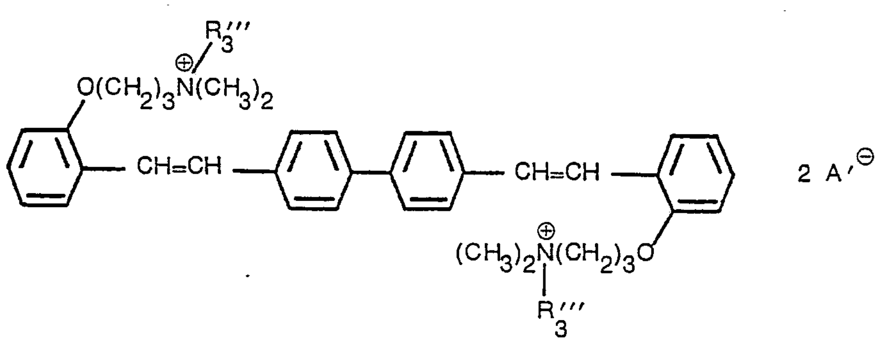

in which $A'^{\ominus}$ is $CH_3OSO_3^{\ominus}$ or $C_2H_5OSO_3^{\ominus}$ and $R_3'''$ is methyl or ethyl.

10. A distyrylbiphenyl according to claim 1 of the formula

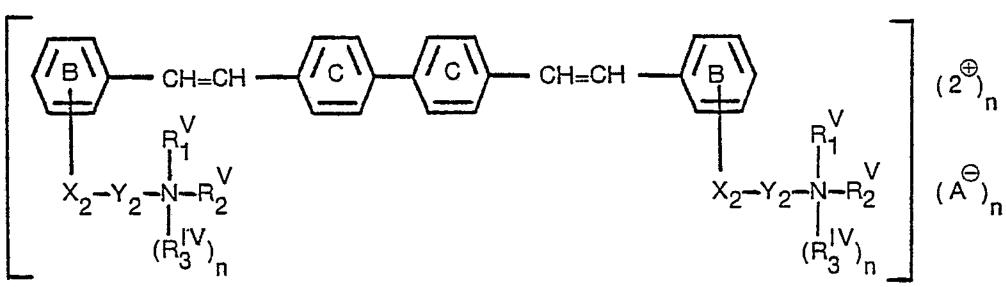

in which $X_2$ is oxygen, sulfur,

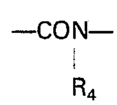

or —COO—, with the proviso that if $A^{\ominus}$ is a phosphite or phosphonate anion, $X_2$ is not —CON($R_4$)— or —COO—, and $Y_2$ is $C_{2-20}$alkylene, $R_1^V$ is $C_{1-8}$alkyl which is unsubstituted or substituted by cyano, hydroxy, alkoxy, phenyl, $C_{1-4}$alkoxycarbonyl or chlorine, or is $C_{3-4}$alkenyl, or $R_1^V$ together with $R_2^V$ and the nitrogen atom to which they are attached is a 5- to 7-membered heteroyclic ring, $R_2^V$ is $C_{1-8}$alkyl which is unsubstituted or substituted by cyano, hydroxy, alkoxy, phenyl, $C_{1-4}$alkoxycarbonyl or chlorine, or is $C_{3-4}$alkenyl, or $R_2^V$ together with $R_1^V$ and the nitrogen atom to which they are attached is a 5- to 7-membered heterocyclic ring, $R_3^{IV}$ is hydrogen or $C_{1-4}$alkyl which is unsubstituted or substituted by cyano, hydroxy, alkoxy, phenyl or $C_{1-4}$alkoxycarbonyl, $R_4$ is hydrogen or $C_{1-6}$alkyl which is unsubstituted or substituted by cyano, carbamoyl, carbalkoxy, hydroxy, halogen or $C_{1-4}$alkoxy, $A^{\ominus}$ is the equivalent of a colourless anion and n is 0 or 1, and the benzene nuclei B and C may also be substituted by non-chromophoric substituents.

11. A distyrylbiphenyl according to claim 10 of the formula

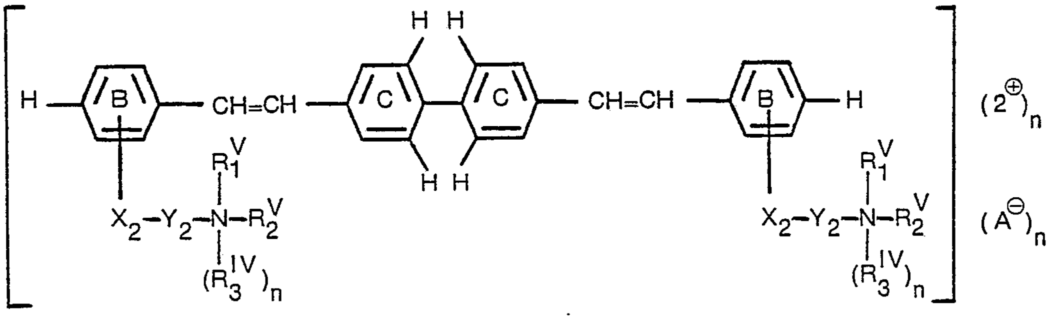

in which $X_2$, $Y_2$, $R_1^V$, $R_2^V$, $R_3^{IV}$, $A^{\ominus}$ and n are as defined in claim 10 and the benzene nuclei B and C may also be substituted by non-chromophoric substituents.

12. A distyrylbiphenyl according to claim 11 of the formula

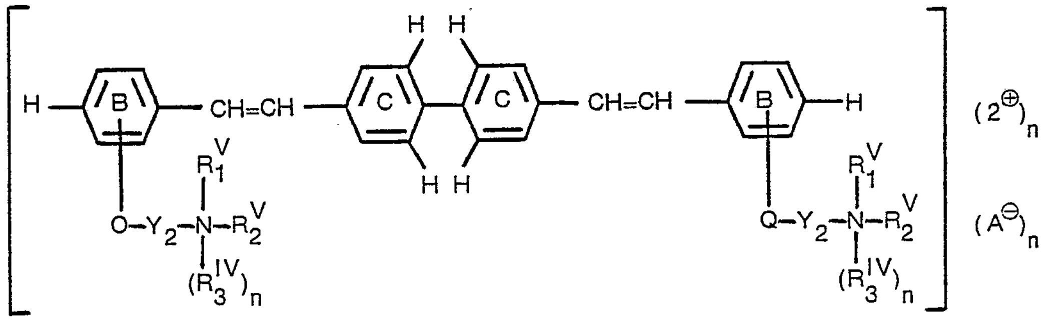

in which $Y_2$ is $C_{2-20}$alkylene, $R_1^V$ is $C_{1-8}$alky which is unsubstituted or substituted by cyano, hydroxy, alkoxy, phenyl, $C_{1-4}$alkoxycarbonyl or chlorine, or is $C_{3-4}$alkenyl, or $R_1^V$ together with $R_2^V$ and the nitrogen atom to which they are attached is a 5- to 7-membered heterocyclic ring, $R_2^V$ is $C_{1-8}$alkyl which is unsubstituted or substituted by cyano, hydroxy, alkoxy, phenyl, $C_{1-4}$alkoxycarbonyl or chlorine, or is $C_{3-4}$alkenyl, or $R_2^V$ together with $R_1^V$ and the nitrogen atom to which they are attached is a 5- to 7-membered heterocyclic ring, $R_3^{IV}$ is hydrogen or $C_{1-4}$alkyl which is unsubstituted or substituted by cyano, hydroxy, alkoxy, phenyl or $C_{1-4}$alkoxycarbonyl, $R_4$ is hydrogen or $C_{1-6}$alkyl which is unsubstituted or substituted by cyano, carbamoyl, carbalkoxy, hydroxy, halogen or $C_{1-4}$alkoxy, $A^{\ominus}$ is the equivalent of a colourless anion and n is 0 or 1, and the benzene nuclei B and C may also be substituted by non-chromophoric substituents.

13. A process for the preparation of a distyrylbiphenyl of the formula

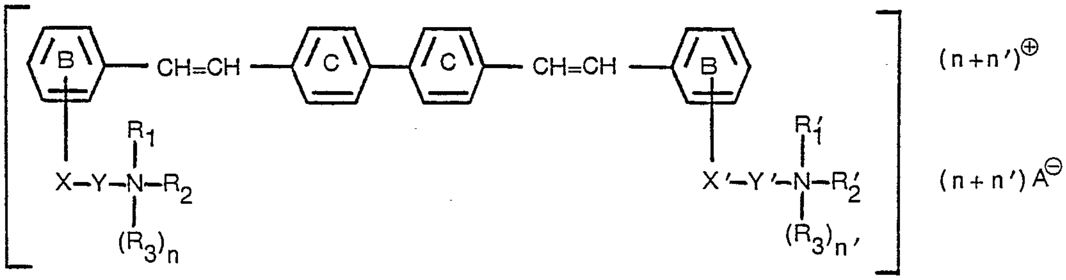

in which X and X' independently of each other are a direct bond, oxygen, sulfur, —O—$C_{1-3}$alkylene-

CON($R_4$)—, —CON($R_4$)—, —O—$C_{1-3}$alkylene-COO—, and —OCO— or —COO—, with the proviso that if n + n' = 0, X and X' may not be —CON($R_4$)— or —O—$C_{1-3}$-alkylene-CON($R_4$)—, and if n + n' = 2 and X and X' is —CON($R_4$)— or —COO—, $A^{\ominus}$ may not be a phosphite or phosphonate anion, Y and Y' independently of each other are $C_{1-20}$— alkylene or $C_{1-20}$alkylene which is substituted by a hydroxyl group, $R_1$ and $R_1'$ independently of each other are $C_{1-8}$alkyl which is unsubstituted or substituted by cyano, hydroxy, alkoxy, phenyl, $C_{1-4}$alkoxycarbonyl or chlorine, or are $C_{3-4}$alkenyl, or $R_1$ together with $R_2$ and the nitrogen atom to which they are attached is a 5- to 7-membered heterocyclic ring and $R_1'$ together with $R_2'$ and the nitrogen atom to which they are attached is a 5- to 7-membered heterocyclic ring, $R_2$ and $R_2'$ independently of each other are $C_{1-8}$alkyl which is unsubstituted or substituted by cyano, hydroxy, alkoxy, phenyl, $C_{1-4}$alkoxycarbonyl or chlorine, or are $C_{3-4}$alkenyl, or $R_2$ together with $R_1$ and the nitrogen atom to which they are attached is a 5- to 7-membered heterocyclic ring and $R_2'$ together with $R_1'$ and the nitrogen atom to which they are attached is a 5- to 7-membered heterocyclic ring, $R_3$ is hydrogen, $C_{1-4}$alkyl which is unsubstituted or substituted by cyano, hydroxy, alkoxy, phenyl or $C_{1-4}$alkoxycarbonyl, or is $C_{3-4}$alkenyl, or $R_1$, $R_2$ and $R_3$ together with the nitrogen atom to which they are attached are a pyridine or picoline ring and $R_1'$, $R_2'$ and $R_3'$ together with the nitrogen atom to which they are attached are a pyridine or picoline ring, $R_4$ is hydrogen or $C_{1-6}$alkyl which is unsubstituted or substituted by cyano, carbamoyl, carbalkoxy, hydroxy, halogen or $C_{1-4}$alkoxy, $A^{\ominus}$ is a colourless anion and n and n' independently of each other are 0 or 1, and the benzene nuclei B and C may also be substituted by non-chromophoric substituents, and in which, if the nuclei B and C do not crary non-chromophoric substituents, the

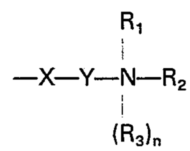 and 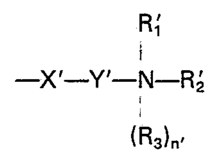

groups, as well as each of the two

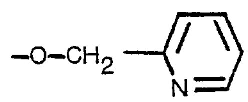

groups or each of the two

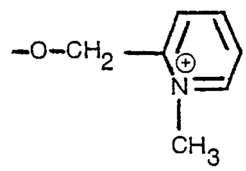

groups, may be ortho to the two —CH=CH— groups, which process comprises quaternising or protonising 1 mol equivalent of a distyrylbiphenyl of the formula

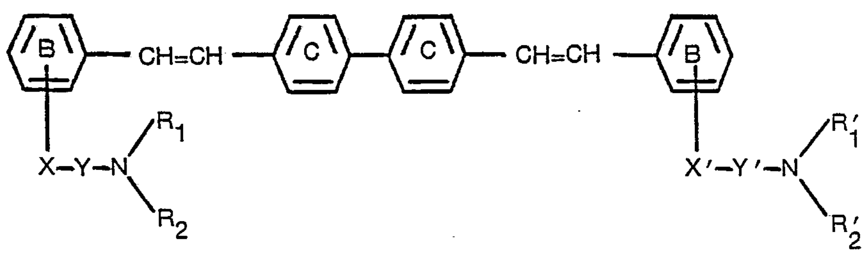

in which the benzene nuclei B and C and X, X', Y, Y', $R_1$, $R_1'$, $R_2$ and $R_2'$ are as defined above, with 1 mol equivalent or with 2 mol equivalents of an alkylating agent or, respectively, of an acid of the formula $R_3$—A, in which $R_3$ and A are as defined above.

14. A process for the preparation of a distyrylbiphenyl of the formula

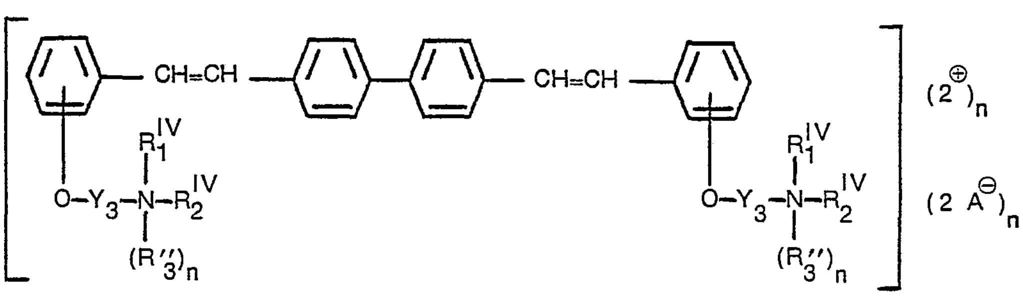

in which $Y_3$ is $C_{2-4}$alkylene, $R_1^{IV}$ is $C_1$-alkyl, or $R_1^{IV}$ together with $R_2^{IV}$ and the nitrogen atom to which they are attached is a pyrrolidine, piperidine, hexamethyleneimine or morpholine ring, $R_2^{IV}$ is $C_{1-3}$alkyl, or $R_2^{IV}$ together with $R_1^{IV}$ and the nitrogen atom to which they are attached is a pyrrolidine, piperidine, hexamethyleneimine or morpholine ring, $R_3'$ is hydrogen or $C_{1-3}$alkyl, n is 0 or and $A^{\ominus}$ is a colourless anion, which process comprises reacting a compound of the formula

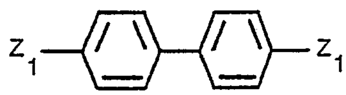

in a molecular ratio of 1:2 with a compound of the formula

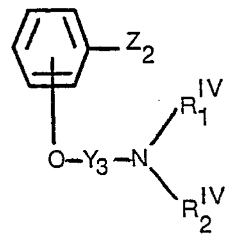

in the presence of a strong base, in which formulae $Y_3$, $R_1^{IV}$ and $R_1^{IV}$ are as defined above and one of the symbols $Z_1$ and $Z_2$ is an OCH group and the other is a grouping of the formula

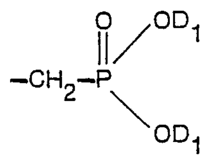
,
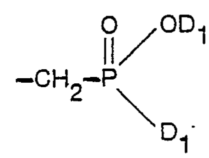
,

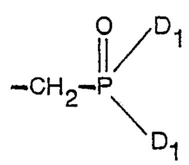
or
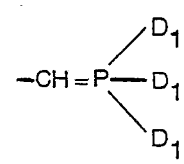

in which formulae $D_1$ is an unsubstituted or substituted alkyl, aryl, cycloalkyl or aralkyl radical, and, if desired, quaternising or protonising the resultant compound of the formula

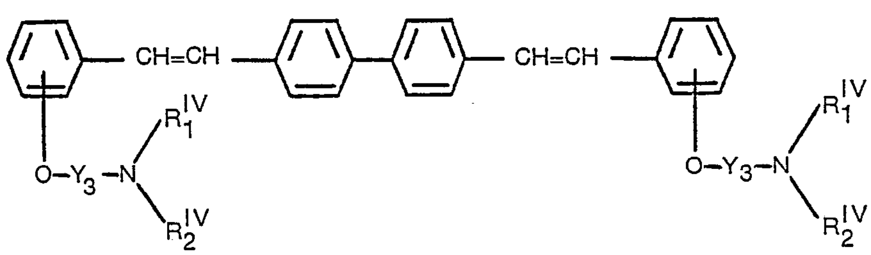

in which $Y_3$, $R_1^{IV}$ and $R_2^{IV}$ are as defined above, with 2 mol equivalents of an alkylating agent or, respectively, of an acid of the formula $R_3''$—A, in which $R_3''$ and A are as defined above.

15. A process for brightening organic material, which comprises incorporating a distyrylbiphenyl of the formula as defined in claim 1 into this material or applying such a distyrylbiphenyl to the surface of said material.

16. A process according to claim 15, wherein the organic material to be brightened is polyacrylonitrile or cellulose.

17. A detergent containing one or more distyrylbiphenyls of the formula as defined in claim 1.

18. A detergent according to claim 17, which contains 10 to 70% by weight of a nonionic surfactant and 1 to 30% of a cationic textile softener.

19. A detergent according to claim 18, which contains 10 to 70% by weight of a nonionic surfactant and 1 to 30% by weight of a quaternary derivative of ammonia and/or of imidazoline, each containing 2 long chain aliphatic radicals, as cationic textile softener, and a solvent to render it in the liquid form.

20. A detergent according to claim 19, which contains 1-methyl-1-oleylamidoethyl-2-oleyl-imidazolinium·$X^{\ominus}$, 1-methyl-1-tallow-amidoethyl-2-tallow-imidazolinium·$X^{\ominus}$, di-tallow-dimethyl-ammonium·$X^{\ominus}$ or a compound of the formula

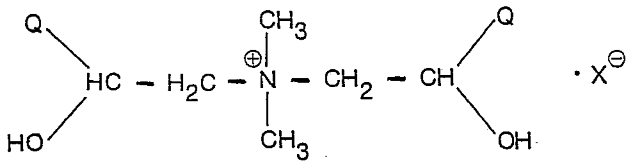

in which Q is $C_{14-16}$alkyl and $X^{\ominus}$ is a chloride, bromide, methylsulfate, ethylsulfate, methanesulfonate, ethanesulfonate or toluenesulfonate anion, as cationic textile softener.

21. A textile treatment agent containing one or more distyrylbiphenyls of the formula as defined in claim 1.

22. A laundry after-treatment agent containing one or more distyrylbiphenyls of the formula as defined in claim 1 and a cationic textile softener.

**Claims for the Contracting State: AT**

1. A fluorescent brightening agent containing a compound of the formula

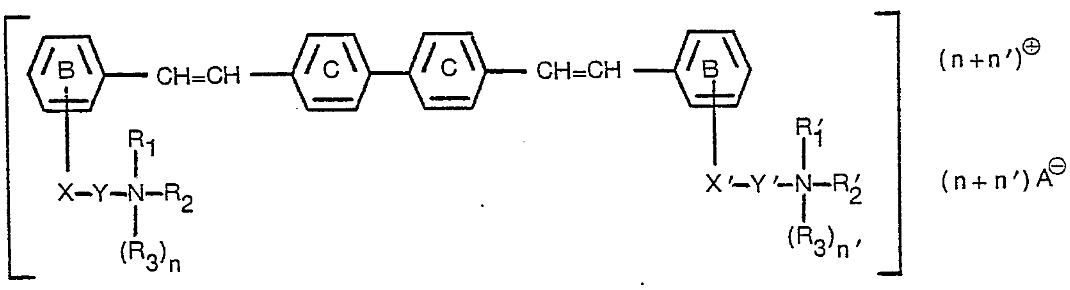

in which X and X′ independently of each other are a direct bond, oxygen, sulfur, —O—$C_{1-3}$alkylene-CON($R_4$)—, —CON($R_4$)—, —O—$C_{1-3}$alkylene-COO—, —OCO— or —COO—, with the proviso that if n +n′ = O, X and X′ may not be —CON($R_4$)— or —O—$C_{1-3}$— alkylene—CON($R_4$)—, and if n + n′ = 2 and X and X′ is —(CON($R_4$)— or —COO, $A^{\ominus}$ may not be a phosphite or phosphonate anion, Y and Y′ independently of each other are $C_{1-20}$— alkylene or $C_{1-0}$alkylene which is substituted by a hydroxyl group, $R_1$ and $R_1'$ independently of each other are $C_{1-8}$alkyl which is unsubstituted or substituted by cyano, hydroxy, alkoxy, phenyl, $C_{1-4}$alkoxycarbonyl or chlorine, or are $C_{3-4}$alkenyl, or $R_1$ together with $R_2$ and the nitrogen atom to which they are attached is a 5- to 7-membered heterocyclic ring and $R_1'$ together with $R_2'$ and the nitrogen atom to which they are attached is a 5- to 7-membered heterocyclic ring, $R_2$ and $R_2'$ independently of each other are $C_{1-8}$alkyl which is unsubstituted or substituted by cyano, hydroxy, alkoxy, phenyl, $C_{1-4}$alkoxy-carbonyl or chlorine, or are $C_{3-4}$alkenyl, or $R_2$ together with $R_1$ and the nitrogen atom to which they are attached is a 5- to 7-membered heterocyclic ring and $R_2'$ together with $R_1'$ and the nitrogen atom to which they are attached is a 5- to 7-membered heterocyclic ring, $R_3$ is hydrogen, $C_{1-4}$alkyl which is unsubstituted or substituted by cyano, hydroxy, alkoxy, phenyl or $C_{1-4}$alkoxycarbonyl, or is $C_{3-4}$alkenyl, or $R_1$, $R_2$ and $R_3$ together with the nitrogen atom to which they are attached are a pyridine or picoline ring and $R_1'$, $R_2'$ $R_3'$ together with the nitrogen atom to which they are attached are a pyridine or picoline ring, $R_4$ is hydrogen or $C_{1-6}$alkyl which is unsubstituted or substituted by cyano, carbamoyl, carbalkoxy, hydroxy, halogen or $C_{1-4}$alkoxy, $A^{\ominus}$ is a colourless anion and n and n′ independently of each other are 0 or 1, and the benzene nuclei B and C may also be substituted by non-chromophoric substituents, and in which, if the nuclei B and C do not carry non-chromophoric substituents, the

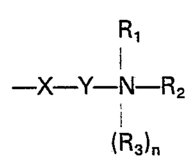

and

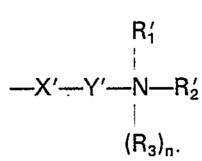

groups, as well as each of the two

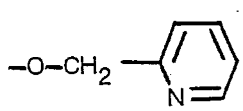

groups or each of the two

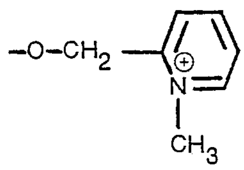

groups, may be ortho to the two —CH=CH— groups, together with adjuvants suitable for fluorescent brightening agents.

2. A fluorescent brightening agent according to claim 1 containing a compound of the formula

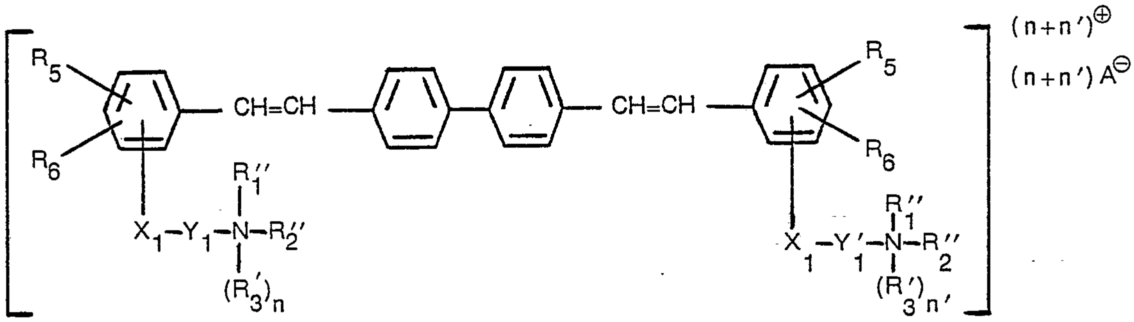

in which $X_1$ is a direct bond, oxygen, sulfur, —O—$C_{1-3}$alkylene-CONH—, —CONH—, —O—$_{1-3}$alkylene-COO—, —OCO— or —COO—, with the proviso that if $n + n' = 0$, $X_1$ may not be —CONH— or —O—$C_{1-3}$alkylene-CONH—, and if $n + n' = 2$ and $X_1$ is —CONH— or —COO—, $A^{\ominus}$ may not be phosphite or phosphonate anions, and $Y_1$ and $Y_1'$ independently of each other are $C_{1-4}$alkylene or hydroxypropylene, $R_1''$ and $R_2''$ independently of each other are $C_{1-4}$alkyl, or $R_1''$ and $R_2''$ together with the nitrogen atom to which they are attached are a pyrrolidine, piperidine, hexamethyleneimine or morpholine ring, $R_3'$ is hydrogen, $C_{1-4}$alkyl, $C_{3-4}$alkenyl, $C_{1-3}$alkoxycarbonylmethyl, benzyl, $C_{2-4}$hydroxyalkyl or $C_{2-4}$cyanoalkyl, or $R_1''$, $R_2''$ and $R_3'$ together with the nitrogen atom to which they are attached are a pyridine or picoline ring, $R_5$ is hydrogen, chlorine, $C_{1-4}$alkyl, $C_{3-4}$alkenyl or $C_{1-3}$alkoxy, or $R_5$ together with $R_6$ is a trimethylene or tetramethylene group, $R_6$ is hydrogen, chlorine, $C_{1-4}$alkyl or $C_{1-3}$alkoxy, or $R_6$ together with $R_5$ is a trimethylene or tetramethylene group, n and n' independently of each other are 0 or 1 and $A^{\ominus}$ is a colourless anion.

3. A fluorescent brightening agent according to claim 1 containing a compound of the formula

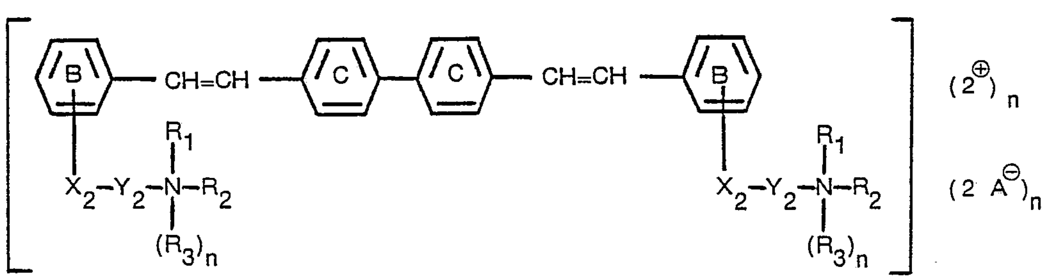

in which $X_2$ is oxygen, sulfur, —CON($R_4$)—, —OCO—, or —COO—, with the proviso that if n = 0, $X_2$ may not be —CON($R_4$)—, and if n = 1 and $X_2$ is —CON($R_4$)— or —COO—, $A^{\ominus}$ may not be phosphite or phosphonate anions, and $Y_2$ is $C_{1-20}$alkylene or $C_{1-20}$alkylene which is substituted by a hydroxyl group, $R_1$ is $C_{1-8}$alkyl which is unsubstituted or substituted by cyano, hydroxy, alkoxy, phenyl, $C_{1-4}$alkoxycarbonyl or chlorine, or is $C_{3-4}$alkenyl, or $R_1$ together with $R_2$ and the nitrogen atom to which they are attached is a 5- to 7-membered heterocyclic ring, $R_2$ is $C_{1-8}$alkyl which is unsubstituted or substituted by cyano, hydroxy, alkoxy, phenyl, $C_{1-4}$alkoxycarbonyl or chlorine, or is $C_{3-4}$alkenyl, or $R_2$ together with $R_1$ and the nitrogen atom to which they are attached is a 5- to 7-membered heterocyclic ring, $R_3$ is hydrogen or $C_{1-4}$alkyl which is unsubstituted or substituted by cyano, hydroxy, alkoxy, phenyl or $C_{1-4}$alkoxycarbonyl, or $R_1$, $R_2$ and $R_3$ together with the nityrogen to which they are attached are a pyridine or picolite ring, $R_4$ is hydrogen or $C_{1-6}$alkyl is unsubstituted or substituted by cyano, carbamoyl, carbalkoxy, hydroxy, halogen or $C_{1-4}$alkoxy, n is 0 or 1 and $A^{\ominus}$ is a coourless anion, and the benzene nuclei B and C may also be substituted by non-chromophoric substituents.

4. A fluorescent brightening agent according to claim 2 containing a compound of the formula

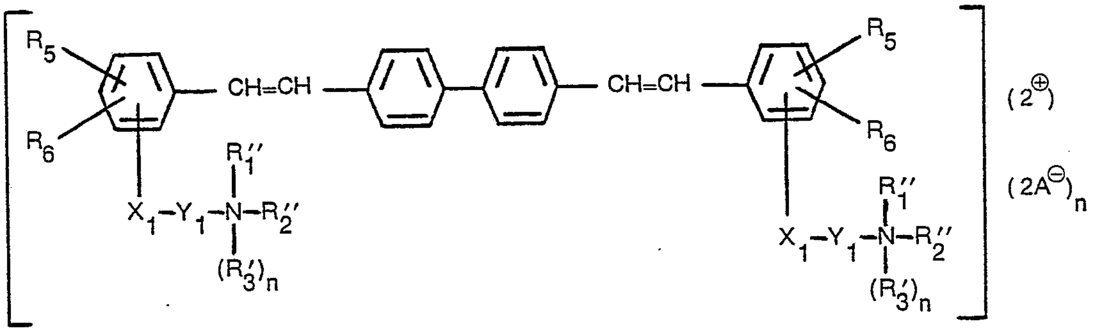

in which $X_1$ is a direct bond, oxygen, sulfur, —O—$C_{1-3}$alkylene-CONH—, —CONH—, —O—$C_{1-3}$alkylene-COO—, —OCO— or —COO—, with the proviso that if n = O, $X_1$ may not be —CONH— or —O—$C_{1-3}$—alkylene- CONH—, and if n = 1 and $X_1$ is —CONH— or —COO—, $A^{\ominus}$ may not be phosphite or phosphonate anions, and $Y_1$ is $C_{1-4}$alkelene or hydroxypropylene, $R_1''$ and $R_2''$ independently of each other are $C_{1-4}$alkyl, or $R_1''$ and $R_2''$ together with the nitrogen atom to which they are attached are a pyrrolidine, piperidine, hexamethyleneimine or morpholine ring, $R_3'$ is hydrogen, $C_{1-4}$alkyl, $C_{3-4}$alkenyl, $C_{1-3}$alkoxycarbonylmethyl, benzyl, $C_{2-4}$hydroxyalkyl or $C_{2-4}$cyanoalkyl, or $R_1''$, $R_2''$ and $R_3'$ together with the nitrogen atom to which they are attached are a pyridine or picoline ring, $R_5$ is hydrogen, chlorine, $C_{1-4}$-alkyl, $C_{3-4}$alkenyl or $C_{1-3}$alkoxy, or $R_6$ together with $R_6$ is a trimethylene or tetramethylene group, $R_6$ is hydrogen, chlorine, $C_{1-4}$alkyl or $C_{1-3}$alkoxy, or $R_6$ together with $R_5$ is a trimethylene or tetramethylene group, n is 0 or 1 and $A^{\ominus}$ is a colourless anion.

5. A fluorescent brightening agent according to claim 4 containing a compound of the formula

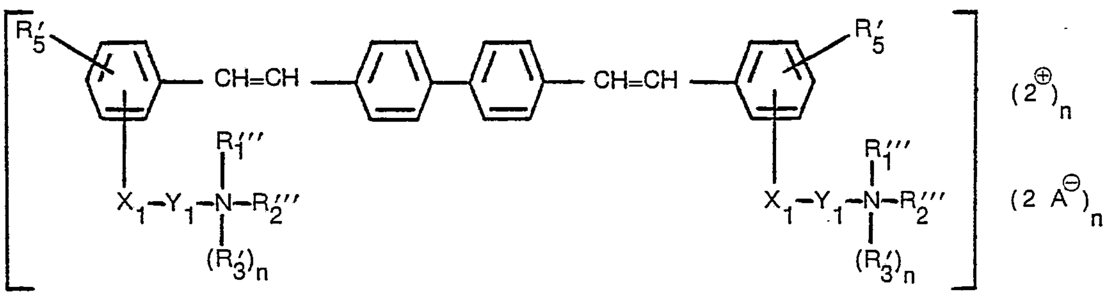

in which $X_1$ is a direct bond, oxygen, sulfur, —O—$C_{1-3}$alkylene-CONH—, —CONH—, —O—$C_{1-3}$alkylene-COO—, —OCO— or —COO—, with the proviso that if n = 0, $X_1$ may not be —CONH— or —O—$C_{1-3}$alkylene-CONH—, and if n = 1 and $X_1$ is —CONH— or —COO—, $A^{\ominus}$ may not be phosphite or phosphonate anions, and $Y_1$ is $C_{1-4}$— alkylene or hydroxypropylene, $R_1'''$ and $R_2'''$ independently of each other are $C_{1-4}$alkyl, $R_1'''$ and $R_2'''$ together with the nitrogen atom to which they are attached are a pyrrolidine, piperidine or morpholine ring, $R_3'$ is hydrogen, $C_{1-4}$alkyl, $C_{3-4}$alkenyl, $C_{1-3}$alkoxycarbonylmethyl, benzyl, $C_{2-4}$hydroxyalkyl or $C_{2-4}$cyanoalkyl, or $R_1'''$, $R_2'''$ and $R_3'$ together with the nitrogen atom to which they are attached are a pyridine ring, $R_5'$ is hydrogen, chlorine, $C_{1-4}$alkyl or $C_{1-3}$alkoxy, n is 0 or 1 and $A^{\ominus}$ is a colourless anion.

6. A fluorescent brightening agent according to claim 4 containing a compound of the formula

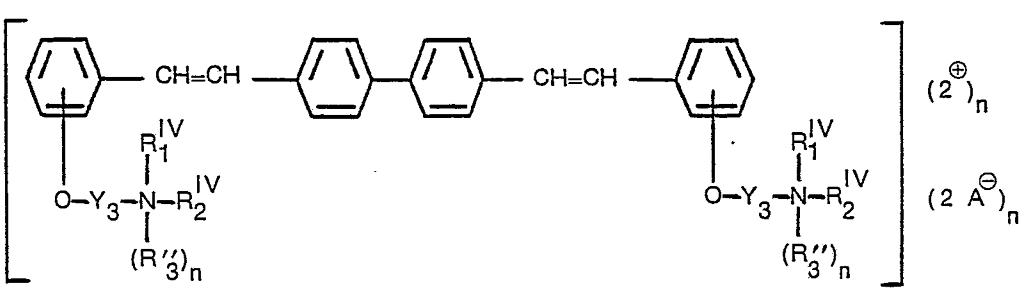

in which $Y_3$ is $C_{2-4}$alkylene, $R_1^{IV}$ is $C_{1-3}$alkyl, or $R_1^{IV}$ together with $R_2^{IV}$ and the nitrogen atom to which they are attached is a pyrrolidine, piperidine, hexamethyleneimine or morpholine ring, $R_2^{IV}$ is $C_{2-3}$alkyl, or $R_2^{IV}$ together with $R_1^{IV}$ and the nitrogen atom to which they are attached is a pyrrolidine, piperidine,

hexamethyleneimine or morpholine ring, $R_3''''$ is hydrogen or $C_{1-3}$ alkyl, n is 0 or 1 and $A^{\ominus}$ is a colourless anion.

7. A fluorescent brightening agent according to claim 6 containing a compound of the formula

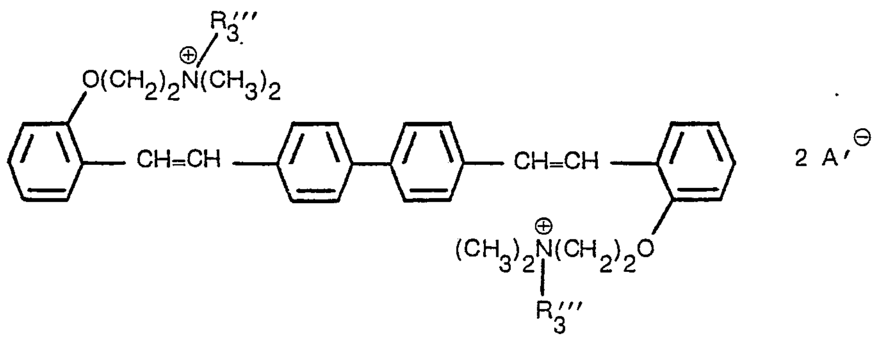

in which $A'^{\ominus}$ is $CH_3OSO_3^{\ominus}$ or $C_2H_5OSO_3^{\ominus}$ and $R_3'''$ is methyl or ethyl.

8. A fluorescent brightening agent according to claim 6 containing a comound of the formula

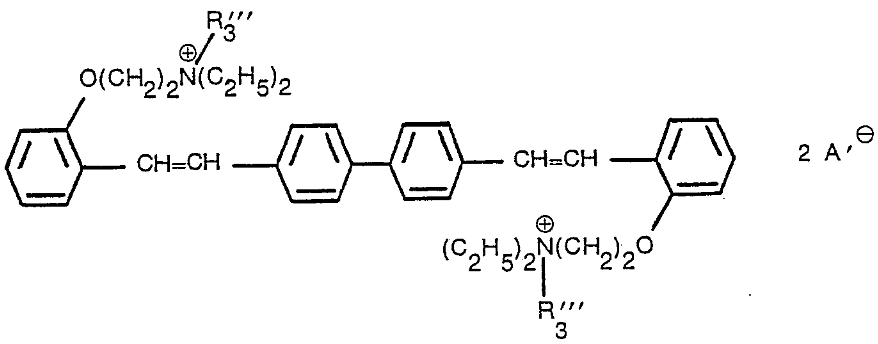

in which $A'^{\ominus}$ is $CH_3OSO_3^{\ominus}$ or $C_2H_5OSO_3^{\ominus}$ and $R_3'''$ is methyl or ethyl.

9. A fluorescent brightening agent according to claim 6 containing a compound of the formula

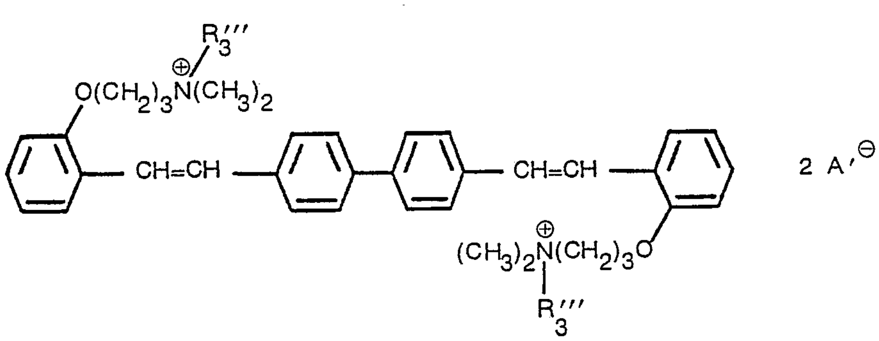

in which $A'^{\ominus}$ is $CH_3OSO_3^{\ominus}$ or $C_2H_5OSO_3^{\ominus}$ and $R_3'''$ is methyl or ethyl.

10. A fluourescent brightening agent according to claim 1 containing a compound of the formula

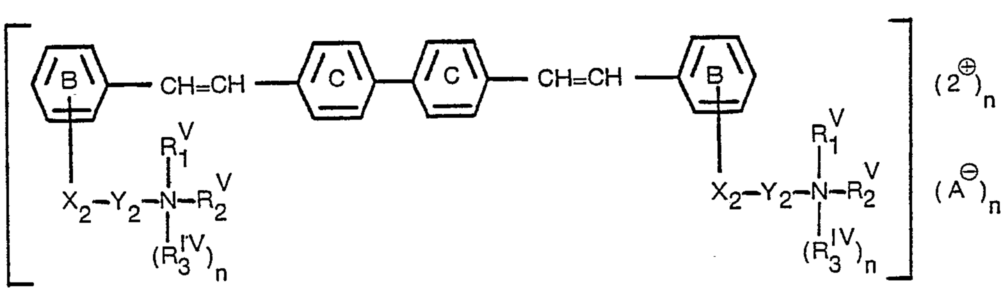

in which $X_2$ is oxygen, sulfur,

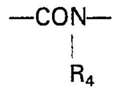

or —COO—, with the proviso that if $A^\ominus$ is a phosphite or phosphonate anion, $X_2$ is not —CON($R_4$)— or —COO—, and $Y_2$ is $C_{2-20}$alkylene, $R_1^V$ is $C_{1-8}$alkyl which is unsubstituted or substituted by cyano, hydroxy, alkoxy, phenyl, $C_{1-4}$alkoxycarbonyl or chlorine, or is $C_{3-4}$alkenyl, or $R_1^V$ together with $R_2^V$ and the nitrogen atom to which they are attached is a 5- to 7-membered heteroyclic ring, $R_2^V$ is $C_{1-8}$alkyl which is unsubstituted or substituted by cyano, hydroxy, alkoxy, phenyl, $C_{1-4}$alkoxycarbonyl or chlorine, or is $C_{3-4}$alkenyl, or $R_2^V$ together with $R_1^V$ and the nitrogen atom to which they are attached is a 5- to 7-membered heterocyclic ring, $R_3^{IV}$ is hydrogen or $C_{1-4}$alkyl which is unsubstituted or substituted by cyano, hydroxy, alkoxy, phenyl or $C_{1-4}$alkoxycarbonyl, $R_4$ is hydrogen or $C_{1-6}$alkyl which is unsubstituted or substituted by cyano, carbamoyl, carbalkoxy, hydroxy, halogen or $C_{1-4}$alkoxy, $A^\ominus$ is the equivalent of a colourless anion and n is 0 or 1, and the benzene nuclei B and C may also be substituted by non-chromophoric substituents.

11. A fluorescent brightening agent according to claim 10 containing a compound of the formula

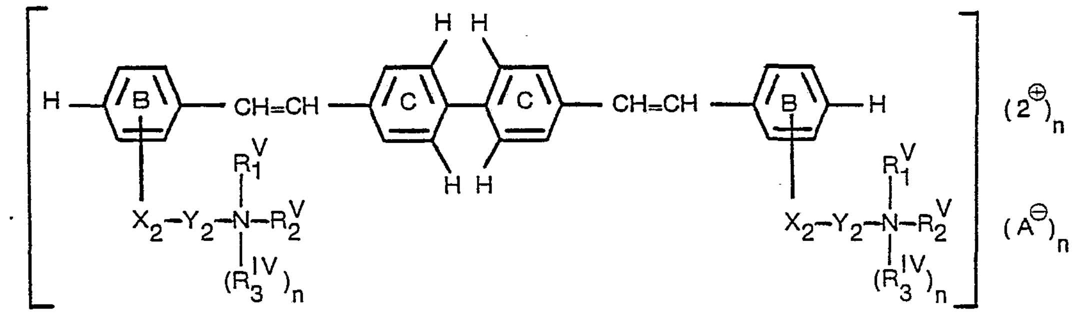

in which $X_2$, $Y_2$, $R_1^V$, $R_2^V$, $R_3^{IV}$, $A^\ominus$ and n are as defined in claim 10 and the benzene nuclei B and C may also be substituted by non-chromophoric substituents.

12. A fluorescent brightening agent according to claim 11 containing a compound of the formula

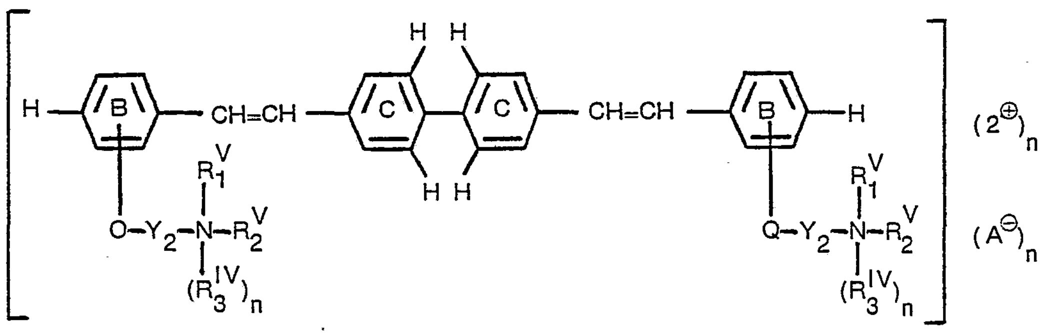

in which $Y_2$ is $C_{2-20}$alkylene, $R_1^V$ is $C_{1-8}$alky which is unsubstituted or substituted by cyano, hydroxy, alkoxy, phenyl, $C_{1-4}$alkoxycarbonyl or chlorine, or is $C_{3-4}$alkenyl, or $R_1^V$ together with $R_2^V$ and the nitrogen atom to which they are attached is a 5- to 7-membered heterocyclic ring, $R_2^V$ is $C_{1-8}$alkyl which is unsubstituted or substituted by cyano, hydroxy, alkoxy, phenyl, $C_{1-4}$alkoxycarbonyl or chlorine, or is $C_{3-4}$alkenyl, or $R_2^V$ together with $R_1^V$ and the nitrogen atom to which they are attached is a 5- to 7-membered heterocyclic ring, $R_3^{IV}$ is hydrogen or $C_{1-4}$alkyl which is unsubstituted or substituted by cyano, hydroxy, alkoxy, phenyl or $C_{1-4}$alkoxycarbonyl, $R_4$ is hydrogen or $C_{1-6}$alkyl which is unsubstituted or substituted by cyano, carbamoyl, carbalkoxy, hydroxy, halogen or $C_{1-4}$alkoxy, $A^\ominus$ is the equivalent of a colourless anion and n is 0 or 1, and the benzene nuclei B and C may also be substituted by non-chromophoric substituents.

13. A process for the preparation of a compound as defined in claim 1, which process comprises quaternising or protonising 1 mol equivalent of a distyrylbiphenyl of the formula

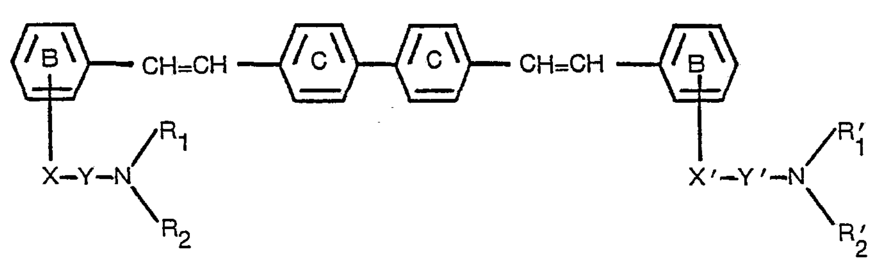

in which the benzene nuclei B and C and X, X', Y, Y', $R_1$, $R_1'$, $R_2$ and $R_2'$ are as defined above, with 1 mol equivalent or with 2 mol equivalents of an alkylating agent, or respectively, of an acid of the formula in which $R_3$ and A are as defined above.

14. A process for the preparation of a compound of as defined in claim 6, which process comprises reacting a compound of the formula

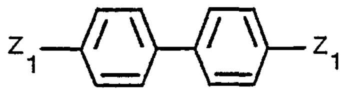

in a molecular ratio of 1:2 with a compound of the formula

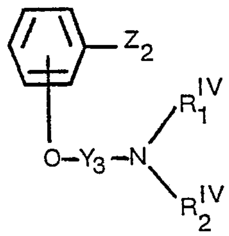

in the presence of a strong base, in which formulae $Y_3$, $R_1^{IV}$ $R_2^{IV}$ are as defined above and one of the symbols $Z_1$ and $Z_2$ is an OCH group and the other is a grouping of the formula

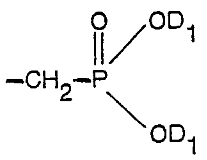 , 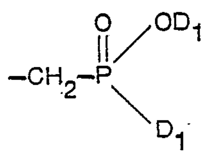 ,

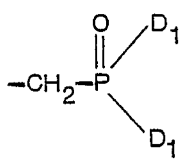 or 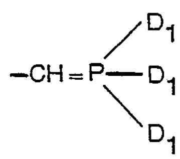

in which formulae $D_1$ is an unsubstituted or substituted alkyl, aryl, cycloalkyl or aralkyl radical, and, if desired, quaternising or protonising the resultant compound of the formula

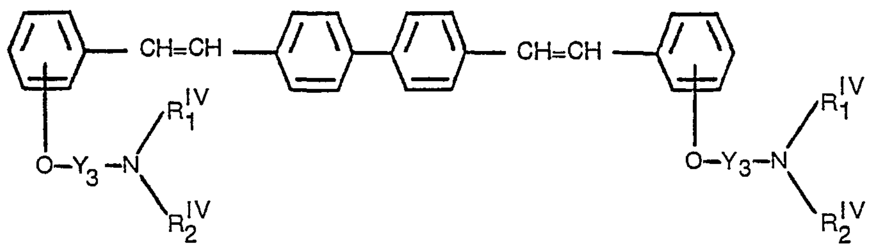

in which $Y_3$, $R_1^{IV}$ and $R_2^{IV}$ are as defined above, with 2 mol equivalents of an alkylating agent or, respectively, of an acid of the formula $R_3''$—A, in which $R_3''$ and A are as defined above.

15. Use of one or more of the compounds as defined in any one of claims 1 to 12 for brightening organic material.

16. Use according to claim 15, wherein the organic material to be brightened is polyacrylonitrile or cellulose.

17. A detergent containing one or more distyrylbiphenyls of the formula as defined in claim 1.

18. A detergent according to claim 17, which contains 10 to 70% by weight of a nonionic surfactant and 1 to 30% of a cationic textile softener.

19. A detergent according to claim 18, which contains 10 to 70% by weight of a nonionic surfactant and 1 to 30% by weight of a quaternary derivative of ammonia and/or of imidazoline, each containing 2 long chain aliphatic radicals, as cationic textile softener, and a solvent to render it in the liquid form.

20. A detergent according to claim 19, which contains 1-methyl-1-oleylamidoethyl-2-oleyl-imidazolinium·$X^{\ominus}$, 1-methyl-1-tallow amidoethyl-2-tallow-imidazolinium·$X^{\ominus}$, di-tallow-dimethyl-ammonium·$X^{\ominus}$ or a compound of the formula

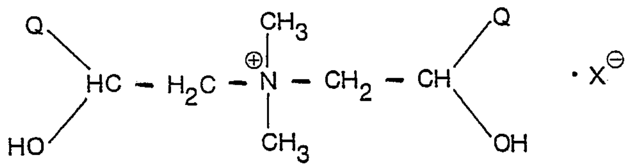

in which Q is $C_{14-16}$alkyl and $X^{\ominus}$ is a chloride, bromide, methylsulfate, ethylsulfate, methanesulfonate, ethanesulfonate or toluenesulfonate anion, as cationic textile softener.

21. A textile treatment agent containing one or more distyrylbiphenyls of the formula as defined in claim 1.

22. A laundry after-treatment agent containing one or more distyrylbiphenyls of the formula as defined in claim 1 and a cationic textile softener.

**Revendications pour les Etats contractants: DE GB FR CH LI IT NL BE SE**

1. Distyrylbiphényles de formule

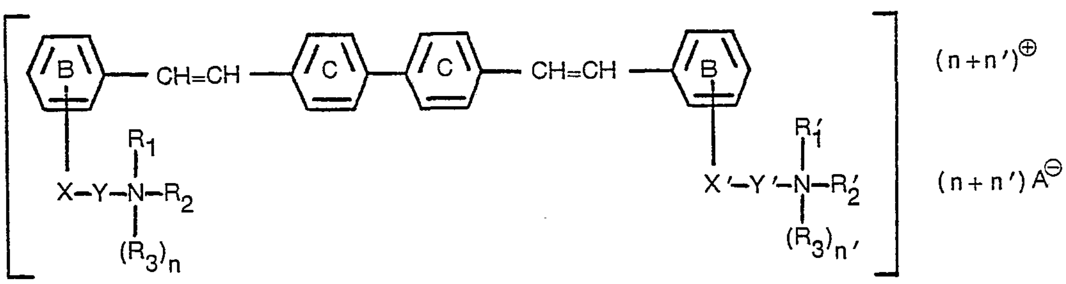

dans laquelle X et X', indépendamment l'un de l'autre, sont chacun une liaison directe, l'oxygène, le soufre, un radical —O—(alkylène en $C_{1-3}$)—CON($R_4$)—, —CON($R_4$)—, —O—(alkylène en $C_{1-3}$)—COO—, —OCO— ou —COO—, où, quand n + n' = 0, X et X' ne peuvent être —CON($R_4$)— ou —O—(alkylène en $C_{1-3}$)—CONR($R_4$)— et, quand n + n' = 2 et X et X' sont —CON($R_4$)— ou —COO—, $A^{\ominus}$ ne peut être un anion phosphite ou phosphonate; Y et Y', indépendamment l'un de l'autre, sont chacun un enchaînement alkylène en $C_{1-20}$ ou un enchaînement alkylène en $C_{1-20}$ substitué par un groupe hydroxyle; $R_1$ et $R_1'$, indépendamment l'un de l'autre, sont chacun un radical alkyle en $C_{1-8}$, ou alcényle en $C_{3-4}$, non-substitué ou substitué par un groupe cyano, hydroxy, alcoxy, phényle (alcoxy en $C_{1-4}$)carbonyle ou chlore, ou bien respectivement $R_1$ et $R_1'$, avec respectivement $R_2$ $R_2'$, et l'atome N auquel ils sont lés, forment un noyau hétérocyclique pentagonal à heptagonal; $R_2$ et $R_2'$ indépendamment l'un de l'autre, sont chacun un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou chlore, ou bien respectivement $R_2$ et $R_2'$, avec respectivement $R_1$ et $R_1'$, et l'atome N auquel ils sont liés, forment un noyau hétérocyclique pentagonal à heptagonal; $R_3$ est l'hydrogène, un radical alkyle en $C_{1-4}$ ou alcényle en $C_{3-4}$, non-substitué ou substitué par un groupe cyano, hydroxy, alcoxy, phényle ou (alcoxy en $C_{1-4}$)carbonyle; $R_1$, $R_2$ et $R_3$, avec l'atome N auquel ils sont liés, ou $R_1'$, $R_1'$ et $R_3$, avec l'atome N auquel ils sont liés, pouvant être aussi le noyau pyridine ou picoline; $R_4$ est l'hydrogène ou un groupe alkyle en $C_{1-6}$ substitué par un groupe cyano, carbamoyle, carbalcoxy, hydroxy, halogène ou alcoxy en $C_{1-4}$; $A^{\ominus}$ est un anion incolore et n et n', indépendamment l'un de l'autre, sont le nombre 0 ou 1; les noyaux benzéniques B et C pouvant aussi être substitués par des substituants non-chromophores; et dans laquelle les groupements

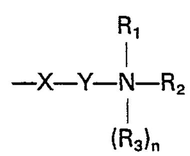

et

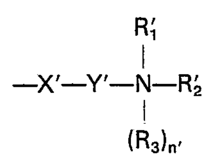

dans le cas où les noyaux B et C ne portent pas de substituants non-chromophores, peuvent aussi être tous les deux chacun

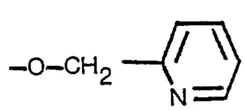

ou chacun

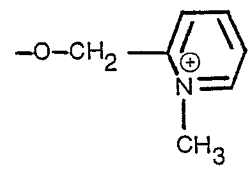

sur les positions ortho par rapport aus deux groupes —CH=CH—.

2. Distyrylbiphényles selon la revendication 1, de formule

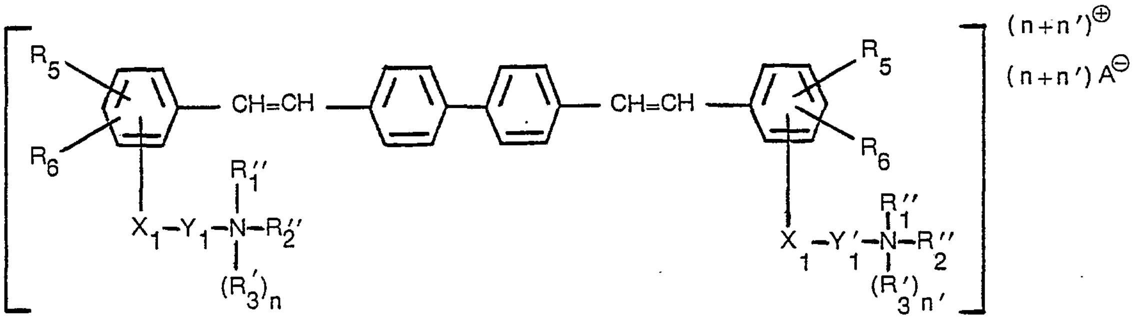

dans laquelle $X_1$ est une liaison directe, l'oxygène, le soufre, un radical —O—(alkylène en $C_{1-3}$)—CONH—, —CONH—, —O—(alkylène en $C_{1-3}$)—COO—, —OCO— ou —COO—, où, quand n + n' = 0, $X_1$ ne peut être —CONH— ou —O—(alkylène en $C_{1-3}$)—CONH— et, quand n + n' = 2 et $X_1$ = —CONH— ou —COO—, $A^\ominus$ ne peut être un anion phosphite ou phosphonate; $Y_1$ et $Y_1'$, indépendamment l'un de l'autre, sont chacun un enchaînement alkylène en $C_{1-4}$ ou hydroxypropylène; $R_1''$ et $R_2''$, indépendamment l'un de l'autre, sont chacun un radical alkyle en $C_{1-4}$, ou bien $R_1''$ et $R_2''$, avec l'atome N auquel ils sont liés, forment un noyau pyrrolidine, pipéridine, hexaméthylénimine ou morpholine; $R_3'$ est l'hydrogène, un radical alkyle en $C_{1-4}$, alcényle en $C_{3-4}$, (alcoxy en $C_{1-3}$)carbonylméthyle, benzyle, hydroxyalkyle en $C_{2-4}$ ou cyano(alkyle en $C_{2-4}$); $R_1''$, $R_2''$ et $R_3'$, avec l'atome N auquels ils sont liés, peuvent être aussi le noyau pyridine ou picoline; $R_5$ est l'hydrogène, le chlore, un radical alkyle en $C_{1-4}$, alcényle en $C_{3-4}$, alcoxy en $C_{1-3}$, ou bien $R_5$, avec $R_6$, forme un enchaînement triméthylène ou tétraméthylène; $R_6$ est l'hydrogène, le chlore, un radical alkyle en $C_{1-4}$, alcoxy en $C_{1-3}$, ou bien $R_6$, avec $R_5$, forme un enchaînement triméthylène ou tétraméthylène; n et n', indépendamment l'un de l'autre, sont le nombre 0 ou 1, et $A^\ominus$ est un anion incolore.

3. Distyrylbiphényles selon la revendication 1, de formule

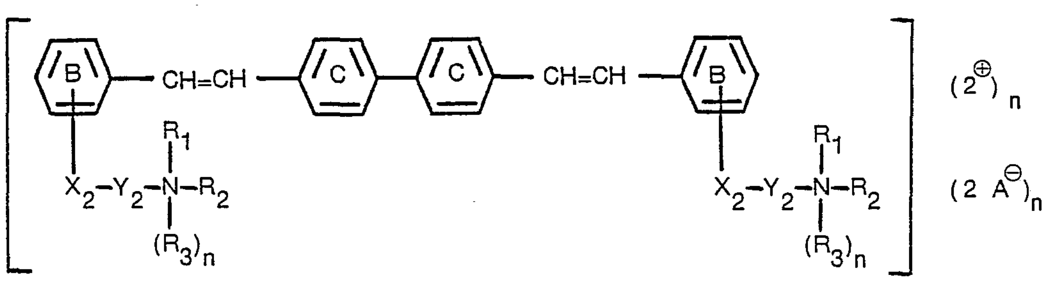

dans laquelle $X_2$ est l'oxygène, le soufre —CON($R_4$)—, —OCO— ou —COO— où, quand n = 0, $X_2$ ne peut être —CON($R_4$)— et quand n = 1 et $X_2$ est —CON($R_4$)— ou —COO—, $A^\ominus$ ne peut être en anion phosphite ou phosphonate; $Y_2$ est un enchaînement alkylène en $C_{1-20}$ ou alkylène en $C_{1-16}$ substitué par un groupe hydroxyle; $R_1$ est un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou chlore, ou bien $R_1$, avec $R_2$ et l'atome N auquel ils sont liés, forme un noyau hétérocyclique pentagonal à heptagonal; $R_2$ est un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou chlore, ou bien $R_2$, avec $R_1$ et l'atome N auquel ils sont liés, forme un noyau hétérocyclique pentagonal à heptagonal; $R_3$ est l'hydrogène, un radical alkyle en $C_{1-4}$ non-substitué ou substitué par un groupe cyano, hydroxy, alcoxy, phényle ou (alcoxy en $C_{1-4}$)carbonyle; $R_1$, $R_2$ et $R_3$, avec l'atome N auquel ils sont liés, peuvent être aussi le noyau pyridine ou picoline; $R_4$ est l'hydrogène ou un radical alkyle en $C_{1-6}$ non-substitué ou substitué par un groupe cyano, carbamoyle, carbalcoxy, hydroxy, halogène ou alcoxy en $C_{1-4}$; n est le nombre 0 ou 1 et $A^\ominus$ est un anion incolore, les noyaux benzéniques B et C pouvant aussi être substitués par des substituants non-chromophores.

4. Distyrylbiphényles selon la revendiction 2, de formule

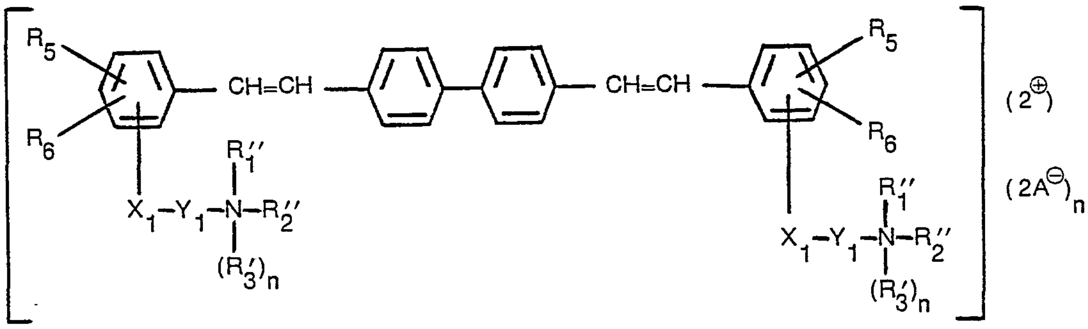

dans laquelle $X_1$ est une liaison directe, l'oxygène, le soufre, un radical —O—(alkylène en $C_{1-3}$)—CONH—, —CONH—, —O—(alkylène en $C_{1-3}$)—COO—, —OCO— ou —COO—, où, quand n = 0, $X_1$ ne peut être —CONH— ou —O—(alkylène en $C_{1-3}$)—CONH— et, quand n = 1 et $X_1$ est —CONH— ou —COO—, $A^{\ominus}$ ne peut être un anion phosphite ou phosphonate; $Y_1$ est un enchaînement en $C_{1-4}$ ou hydroxypropylène; $R_1''$ et $R_2''$, indépendamment alkylène l'un de l'autre, sont chacun un radical alkyle en $C_{1-4}$, ou bien $R_1''$ et $R_2''$, avec l'atome N auquel ils sont liés forment un noyau pyrrolidine, pipéridine, hexaméthylénimine ou morpholine; $R_3'$ est l'hydrogène, un radical alkyle en $C_{1-4}$, alcényle en $C_{3-4}$, (alcoxy en $C_{1-3}$)carbonylméthyle, benzyle, hydroxyalkyle en $C_{2-4}$ ou cyano(alkyle en $C_{2-4}$) ou bien $R_1''$, $R_2''$ et $R_3'$, avec l'atome N auquel ils sont liés, forment aussi le noyau pyridine ou picoline; $R_5$ est l'hydrogène, le chlore, un radical alkyle en $C_{1-4}$, alcényle en $C_{3-4}$, alcoxy en $C_{1-3}$, ou bien $R_5$, avec $R_6$, forme un enchaînement triméthylène ou tétraméthylène; $R_6$ est l'hydrogène, le chlore, un radical alkyle en $C_{1-4}$, alcoxy en $C_{1-3}$, ou bien $R_6$, avec $R_5$, forme un enchaînement triméthylène ou tétraméthylène; n est le nombre 0 ou 1 et $A^{\ominus}$ est un anion incolore.

5. Distyrylbiphényles selon la revendication 4, de formule

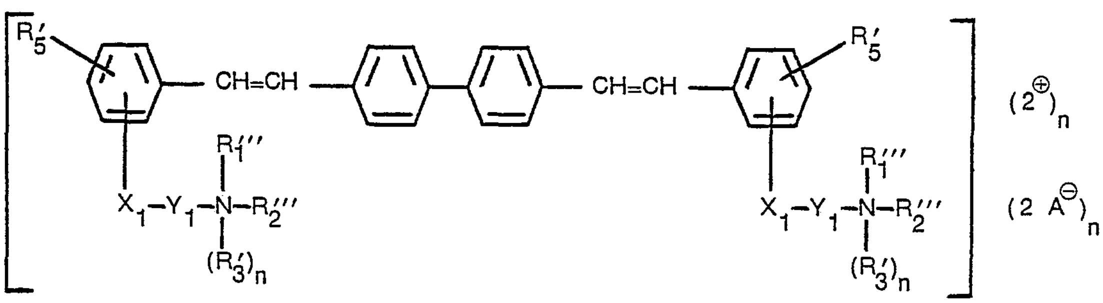

dans laquelle $X_1$ est une liaison directe, l'oxygène, le soufre, un radical —O—(alkylène en $C_{1-3}$)—CONH—, —CONH—, —O—(alkylène en $C_{1-3}$)—COO—, —OCO— ou —COO—, où, quand n = 0, $X_1$ ne peut être —CONH— ou —O—(alkylène en $C_{1-3}$)—CONH—, et, quand n = 1 et $X_1$ est —CONH— ou —COO—, $A^{\ominus}$ ne peut être un anion phosphite ou phosphonate; $Y_1$ est un enchaînement alkylène en $C_{1-4}$ ou hydroxypropylène, $R_1'''$ et $R_2'''$, indépendament l'un de l'autre, sont chacun un radical alkyl en $C_{1-4}$, ou bien $R_1'''$ et $R_2'''$, avec l'atome N auquel ils sont liés, forment un noyau pyrrolidine, pipéridine ou morpholine; $R_3'$ est l'hydrogène, un radical alkyle en $C_{1-4}$, alcényle en $C_{3-4}$, (alcoxy en $C_{1-3}$)carbonylméthyle, benzyle, hydroxyalkyle en $C_{2-4}$ ou cyano(alkyle en $C_{2-4}$); $R_1'''$, $R_2'''$ et $R_3'$, avec l'atome N auquel ils sont lés, pouvant être aussi le noyau pyridine; $R_5'$ est l'hydrogène, le chlore, un radical alkyle en $C_{1-4}$ ou alcoxy en $C_{1-3}$, n est le nombre 0 ou 1 et $A^{\ominus}$ est un anion incolore.

6, Distyrylbiphényles selon la revendication 4, de formule

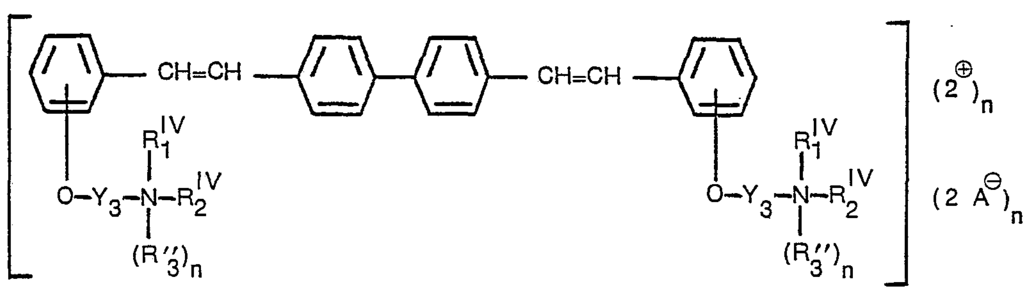

dans laquelle $Y_3$ est un enchaînement alkylène en $C_{2-4}$, $R_1^{IV}$ est un radical alkyle en $C_{1-3}$ ou bien $R_1^{IV}$, avec $R_2^{IV}$ et l'atome N auquel ils sont liés, forme un noyau pyrrolidine, pipéridiné hexaméthylénimine ou morpholine; $R_2^{IV}$ est un radical alkyle en $C_{1-3}$ ou bien $R_2^{IV}$, avec $R_1^{IV}$ et l'atome N auquel ils sont liés, forme un noyau pyrrolidine, pipéridine, hexaméthylénimine ou morpholine; $R_3''$ est l'hydrogène ou un radical alkyle en $C_{1-3}$, n est le nombre 0 ou 1 et $A^{\ominus}$ est un anion incolore.

7. Distyrylbiphényles selon la revendication 6, de formule

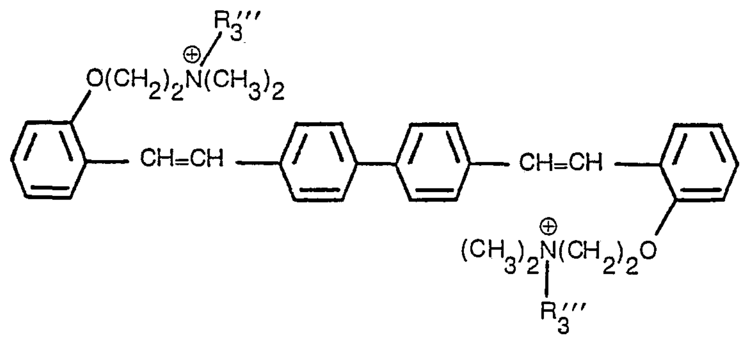

2 $A'^{\ominus}$

dans laquelle $A'^{\ominus}$ est $CH_3OSO_3^{\ominus}$ ou $C_2H_5OSO_3^{\ominus}$ et $R_3'''$ est le radical méthyle ou éthyle.

8. Distyrylbiphényles selon la revendication 6, de formule

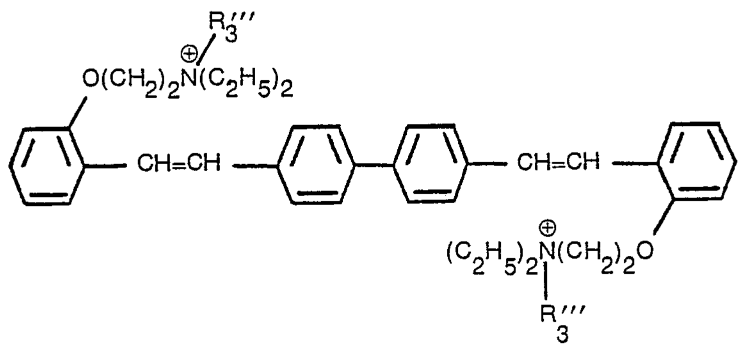

2 $A'^{\ominus}$

dans laquelle $A^{\ominus}$ est $CH_3OSO_3^{\ominus}$ ou $C_2H_5OSO_3^{\ominus}$, et $R_3'''$ est le radical méthyle ou éthyle.

9. Distyrylbiphényles selon la revendication 6, de formule

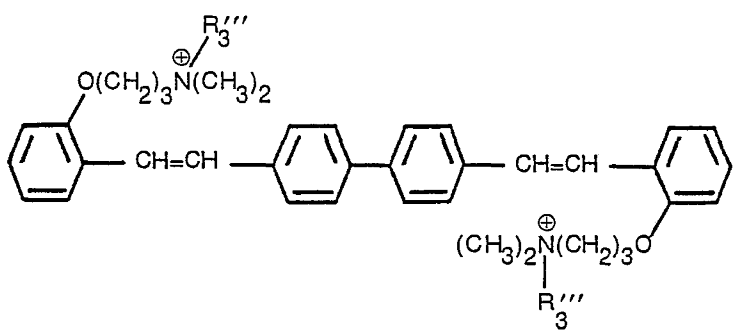

2 $A'^{\ominus}$

dans laquelle $A'^{\ominus}$ est $CH_3OSO_3^{\ominus}$ et $C_2H_5OSO_3^{\ominus}$ et $R_3'''$ est le radical méthyle ou éthyle.

10. Distyrylbiphényles selon la revendication 1, de formule

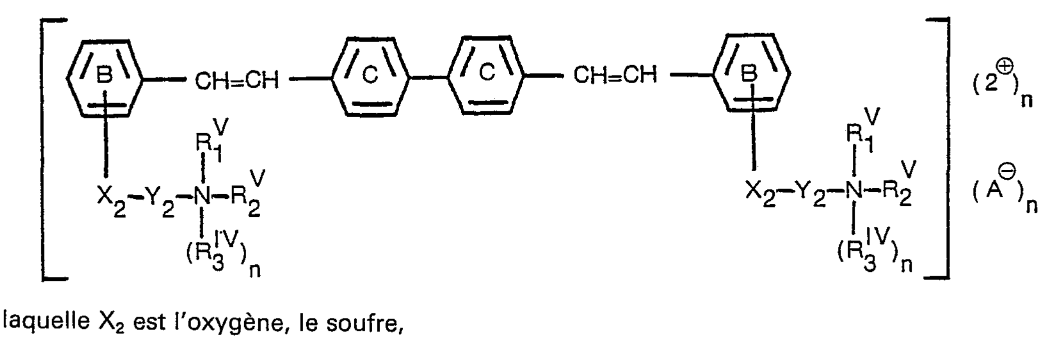

dans laquelle $X_2$ est l'oxygène, le soufre,

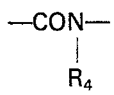

ou —COO—, où, quand $A^{\ominus}$ est un anion phosphite ou phosphonate, $X_2$ n'est pas —$CON(R_4)$— ou —COO—; $Y_2$ est un enchaînement alkylène en $C_{2-20}$, $R_1^V$ est un radical alkyle en $C_{1-8}$, ou alcényle en $C_{3-4}$, non-substitué ou substitue par un groupe cyano, hydroxy, alcoxy, phényle, (alcoxy en $C_{1-4}$)carbonyle ou chlore, ou bien $R_1^V$, avec $R_2^V$ et l'atome N auquel ils sont liés, forme un noyau hétérocyclique pentagonal à hexagonal; $R_2^V$ est un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou chlore, ou bien $R_2^V$, avec $R_1^V$ et l'atome N auquel ils sont liés, forme un noyau hétérocyclique pentagonal à heptagonal; $R_3^{IV}$ est l'hydrogène ou un radical alkyle en $C_{1-4}$ non-substitué par un groupe cyano, hydroxy, alcoxy, phényle ou (alcoxy en $C_{1-4}$)carbonyle; $R_4$ est l'hydrogène ou un radical alkyle en $C_{1-6}$ nonsubstitué ou substitué par un groupe cyano, carbamoyle, carbalcoxy, hydroxy, halogène ou alcoxyen $C_{1-4}$; $A^{\ominus}$ est l'équivalent d'un anion incolore et n est le nombre 0 ou 1, les noyaux benzéniques B et C pouvant aussi être substitués par des substituants non-chromophores.

11. Distyrylbiphényles selon la revendication 10, de formule

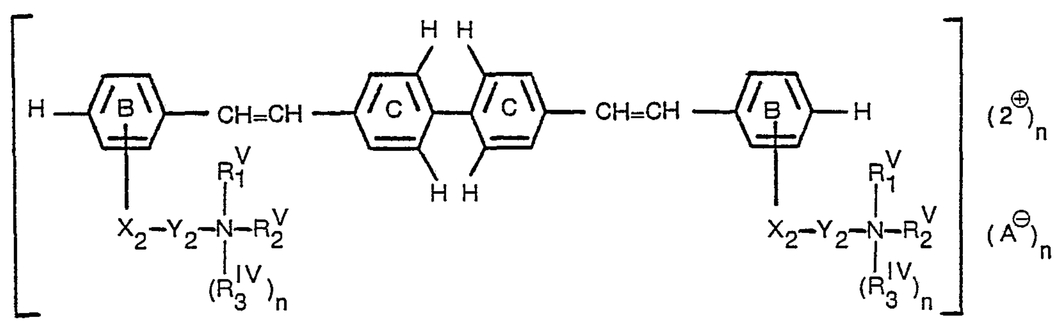

dans laquelles $X_2$, $Y_2$, $R_1^V$, $R_2^V$, $R_3^{IV}$, $A^{\ominus}$ et n ont les significations données dans la revendication 10, et les noyauz benzéniques B et C peuvent aussi être substitués par des substituants non-chromophores.

12. Distyrylbiphényles selon la revendication 11, de formule

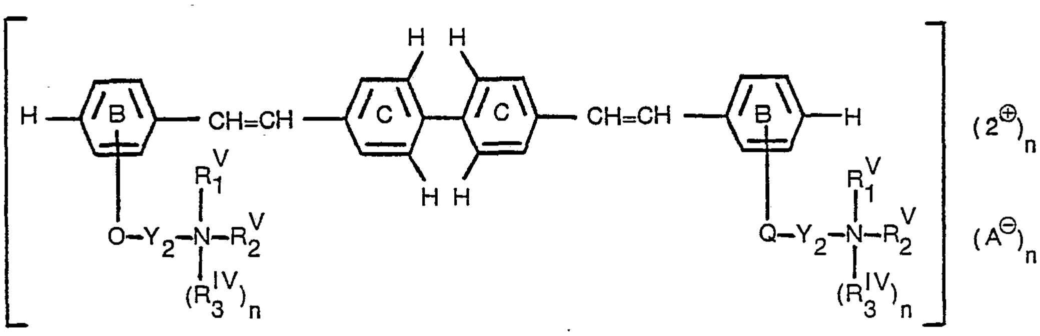

dans laquelle $Y_2$ est un enchaînement alkylène en $C_{2-20}$, $R_1^V$ est un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou chlore, ou bien $R_1^V$, avec $R_2^V$ et l'atome N auquel ils sont liés, forme un noyau hétérocyclique pentagonal à heptagonal; $R_2^V$ est un radical alkyle en $C_{1-8}$, ou alcényle en $C_{3-4}$, non-substitué ou substitué par un groupe cyano, hydroxy, alcoxy, phényle, (alcoxy en $C_{1-14}$)carbonyle ou chlore, ou bien $R_2^V$, avec $R_1^V$ et l'atome N auquel ils sont liés, forme un noyau hétérocyclique pentagonal ou heptagonal; $R_3^{IV}$ est l'hydrogène ou un radical alkyle en $C_{1-4}$ non-substitué ou substitué par un groupe cyano, hydroxy, alcoxy, phényle ou (alcoxy en $C_{1-4}$)carbonyle, $R_4$ est l'hydrogène ou un radical alkyle en $C_{1-6}$ non-substitué ou substitué par un groupe cyano, carbamoyle, carbalcoxy, hydroxy, halogène ou alcoxy en $C_{1-4}$; $A^{\ominus}$ est l'équivalent d'un anion incolore et n est le nombre 0 ou 1, les noyaux benzéniques B et C pouvant aussi être substitués par des substituants non-chromophores.

13. Procédé pour la préparation de distyrylbiphényles de formule

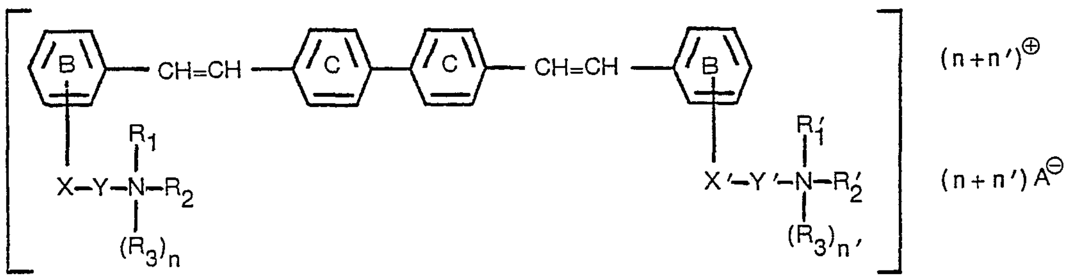

dans laquelle X et X', indépendamment l'un de l'autre, sont chacun une liaison directe, l'oxygène, le soufre, un radical —O—(alkylène en $C_{1-3}$)—CON($R_4$)—, —CON($R_4$)—, —O—(alkylène en $C_{1-3}$)—COO—, —OCO— ou —COO—, où, quand n + n' = 0, X et X' ne peuvent être —CON($R_4$)— ou —O—(alkylène en $C_{1-3}$)—CONR($R_4$)— et, quand n + n' = 2 et X et X' sont —CON($R_4$)— ou —COO—, $A^{\ominus}$ ne peut être un anion phosphite ou phosphonate; Y et Y', indépendamment l'un de l'autre, sont chacun un enchaînement alkylène en $C_{1-20}$ ou un alkylène en $C_{1-20}$ substitué par un groupe hydroxyle; $R_1$ et $R_1'$, indépendamment l'un de l'autre, sont chacun un radical alkyle en $C_{1-8}$, ou alcényle en $C_{3-4}$, non-substitué ou substitué par un groupe cyano, hydroxy, alcoxy, phényle (alcoxy en $C_{1-4}$)carbonyle ou chlore, ou bien $R_1$ et $R_1'$, respectivement $R_2$ et $R_2'$, et l'atome N auquel ils sont liés, forment un noyau hétérocyclique pentagonal à heptagonal; $R_2$ et $R_2'$ indépendamment l'un de l'autre, sont chacun un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou chlore, ou bien $R_2$ et $R_2'$, respectivement avec $R_1$ et $R_1'$, et avec l'atome N auquel ils sont liés, forment un noyau hétérocyclique pentagonal à heptagonal, $R_3$ est l'hydrogène, un radical alkyle en $C_{1-4}$ ou alcényle en $C_{3-4}$, non-substitué ou substitué par un groupe cyano, hydroxy, alcoxy, phényle ou (alcoxy en $C_{1-4}$)carbonyle; $R_1$, $R_2$ et $R_3$, avec l'atome N auquel ils sont liés, ou $R_1'$, $R_1'$ et $R_3'$, avec l'atome N auquel ils sont liés, pouvant aussi former le noyau pyridine ou picoline; $R_4$ est l'hydrogène ou un radical alkyle en $C_{1-6}$ non-substitué ou substitué par un groupe cyano, carbamoyle, carbalcoxy, hydroxy, halogène ou alcoxy en $C_{1-4}$; $A^{\ominus}$ est un anion incolore et n et n', indépendamment l'un de l'autre, sont chacun le nombre 0 ou 1; les noyaux benzéniques B et C pouvant aussi être substitués par des substituants non-chromophores; et dans laquelle les groupements

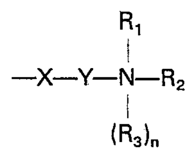 ainsi que 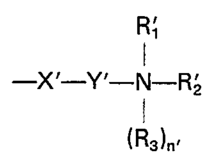

dans le cas où les noyaux B et C ne portent pas de substituants non-chromophores, peuvent aussi être tous les deux chacun

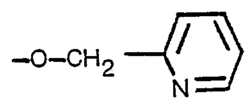

ou tous les deux chacun

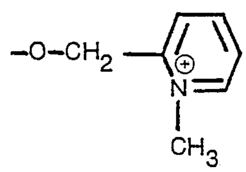

sur les positions ortho par rapport aus deux groupes —CH=CH—, caractérisé en ce qu'on quaternise ou protone, avec un équivalent molaire ou avec deux équivalents molaires, respectivement d'un agent d'alkylation ou d'un acide de formule $R_3$—A dans laquelle $R_3$ et $A^{\ominus}$ ont les significations données ci-dessus, un équivalent molaire d'un distyrylbiphényle de formule

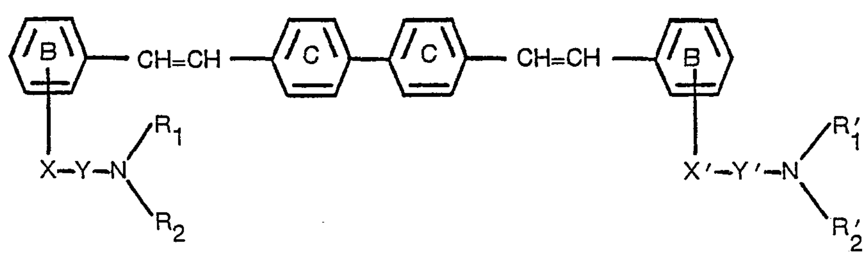

dans laquelle les noyaux benzéniques B et C, et X, X', Y, Y', $R_1$, $R_1'$, $R_2$ et $R_2'$ ont les significations données ci-dessus.

14. Procédé pour la préparation de distrylbiphényles de formule

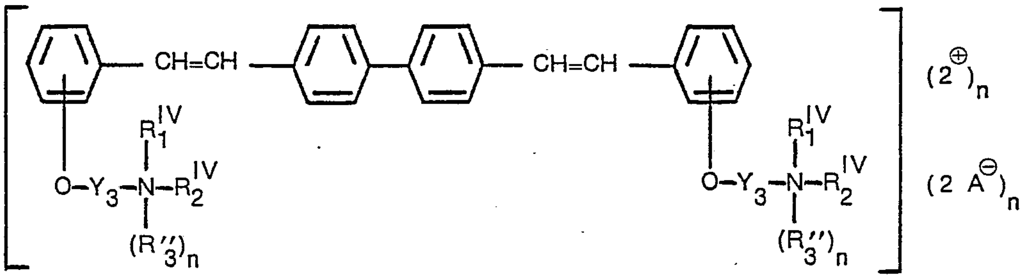

dans laquelle $Y_3$ est un enchaînement alkylène en $C_{2-4}$, $R_1^{IV}$ est un radical alkyle en $C_{1-3}$ ou bien $R_1^{IV}$, avec $R_2^{IV}$ et l'atome N auquel ils sont liés, forme un noyau pyrrolidine, pipéridiné hexaméthylénimine ou morpholine; $R_2^{IV}$ est un radical alkyle en $C_{1-3}$ ou bien $R_2^{IV}$, avec $R_1^{IV}$ et l'atome N auquel ils sont liés, forme un noyau pyrrolidine, pipéridine, hexaméthylénimine ou morpholine; $R_3''$ est l'hydrogène ou un radical alkyle en $C_{1-3}$, n est le nombre 0 ou 1 et $A^{\ominus}$ est un anion incolore, caractérisé en ce qu'on fait réagir selon le rapport moléculaire 1:2 un composé de formule

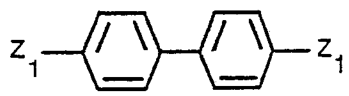

sur des composés de formule

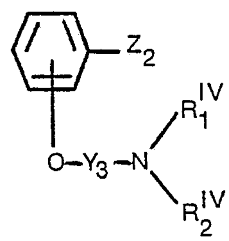

en présence d'une base forte, formules dans lesquelles $Y_3$, $R_1^{IV}$ et $R_2^{IV}$ ont les significations données ci-dessus, et l'un des symboles $Z_1$ et $Z_2$ est un groupe —OCH et l'autre un groupement de formule

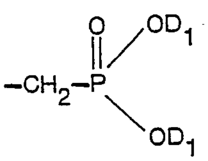 , 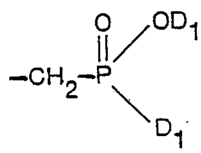 ,

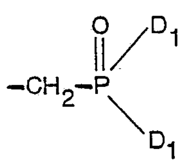 ou 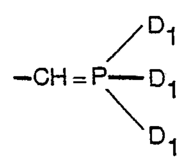

où $D_1$ est un radical alkyle, aryle, cycloalkyle ou aralkyle, non-substitue ou substitué, et que selon le cas désiré, quaternise ou protone, respectivement avec deux équivalents molaires d'un agent d'alkylation ou d'un acide, de formule $R_3''$—A, dans laquelle $R_3''$et A ont les significations données ci-dessus, le composé obtenu, de formule

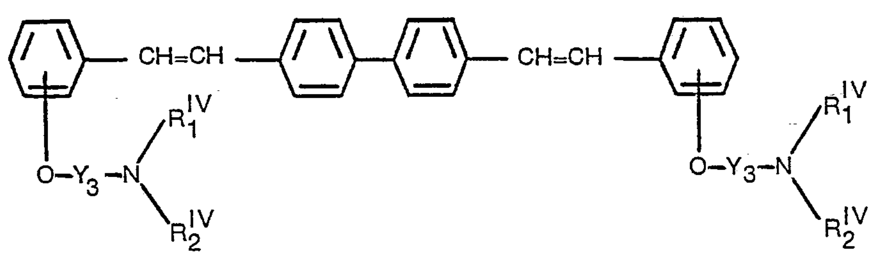

dans laquelle $Y_3$, $R_1^{IV}$ et $R_2^{IV}$ ont la signification donnée ci-dessus.

15. Procédé pour le blanchiment optique de matériaux organiques, caractérisé en ce qu'on incorpore à ces matériaux, ou applique sur leur surface, des distyrylbiphényles ayant la formule définie dans la revendication 1.

16. Procédé selon la revendication 15, pour le blanchiment optique des matériaux organiques polyacrylonitrile ou cellulose.

17. Détergent contenant un ou plusieurs distyrylbiphényles ayant la formule définie dans la revendication 1.

18. Détergent selon la revendication 17, qui contient 10 à 70% en poids d'un tensioactif non-ionique et 1 à 30% d'un adoucissant cationique pour textile.

19. Détergent selon la revendication 18, qui contient 10 à 70% en poids d'un tensioactif non-ionique et 1 à 30% en poids d'un dérivé quaternaire de l'ammoniac et/ou de l'imidazoline possédant chacun deux résidus aliphatiques à longue chaîne en tant qu'adoucissant cationique pour textile et, pour conférer la forme liquide, un solvant.

20. Détérgent selon la revendication 19, qui contient en tant qu'adoucissant cationique pour textile de 1-méthyl-1-oléylamidoéthyl-2-oléyl-imidazolinium·$X^{\ominus}$, du 1-méthyl-1-suif-amidoéthyl-2-suif-imidazolinium·$X^{\ominus}$, du di-suif-diméthylammonium·$X^{\ominus}$ ou un composé de formule

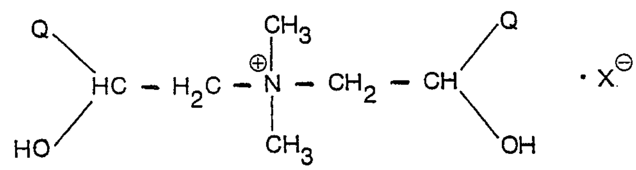

dans laquelle Q est un radical alkyle en $C_{14-16}$ et $X^{\ominus}$ est un anion chlorure, bromure, méthylsulfate, éthylsulfate, méthanesulfonate, éthanesulfonate ou toluènesulfonate.

21. Agent de traitement de textiles contenant un ou plusieurs distyrylbiphényles ayant la formule définie dans la revendication 1.

22. Agent de post-traitement de linge un ou plusieurs distyrylbiphényles ayant la formule définie dans la revendication 1, et un adoucissant cationique pour textiles.

**Revendications pour l'Etat contractant: AT**

1. Azurant contenant un composé de formule

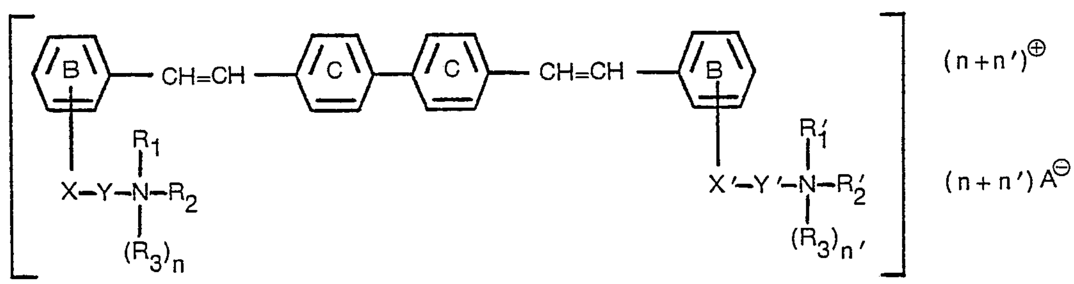

dans laquelle X et X', indépendamment l'un de l'autre, sont chacun une liaison directe, l'oxygène, le soufre, un radical —O—(alkylène en $C_1$—$C_3$)—CON($R_4$)—, —CON($R_4$)—, —O—(alkylène en $C_1$—$C_3$)—COO—, —OCO— ou —COO—, où, quand n + n' = 0, X et X' ne peuvent être —CON($R_4$)— ou —O—(alkylène en $C_{1-3}$)—CONR($R_4$)— et, quand n + n' = 2 et X et X' sont —CON($R_4$)— ou —COO—, $A^{\ominus}$ ne peut être un anion phosphite ou phosphonate; Y et Y', indépendamment l'un de l'autre, sont chacun un enchaînement alkylène en $C_{1-20}$ ou alkylène en $C_{1-20}$ substitué par un groupe hydroxyle; $R_1$ et $R_1'$, indépendamment l'un de l'autre, sont chacun un radical alkyle en $C_{1-8}$, ou alcényle en $C_{3-4}$, non-substitué ou substitué par un groupe cyano, hydroxy, alcoxy, phényle (alcoxy en $C_{1-4}$)carbonyle ou chlore, ou bien $R_1$ et $R_1'$, respectivement avec $R_2$ et $R_2'$, et l'atome N auquel ils sont lés, formentun noyau hétérocyclique pentagonal à heptagonal; $R_2$ et $R_2'$ indépendamment l'un de l'autre, sont chacun un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou chlore, ou bien $R_2$ et $R_2'$, respectivement avec $R_1$ et $R_1'$, et l'atome N auquel ils sont liés, forment un noyau hétérocyclique pentagonal à heptagonal; $R_3$ est l'hydrogène, un radical alkyle en $C_{1-4}$ ou alcényle en $C_{3-4}$, non-substitué ou substitué par un groupe cyano, hydroxy, alcoxy, phényle ou (alcoxy en $C_{1-4}$)carbonyle; $R_1$, $R_2$ et $R_3$, avec l'atome N auquel ils sont liés, ou $R_1'$, $R_1'$ et $R_3$, avec l'atome N auquel ils sont liés, peuvent être aussi le noyau pyridine ou picoline, $R_4$ est l'hydrogène ou un radical alkyle en $C_{1-6}$ non substitué ou substitué par un groupe cyano, carbamoyle, carbalcoxy, hydroxy, halogène ou alcoxy en $C_{1-4}$; $A^{\ominus}$ est un anion incolore et n et N', indépendamment l'un de l'autre, sont chacun le nombre 0 ou 1; les noyaux benzéniques B et C pouvant aussi être substitués par des substituants non-chromophores; et dans laquelle les groupements

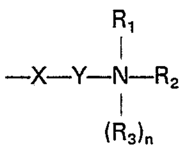

et

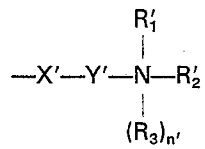

dans le cas où les noyaux B et C ne portent pas de substituants non-chromophores, peuvent aussi être tous les deux chacun

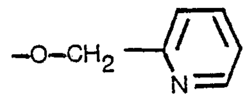

ou tous les deux chacun

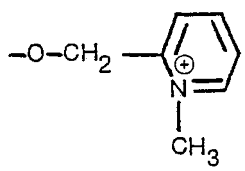

sur les positions ortho par rapport aux deux groupes —C=C—, avec des additifs convenant aux azurants.

2. Azurant selon la revendication 1, contenant un composé de formule

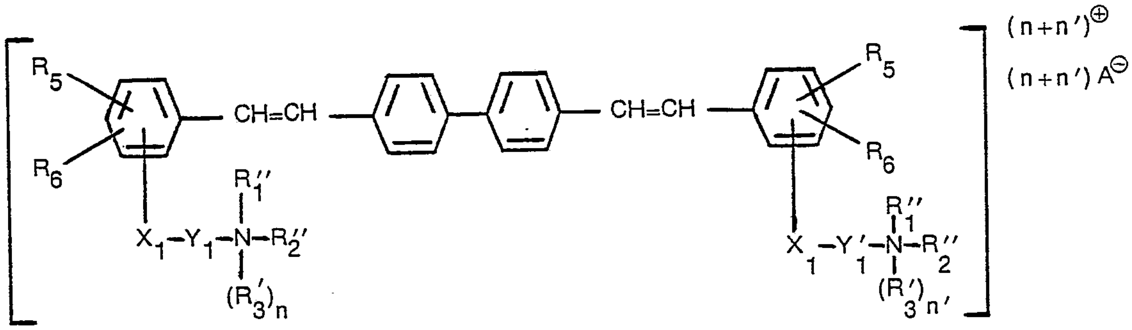

dans laquelle $X_1$ est une liaison directe, l'oxygène, le soufre, un radical —O—(alkylène en $C_{1-3}$)—CONH—, —CONH—, —O—(alkylène en $C_{1-3}$)—COO—, —OCO— ou —COO—, où, quand n + n' = 0, $X_1$ ne peut être —CONH— ou —O—(alkylène en $C_{1-3}$)—CONH— et, quand n + n' = 2 et $X_1$ = —CONH— ou —COO—, $A^\ominus$ ne peut être un anion phosphite ou phosphonate; $Y_1$ et $Y_1'$, indépendamment l'un de l'autre, sont chacun un enchaînement alkylène en $C_{1-4}$ ou hydroxypropylène; $R_1''$ et $R_2''$, indépendamment l'un de l'autre sont chacun un radical alkyle en $C_{1-4}$, ou bien $R_1''$ et $R_2''$, avec l'atome N auquel ils sont liés, forment un noyau pyrrolidine, pipéridine, hexaméthylénimine ou morpholine; $R_3'$ est l'hydrogène, un radical alkyle en $C_{1-4}$, alcényle en $C_{3-4}$, (alcoxy en $C_{1-3}$)carbonylméthyle, benzyle, hydroxyalkyle en $C_{2-4}$ ou cyano(alkyle en $C_{2-4}$); $R_1''$, $R_2''$ et $R_3'$, avec l'atome N auquels ils sont liés, peuvent former aussi le noyau pyridine ou picoline; $R_5$ est l'hydrogène, le chlore, un radical alkyle en $C_{1-4}$, alcényle en $C_{3-4}$, alcoxy en $C_{1-3}$, ou bien $R_5$, avec $R_6$, forme un enchaînement triméthylène ou tétraméthylène; $R_6$ est l'hydrogène, le chlore, un radical alkyle en $C_{1-4}$, alcoxy en $C_{1-3}$, ou bien $R_6$, avec $R_5$, forme un enchaînement triméthylène ou tétraméthylène; n et n', indépendamment l'un de l'autre, sont chacun le nombre 0 ou 1, et $A^\ominus$ est un anion incolore.

3. Azurant selon la revendication 1, contenant un composé de formule

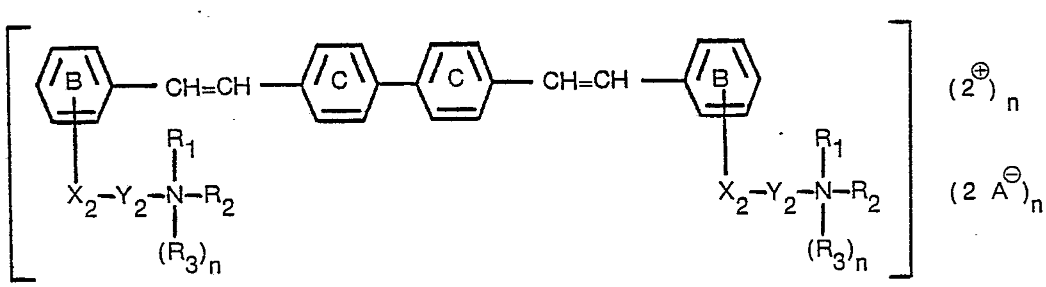

dans laquelle $X_2$ est l'oxygène, le soufre —CON($R_4$)—, —OCO— ou —COO—, où, quand n = 0, $X_2$ ne peut être —CON($R_4$)— et quand n = 1 et $X_2$ est —CON($R_4$)— ou —COO—, $A^\ominus$ ne peut être en anion phosphite ou phosphonate; $Y_2$ est un enchaînement alkylène en $C_{1-20}$ ou un echaînement en $C_{1-20}$ substitué par un

groupe hydroxyle; $R_1$ est un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou chlore, ou bien $R_1$, avec $R_2$ et l'atome N auquels ils sont liés, forme un noyau hétérocyclique pentagonal à heptagonal; $R_2$ est un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou chlore, ou bien $R_2$, avec $R_1$ et l'atome N auquel ils sont liés, forme un noyau hétérocyclique pentagonal à heptagonal; $R_3$ est l'hydrogène, un radical alkyle en $C_{1-4}$ non-substitué ou substitué par un groupe cyano, hydroxy, alcoxy, phényle ou (alcoxy en $C_{1-4}$)carbonyle; $R_1$, $R_2$ et $R_3$, avec l'atome N auquel ils sont liés, peuvent former aussi le noyau pyridine ou picoline; $R_4$ est l'hydrogène ou un radical alkyle en $C_{1-6}$ non-substitué ou substitué par un groupe cyano, carbamoyle, carbalcoxy, hydroxy, halogène ou alcoxy en $C_{1-4}$; n est le nombre 0 ou 1 et $A^{\ominus}$ est un anion incolore, les noyaux benzéniques B et C pouvant aussi être substitués par des substituants non-chromophores.

4. Azurant selon la revendication 2, contenant un composé de formule

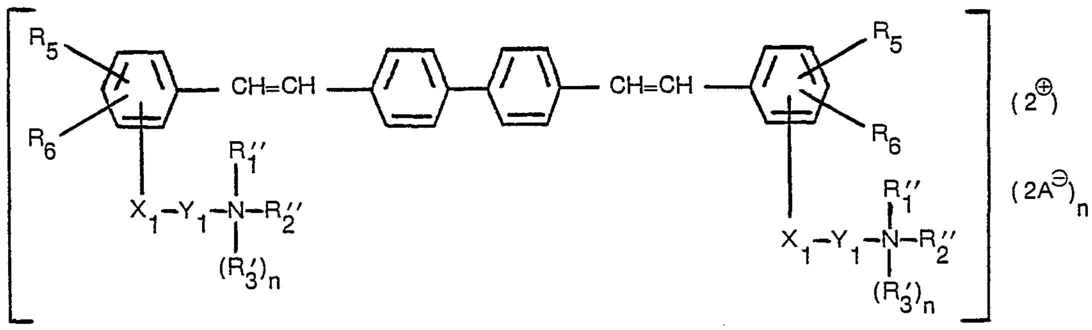

dans laquelle $X_1$ est une liaison directe, l'oxygène, le soufre, un radical —O—(alkylène en $C_{1-3}$)—CONH—, —CONH—, —O—(alkylène en $C_{1-3}$)—COO—, —OCO— ou —COO—, où, quand n = 0, $X_1$ ne peut être —CONH— ou —O—(alkylène en $C_{1-3}$)—CONH— et, quand n = 1 et $X_1$ est —CONH— ou —COO—, $A^{\ominus}$ ne peut être un anion phosphite ou phosphonate; $Y_1$ est un enchaînement alkylène en $C_{1-4}$ ou hydroxypropylène; $R_1''$ et $R_2''$, indépendamment l'un de l'autre, sont chacun un radical alkyle en $C_{1-4}$, ou bien $R_1''$ et $R_2''$, avec l'atome N auquel ils sont liés forment un noyau pyrrolidine, pipéridine, hexaméthylénimine ou morpholine; $R_3'$ est l'hydrogène, un radical alkyle en $C_{1-4}$, alcényle en $C_{3-4}$, (alcoxy en $C_{1-3}$)carbonylméthyle, benzyle, hydroxyalkyle en $C_{2-4}$ ou cyano(alkyle en $C_{2-4}$) ou bien $R_1''$, $R_2''$ et $R_3'$, avec l'atome N auquel ils sont liés, peuvent former aussi le noyau pyridine ou picoline; $R_5$ est l'hydrogène, le chlore, un radical alkyle en $C_{1-4}$, alcényle en $C_{3-4}$, alcoxy en $C_{1-3}$, ou bien $R_5$, avec $R_6$, forme un enchaînement triméthylène ou tétraméthylène; $R_6$ est l'hydrogène, le chlore, un radical alkyle en $C_{1-4}$, alcoxy en $C_{1-3}$, ou bien $R_6$, avec $R_5$, forme un enchaînement triméthylène ou tétraméthylène; n est le nombre 0 ou 1 et $A^{\ominus}$ est un anion incolore.

5. Azurant selon la revendication 4, contenant un composé de formule

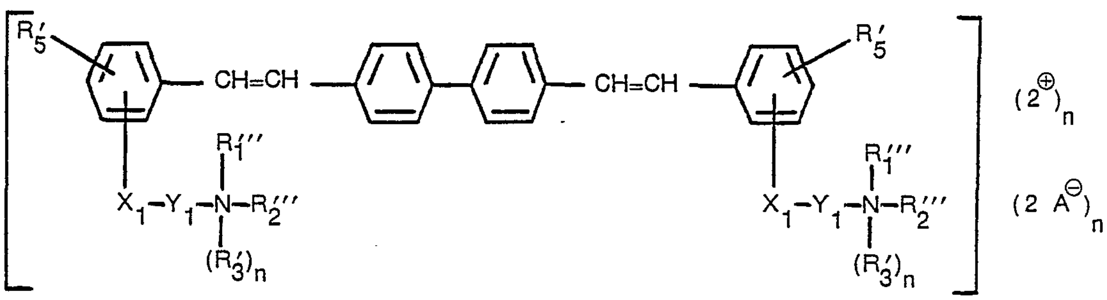

dans laquelle $X_1$ est une liaison directe, l'oxygène, le soufre, un radical —O—(alkylène en $C_{1-3}$)—CONH—, —CONH—, —O—(alkylène en $C_{1-3}$)—COO—, —OCO— ou —COO—, où, quand n = 0, $X_1$ ne peut être —CONH— ou —O—(alkylène en $C_{1-3}$)—CONH—, et, quand n = 1 et $X_1$ est —CONH— ou —COO—, $A^{\ominus}$ ne peut être un anion phosphite ou phosphonate; $Y_1$ est un enchaînement alkylène en $C_{1-4}$ ou hydroxypropylène, $R_1'''$ et $R_2'''$, indépendament l'un de l'autre, sont chacun un radical alkyl en $C_{1-4}$, ou bien $R_1'''$ et $R_2'''$, avec l'atome N auquel ils sont liés, peuvent aussi former un noyau pyrrolidine, pipéridine ou morpholine; $R_3'$ est l'hydrogène, un radical alkyle en $C_{1-4}$, alcényle en $C_{3-4}$, (alcoxy en $C_{1-3}$)carbonylméthyle, benzyle, hydroxyalkyle en $C_{2-4}$ ou cyano(alkyl en $C_{2-4}$); $R_1'''$, $R_2''$ et $R_3'$, avec l'atome N auquel ils sont liés, peuvent former aussi le noyau pyridine; $R_5'$ est l'hydrogène, le chlore, un radical alkyle en $C_{1-4}$ ou alcoxy en $C_{1-3}$, n est le nombre 0 ou 1 et $A^{\ominus}$ est un anion incolore.

6. Azurant selon la revendication 4, contenant un composé de formule

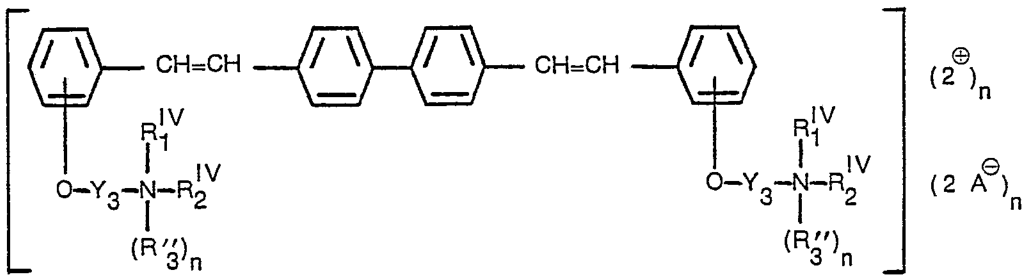

dans laquelle $Y_3$ est un enchaînement alkylène en $C_{2-4}$, $R_1^{IV}$ est un radical alkyle en $C_{1-3}$ ou bien $R_1^{IV}$, avec $R_2^{IV}$ et l'atome N auquel ils sont liés, forme un noyau pyrrolidine, pipéridiné hexaméthylénimine ou morpholine; $R_2^{IV}$ est un radical alkyle en $C_{1-3}$ ou bien $R_2^{IV}$, avec $R_1^{IV}$ et l'atome N auquel ils sont liés, forme un noyau pyrrolidine, pipéridine, hexaméthylénimine ou morpholine; $R_3''$ est l'hydrogène ou un radical alkyle en $C_{1-3}$, n est le nombre 0 ou 1 et $A^{\ominus}$ est un anion incolore.

7. Azurant selon la revendication 6, contenant un composé de formule

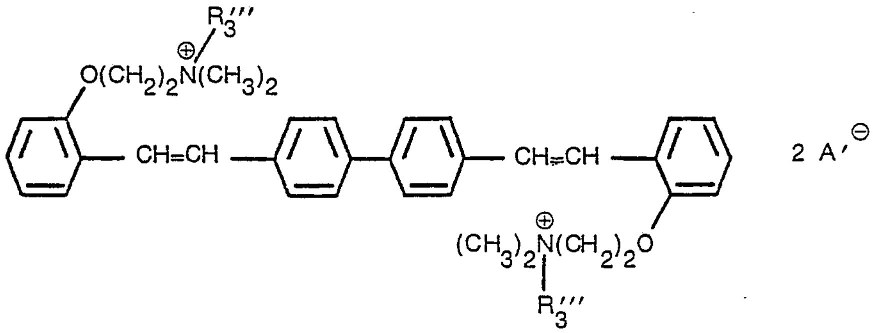

dans laquelle $A'^{\ominus}$ est $CH_3OSO_3^{\ominus}$ ou $C_2H_5OSO_3^{\ominus}$ et $R_3'''$ est le radical méthyle ou éthyle.

8. Azurant selon la revendication 6, contenant un composé de formule

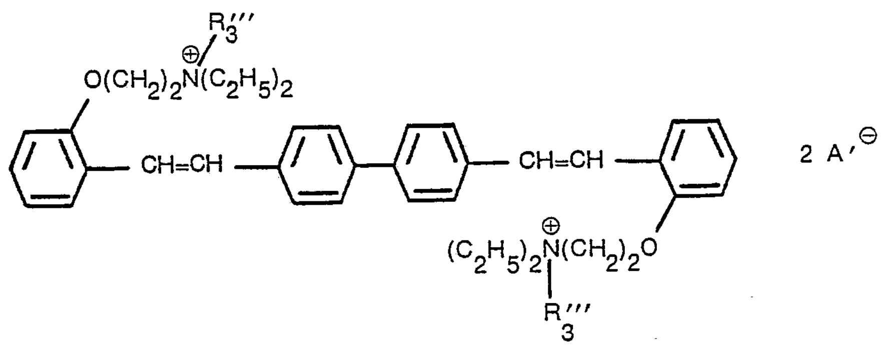

dans laquelle $A'^{\ominus}$ est $CH_3OSO_3^{\ominus}$ ou $C_2H_5OSO_3^{\ominus}$, et $R_3'''$ est le radical méthyle ou éthyle.

9. Azurant selon la revendication 6, contenant un composé de formule

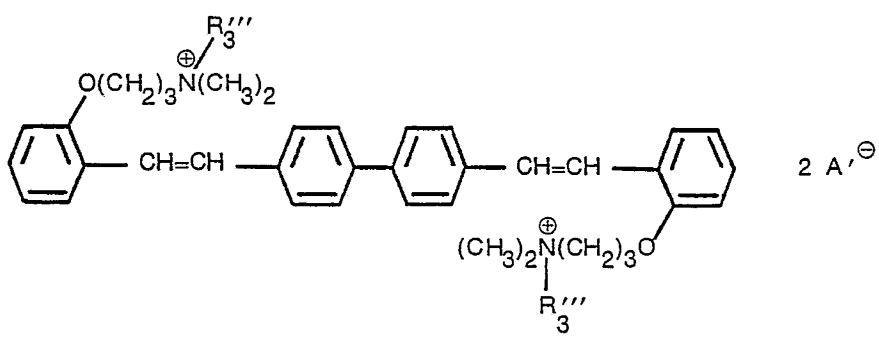

dans laquelle $A'^{\ominus}$ est $CH_3OSO_3^{\ominus}$ et $C_2H_5OSO_3^{\ominus}$ et $R_3'''$ est le radical méthyle ou éthyle.

10. Azurant selon la revendication 1, contenant un composé de formule

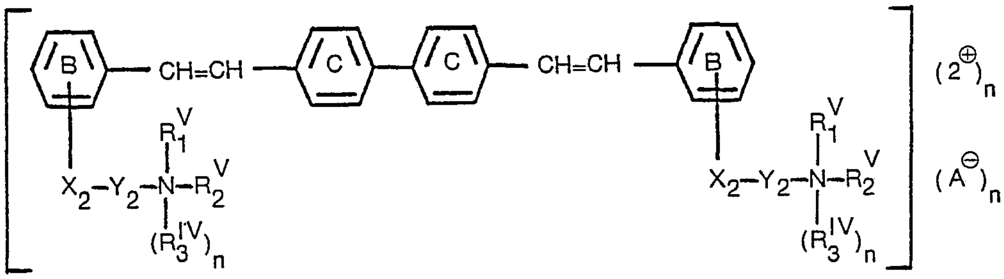

dans laquelle $X_2$ est l'oxygène, le soufre,

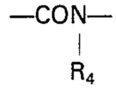

ou —COO—, où, quand $A^{\ominus}$ est un anion phosphite ou phosphonate, $X_2$ n'est pas —CON($R_4$)— ou —COO—; $Y_2$ est un enchaînement alkylène en $C_{2-20}$, $R_1^V$ est un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou chlore, ou bien $R_1^V$, avec $R_2^V$ et l'atome N auquel ils sont liés, forme un noyau hétérocyclique pentagonal à heptagonal; $R_2^V$ est un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou chlore, ou bien $R_2^V$, avec $R_1^V$ et l'atome N auquel ils sont liés, forme un noyau hétérocyclique pentagonal à heptagonal; $R_3^{IV}$ est l'hydrogène ou un radical alkyle en $C_{1-4}$ non-substitué par un groupe cyano, hydroxy, alcoxy, phényle ou (alcoxy en $C_{1-4}$)carbonyle; $R_4$ est l'hydrogène ou un radical alkyle en $C_{1-6}$ nonsubstitué ou substitué par un groupe cyano, carbamoyle, carbalcoxy, hydroxy, halogène ou alcoxy en $C_{1-4}$; $A^{\ominus}$ est l'équivalent d'un anion incolore et n est le nombre 0 ou 1, les noyaux benzéniques B et C pouvant aussi être substitués par des substituants non-chromophores.

11. Azurant selon la revendication 10, contenant un composé de formule

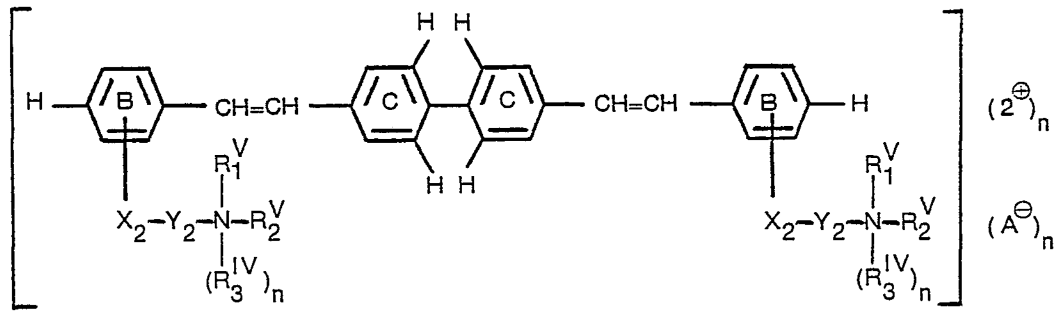

dans laquelles $X_2$, $Y_2$, $R_1^V$, $R_2^V$, $R_3^{IV}$, $A^{\ominus}$ et n ont les significations données dans la revendication 10, et les noyaux benzéniques B et C peuvent aussi être substitués par des substituants non-chromophores

12. Azurant selon la revendication 11, contenant un composés de formule

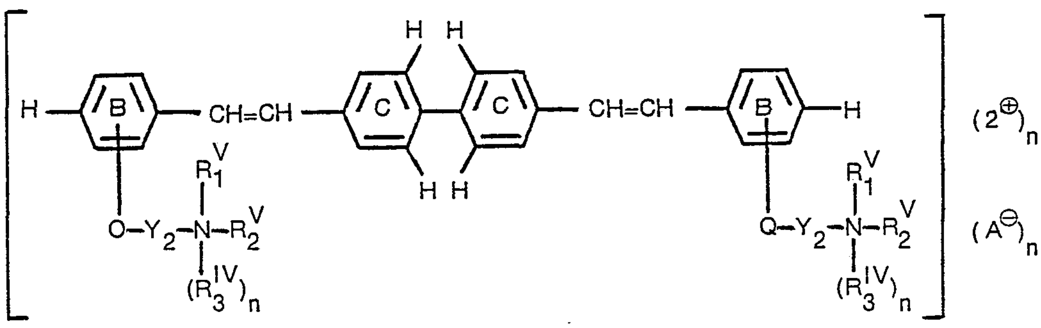

dans laquelle $Y_2$ est un enchaînement alkylène en $C_{2-20}$, $R_1^V$ est un radical alkyle en $C_{1-8}$, ou alcényle en $C_{3-4}$, non-substitué ou substitué par un groupe cyano, hydroxy, alcoxy, phényle, (alcoxy en $C_{1-4}$)carbonyle ou

chlore, ou bien $R_1^V$, avec $R_2^V$ et l'atome N auquel ils sont liés, forme un noyau hétérocyclique pentagonal à hexagonal; $R_2^V$ est un radical alkyle en $C_{1-8}$, ou alcényle en $C_{3-4}$, non-substitué ou substitué par un groupe cyano, hydroxy, alcoxy, phényle, (alcoxy en $C_{1-14}$)carbonyle ou chlore, ou bien $R_2^V$, avec $R_1^V$ et l'atome N auquel ils sont liés, forme un noyau hétérocyclique pentagonal ou hexagonal; $R_3^{IV}$ est l'hydrogène ou un radical alkyle en $C_{1-4}$ non-substitué ou substitué par un groupe cyano, hydroxy, alcoxy, phényle ou (alcoxy en $C_{3-4}$)carbonyle, $R_4$ est l'hydrogène ou un radical alkyle en $C_{1-6}$ non-substitué ou substitué par un groupe cyano, carbamoyle, carbalcoxy, hydroxy, halogène ou alcoxy en $C_{1-4}$; $A^{\ominus}$ est l'équivalent d'un anion incolore et n est le nombre 0 ou 1, les noyaux benzéniques B et C pouvant aussi être substitués par des substituants non-chromophores.

13. Procédé pour la préparation de composés définis dans la revendication 1, caractérisé en ce qu'on quaternise ou protone, respectivement avec un équivalent molaire ou deux équivalents molaires d'un agent d'alkylation ou d'un acide de formule $R_3$—A, dans laquelle $R_3$et A ont les significations données ci-dessus, un équivalent molaire d'un distyrylbiphényle de formule

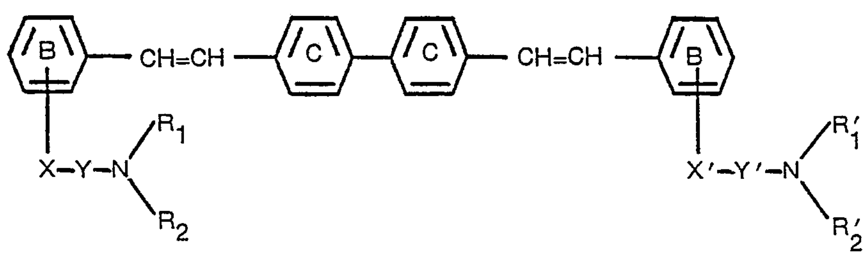

dans laquelle les noyaux benzéniques B et C, et X, X', Y, Y', $R_1$, $R_1'$, $R_2$ et $R_2'$ ont les significations correspondantes données dans la revendication 1.

14. Procédé pour la préparation de composés définis dans la revendication 6, caractérisé en ce qu'on fait réagir selon un rapport moléculaire 1:2 un composé de formule

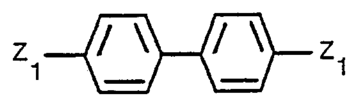

sur des composés de formule

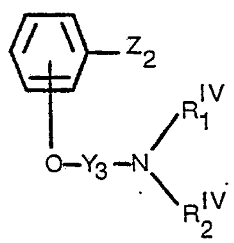

en présence d'un base forte, formules dans lesquelles $Y_3$, $R_1^{IV}$ et $R_2^{IV}$ ont la signification donnée ci-dessus, et l'un des symboles $Z_1$ et $Z_2$ est un groupe OCH—, et l'autre un groupement de formule

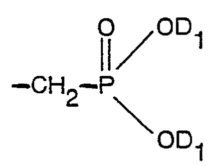

,

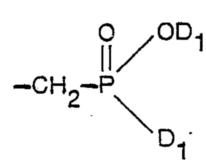

,

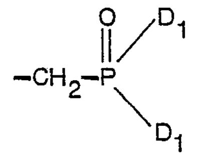

ou

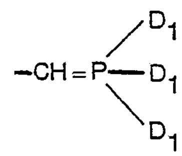

où $D_1$ est un radical alkyle, aryle, cycloalkyle ou aralkyle, non substitué ou substitué, et qu'on quaternise ou protone selon le cas désiré, respectivement avec deux équivalents molaire d'un agent d'alkylation ou un acidede formule $R_3''$—A, dans laquelle $R_3''$ et A ont les significations données ci-dessus, le composé obtenu de formule

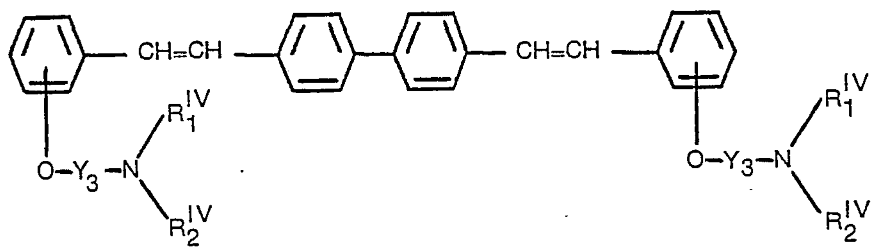

dans laquelle $Y_3$, $R_1^{IV}$ et $R_2^{IV}$ ont les significations données ci-dessus.

15. Utilisation d'un ou plusieurs des composés définis dans l'une des revendications 1 à 12 pour le blanchiment optique de matériaux organiques.

16. Utilisation selon la revendication 15, pour le blanchiment optique des matériaux organiques polyacrylonitrile ou cellulose.

17. Détergent contenant un ou plusieurs distyrylbiphényles ayant la formule définie dans la revendication 1.

18. Détergent selon la revendication 17, qui contient 10 à 70% en poids d'un tensioactif non ionique et 1 à 30% en poids d'un assouplissant cationique pour textiles.

19. Détergent selon la revendication 18, qui contient 10 à 70% en poids d'un tensioactif non ioniquie et 1 à 30% en poids d'un dérivé quaternaire de l'ammoniac et/ou de l'imidazoline, chacun ayant deux résidus aliphatiques à longue chaîne, servant d'assouplissant cationique pour textiles, et, pour conférer la forme liquide, un solvant.

20. Détergent selon la revendication 19, qui contient du 1-méthyle-1-oléylamidoéthyl-2-oléyl-imidazolinium·$X^{\ominus}$, du 1-méthyl-1-suif-amidoéthyl-2-suif-imidazoinium-$X^{\ominus}$, du di-suif-diméthylammonium·$X^{\ominus}$ ou un composé de formule

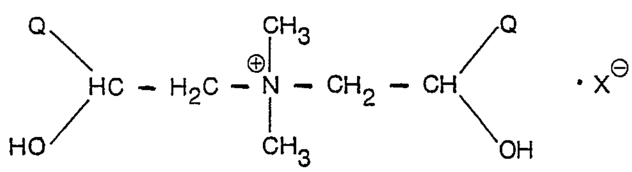

dans laquelle Q est un radical alkyle en $C_{14-16}$ et $X^{\ominus}$ est un anion chlorure, bromure, méthylsulfate, éthylsulfate, méthanesulfonate, éthanesulfonate ou toluènesulfonate, servant d'assouplissane cationique pour textiles.

21. Agent de traitement de textiles contenant un ou plusieurs distyrylbiphényles ayant la formule définie dans la revendication 1.

22. Agent de post-traitement de linge, contenant un plusieurs distyrylbiphényles ayant la formule définie dans la revendication 1, et un assouplissant cationique pour textiles.